# EUROPEAN PATENT APPLICATION

(11) **EP 1 634 961 A1**
(43) Date of publication of application: **15.03.2006**
(21) Application number: 04077506.6
(22) Date of filing: 10.09.2004
(51) Int. Cl.: C12Q 1/68

(54) **FMO3 gene polymorphisms**

(71) Applicant: Hendrix Poultry Breeders B.V., 5831 CK Boxmeer (NL)
(72) Inventor: Rattink, Annemieke Paula, 4191 WB Geldermalsen (NL)
(74) Representative: Winckels, Johannes Hubertus F.

(57) **Abstract**

The present invention relates to an isolated nucleic acid sequence corresponding to the genomic region encoding the FMO3 enzyme of a bird, or a complement thereof, said nucleic acid sequence further comprising at least one mutation resulting from a single nucleotide polymorphism (SNP) associated with the predisposition of said bird to produce fishy taint eggs, wherein said at least one single nucleotide polymorphism (SNP) is at least one of the group of single nucleotide polymorphisms (SNPs) SNP 1 to SNP 39 as shown in Table 1 or an equivalent thereof.

## Description

The present invention is in the field of bird breeding and molecular biology. More specifically, the present invention relates to methods of detecting gene polymorphisms in birds, to methods of producing birds and in particular poultry breeding lines with favourable commercial characteristics, and to birds and in particular egg-laying poultry breeding lines thus obtained.

The poultry industry is a leading agricultural commodity in many parts of the world. The industry involves both the raising of domesticated fowl (predominantly chicken and turkey) for meat (broilers), or the keeping thereof for eggs (layers). One problem associated principally with egg production is that sometimes eggs can have a fishy taint or smell.

Fishy taint in eggs of laying hens was first described in 1948 by Vondell (Vondell, 1948). Rhode Island Red hens produced eggs that had a fishy odor. This strong smelling odour is highly undesirable in modern layer breeds since consumers and farmers do not accept this deviant odour. It is therefore of considerable economic interest for the chicken companies to find the cause of this phenomenon.

A number of studies have demonstrated the occurrence of tainted eggs in brown egg layers and several of these studies haven shown an interaction with the diet of the chicken. In particular, chickens fed with diets containing high concentrations of rapeseed or fish meal produced tainted eggs.

Hobson-Frohock et al., (1973) found that TMA (trimethylamine) was the substance that causes the fishy smell of eggs, with increased levels of TMA in mainly the yolk of the egg relating to the fishy taint. In normal metabolism, TMA will be enzymatically oxidized in the liver into non-smelling trimethylamine-oxide (TMANO). This will be excreted from the body without leaving any odour. In chicken that produce tainted eggs, an increased amount of TMA is present in the body and is mainly transferred to and excreted by the egg yolk. The increase in TMA can result from increased dietary intake or from endogenous metabolism. TMA may be produced in the gut of the chicken through bacterial activity from sources such as sinapine (present in rape seed), choline and betaine.

Breeds that produce white-shelled eggs have never been found to produce eggs with a fishy taint. It was suggested that the production of tainted eggs is however not associated with the color of the eggs and indicated that it is associated with the ancestry of the birds to produce brown-shelled eggs. It was later shown that also in eggs produced by broilers a very low incidence of tainted eggs could be detected. It was concluded from these studies that the presence of tainted eggs was depending on an autosomal semidominant mutant gene with variable expression depending on environmental factors.

In humans, an autosomal recessive inborn error of metabolism, called trimethylaminuria, resulting in a partial or total inability to oxidize TMA to TMANO has been described (Treacy et al., 1998). This results in the excretion of relatively large amounts of smelling free amine in their urine, sweat, and breath. The oxidation of TMA to TMANO in humans is controlled by the enzyme flavin-containing monooxygenase 3 (FM03), with mutations in the FM03 gene causing the trimethylaminuria. The human FM03 gene consists of 9 exons, from which exon 1 is noncoding. The FM03 gene, within a cluster that also contains the genes FMO1, FM02, FM04 and FM06, has been mapped to human chromosome 1q23-q25. A human FM03 mutation database containing at least 16 variations in the gene that are known to be associated with trimethylaminuria is publicly available (http://human-fmo3.biochem.ucl.ac.uk/Human_FMO3).

It is known from WO03/057886 that fishy off-flavor in cow's milk is caused by a mutation in the bovine FM03 gene. A mutation in exon 6 (R238X) that introduces a premature stop codon is closely associated with the presence of fishy off-flavor in cow's milk. FM03 polymorphisms have also been described for humans (WO01/23603)

The genetic origin of the fishy taint in eggs of chicken is also believed to reside in polymorphisms of the FMO gene (WO03/057886), but the location of the gene and its structure are presently unknown. However, there is a need for a method for selecting breeding candidates in chicken breeding that will result in lines of layer that have no predisposition to produce fishy taint eggs.

The present inventors screened candidate genes in a chromosomal region on GGA8 (*Gallus gallus* chromosome 8) of chicken for mutations associated with the presence of tainted eggs and found that complete association exists between a large number of mutations in the introns and exons of the chicken FM03 gene and the fishy taint of eggs. These mutations were detected in an SNP discovery panel consisting of 16 chickens from a commercial pure line for the production of brown layers known to produce some eggs with fishy taint. One particular mutation in exon 7 of the FM03 gene (a T->S missense mutation in codon 329) resulted in an amino acid substitution, thus effectively altering the protein sequence. The T319S mutation was located in a large haplotype block further comprising 27 SNPs that corresponded to non-sense mutations. Five SNPs located within this haplotype block were typed on a larger population consisting of 250 animals and all showed very significant association with fishy taint eggs.

In addition to the T329S mutation and the 27 SNPs that corresponded to non-sense mutations, 3 other non-sense mutations and one additional missense mutation (L30P) were detected in a genomic region found to encode part of the FM03 enzyme and were shown to be associated with fishy taint eggs in a commercial pure line brown layers. This latter missense mutation resulted in an amino acid substitution and was detected in a predicted exon in intron 8 of the chicken FM03 gene. Seven more SNPs were found in the region corresponding to the exon 2 and a part of intron 2 of the gene sequence.

In total 39 SNPs were found in genomic regions encoding the FM03 enzyme that were completely associated with fishy taint eggs, and may accordingly be used to detect the predisposition of chicken to produce eggs with a fishy taint.

The present invention relates in a first aspect to an isolated nucleic acid sequence corresponding to the genomic region encoding the FM03 enzyme of a bird, or a complement thereof, said nucleic acid sequence further comprising at least one mutation resulting from a single nucleotide polymorphism (SNP) associated with the predisposition of said bird to produce fishy taint eggs, wherein said at least one single nucleotide polymorphism (SNP) is selected from the group consisting of the single nucleotide polymorphisms (SNPs) SNP 1 to SNP 39 as shown in Table 1, and equivalents thereof.

**Table 1. SNPs showing complete association with the fishy taint egg phenotype in the genomic region encoding the FM03 gene in chicken.**

| SNP # | Indication | Location | WT | | Mutant | | Nucl. Position (SEQ ID NO) |
|---|---|---|---|---|---|---|---|
| | | | codon | nucl. | codon | nucl. | |
| SNP1 | 229C>T | exon 2 | TGC | C | TGT | T | 229 (SEQ ID NO: 1) |
| SNP2 | 284G>T | intron 2 | - | G | - | T | 284 (SEQ ID NO: 1) |
| SNP3 | 355G>A | intron 2 | - | G | - | A | 355 (SEQ ID NO: 1) |
| SNP4 | 361G>A | intron 2 | - | G | - | A | 361 (SEQ ID NO: 1) |
| SNP5 | 369C>T | intron 2 | - | C | - | T | 369 (SEQ ID NO: 1) |
| SNP6 | 379C>T | intron 2 | - | C | - | T | 379 (SEQ ID NO: 1) |
| SNP7 | 387G>A | intron 2 | - | G | - | A | 387/393 (SEQ ID NO: 1) |
| SNP8 | 127C>T | intron 2 | - | C | - | T | 127 (SEQ ID NO: 2) |
| SNP9 | 264G>A | intron 2 | | G | - | A | 264 (SEQ ID NO: 2) |
| SNP10 | 281A>T | intron 2 | - | A | - | T | 281 (SEQ ID NO: 2) |
| SNP11 | 309G>C | intron 2 | - | G | - | C | 309 (SEQ ID NO: 2) |
| SNP12 | 422G>C | intron 2 | - | G | - | C | 422 (SEQ ID NO: 2) |
| SNP13 | 595T>C | intron 2 | - | T | - | C | 595 (SEQ ID NO: 2) |
| SNP14 | 596T>C | intron 2 | - | T | - | C | 696 (SEQ ID NO: 2) |
| SNP15 | 738C>T | exon 3 | CCC | C | CCT | T | 738 (SEQ ID NO: 2) |
| SNP16 | 1112T>A | intron 3 | - | T | - | A | 1112 (SEQ ID NO: 2) |
| SNP17 | 1142C>T | intron 3 | - | C | - | T | 1142 (SEQ ID NO: 2) |
| SNP18 | 1947T>C | intron 4 | - | T | - | C | 1947 (SEQ ID NO: 2) |
| SNP19 | 2221C>T | exon 5 | GTC | C | GTT | T | 2221 (SEQ ID NO: 2) |
| SNP20 | 2319A>G | intron 5 | - | A | - | G | 2319 (SEQ ID NO: 2) |
| SNP21 | 2326G>C | intron 5 | - | G | - | C | 2326 (SEQ ID NO: 2) |
| SNP22 | 2384C>T | exon 6 | GGC | C | GGT | T | 2384 (SEQ ID NO: 2) |
| SNP23 | 2531T>C | exon 6 | CAT | T | CAC | C | 2531 (SEQ ID NO: 2) |
| SNP24 | 2750C>T | exon 7 | AAC | C | AAT | T | 2750 (SEQ ID NO: 2) |
| SNP25 | **T329S** | exon 7 | ACT | A | TCT | T | 2829 (SEQ ID NO: 2) |
| SNP26 | 2870A>T | intron 7 | - | ?/A | - | T | 2870 (SEQ ID NO: 2) |
| SNP27 | 2943A>G | intron 7 | - | A | - | G | 2943 (SEQ ID NO: 2) |
| SNP28 | 3323A>G | ? | | A | | G | 3323 (SEQ ID NO: 2) |
| SNP29 | 3355T>C | intron 7 | - | T | - | C | 3355 (SEQ ID NO: 2) |
| SNP30 | 3505T>C | intron 8 | - | T | - | C | 3505 (SEQ ID NO: 2) |
| SNP31 | 3697C>T | intron 8 | - | C | - | T | 3697 (SEQ ID NO: 2) |
| SNP32 | 4126T>C | intron 8 | - | T | - | C | 4126 (SEQ ID NO: 2) |
| SNP33 | 4145G>T | intron 8 | - | G | - | T | 4145 (SEQ ID NO: 2) |
| SNP34 | 4189C>T | intron 8 | - | C | - | T | 4189 (SEQ ID NO: 2) |
| SNP35 | 4375T>C | intron 8 | - | T | - | C | 4375 (SEQ ID NO: 2) |
| SNP36 | **L30P** | intron 8/exon 9 | CTA | T | CCA | C | 137 (SEQ ID NO: 68) |
| SNP37 | 156G>T | intron 8/exon 9 | | G | | T | 156 (SEQ ID NO: 68) |
| SNP38 | 200C>T | intron 8/exon 9 | | C | | T | 200 (SEQ ID NO: 68) |
| SNP39 | 386T>C | intron 8/exon 9 | | T | | C | 386 (SEQ ID NO: 68) |

Preferably, said at least one single nucleotide polymorphism corresponds to a disabling mutation in an exonic sequence, meaning that the polymorphism results in an aberrant, partially, essentially or fully non-functional enzyme. In an even more preferred embodiment said at least one single nucleotide polymorphism corresponds to the missense mutation T329S in exon 7 and/or the missense mutation L30P in the predicted exon in intron 8, which corresponds to said at least one single nucleotide polymorphism (SNP) being SNP25 and/or SNP36 as shown in Table 1. The isolated nucleic acid preferably comprises essentially only exonic sequences of said genomic region encoding the enzyme, and is preferably DNA. Examples of nucleic sequences of the genomic region encoding the FM03 enzyme according to the invention, wherein the various 39 SNPs are incorporated are depicted as SEQ ID NO: 1 and SEQ ID NO: 2. Together, these sequences comprise 8 confirmed exonic regions (SEQ ID NOs: 49, 51, 54, 56, 58, 60, 62 and 64; compiled in SEQ ID NO: 69) and 8 intronic regions (SEQ ID NOs: 50, 52, 53, 55, 57, 59, 61, 63 and 65). Intron 2 (SEQ ID NOs: 52 and 53) is indicated by two partial sequences. In a much preferred embodiment, the isolated nucleic acid sequence of the invention is the sequence shown in SEQ ID NO: 69.

In another aspect, the invention relates to a polypeptide, or a part thereof, encoded by a nucleic acid sequence of the invention as described above.

In another aspect, the invention relates to a polypeptide comprising a sequence that has at least 85% identity, preferably at least 90% identity, more preferably at least 95% identity, even more preferably at least 98% identity with the polypeptide sequence shown in SEQ ID NO: 3. This predicted polypeptide may be encoded by the exon sequence of SEQ ID NO: 69 starting from (and including) nucleotide 48.

In preferred embodiments, a polypeptide according to the invention is a functionally altered mutant of the FM03 enzyme of chicken.

In another aspect, the invention relates to a polypeptide comprising a sequence that has at least 85% identity, preferably at least 90% identity, more preferably at least 95% identity, even more preferably at least 98% identity with the polypeptide sequence shown in SEQ ID NO: 75.

In much preferred embodiments, a polypeptide according to the invention comprises the T329S mutation caused by SNP25.

In another aspect, the invention relates to a set of primers suitable for amplifying a nucleic acid sequence according to any one of claims 1 to 6, comprising at least one primer selected from the group consisting of a) a specific fragment of a nucleic acid sequence shown in SEQ ID NO: 1 or 2, b) a nucleic acid fragment which specifically hybridizes under stringent conditions with a nucleic acid sequence shown in SEQ ID NO: 1 or 2, and c) SEQ ID NOs: 4 - 48, 66 and 67.

In another aspect, the invention relates to a oligonucleotide probe suitable for specifically detecting a nucleic acid sequence according to any one of claims 1 to 6, selected from the group consisting of a) a specific fragment of a nucleic acid sequence shown in SEQ ID NO: 1 or 2, wherein said fragment extends over a region of said nucleic acid comprising at least one mutation resulting from a single nucleotide polymorphism (SNP) selected from the group consisting of the single nucleotide polymorphisms (SNPs) SNP 1 to SNP 39 as shown in Table 1, and b) a nucleic acid fragment which specifically hybridizes under stringent conditions to a target region of a nucleic acid sequence shown in SEQ ID NO: 1 or 2, wherein said target region comprises at least one mutation resulting from a single nucleotide polymorphism (SNP) selected from the group consisting of the single nucleotide polymorphisms (SNPs) SNP 1 to SNP 39 as shown in Table 1.

The term "specific fragment" as used herein above refers to a nucleic acid fragment that - over its length - has 100% identity with the indicated nucleic acid sequence, is found only in the indicated nucleic acid sequence and is designed to be used as primers in specific amplification reaction of or hybridization reactions with the indicated nucleic acid sequence. Such fragments are typically at least 10 bp in length, such as at least 15 bp, more preferably 20 bp, but may also be longer such as at least 50 bp or 100 bp;.

In another aspect, the invention relates to a method for detecting a bird with a predisposition for producing fishy taint eggs, comprising providing a female bird or an egg of said bird, obtaining a nucleic acid sample of said bird or of said egg and determining in said nucleic acid sample the presence of at least one mutation in the genomic region encoding the FM03 enzyme, wherein said mutation results from a single nucleotide polymorphism (SNP) selected from the group consisting of the single nucleotide polymorphisms (SNPs) SNP 1 to SNP 39 as shown in Table 1. Preferably, the homozygous presence of said at least one allelic mutation is determined because birds homozygous for any one of the allelic mutations have shown to be essentially always affected. It is however also an aspect of the invention to determine the heterozygous presence of at least one allelic mutation selected from the SNPs of the invention as such heterozygous birds may also have a predisposition for producing fishy taint eggs under certain conditions.

In another aspect, the invention relates to a method for selecting a candidate breeder bird for use in bird breeding, comprising providing a candidate breeder bird, preferably a male bird, obtaining a nucleic acid sample of said bird, and screening said nucleic acid sample for the absence of at least one mutation in the genomic region encoding the FM03 enzyme, wherein said mutation results from a single nucleotide polymorphism (SNP) selected from the group consisting of the single nucleotide polymorphisms (SNPs) SNP 1 to SNP 39 as shown in Table 1. The absence of said at least one mutation may be heterozygous, but is preferably homozygous in order to select candidate breeder birds that are free of mutations on both alleles. It is emphasized that it is not necessary to screen all potential sites that are herein shown to be allelic for the presence of mutations in order to exclude a bird as a breeding candidate. A bird, be it male or female, which is a carrier of genes that may case its female offspring to have an increased risk of having a genetic predisposition to produce fishy taint eggs may be excluded from the breeding process based on the presence of a single SNP associated with fishy taint eggs in its genome. The presence or absence of one mutations is generally sufficient in order to make a selection, which refers to, respectively, either excluding or including said bird as a breeding candidate. A very advantageous screening procedure comprises the screening for disabling mutations. Preferably, therefore the mutation screened for absence in the genomic region is the missense mutation T329S in exon 7 and/or the missense mutation L30P in the predicted exon in intron 8. Most preferably the missense mutation T329S in exon 7 is screened.

In yet another aspect, the invention relates to a method of producing a bird breeding line, said method comprising selecting a candidate breeder bird according to a method of the invention, and crossing said bird with an other bird. Preferably, said candidate breeder bird and/or said other bird is a bird from a pure breeding line. Preferably said method further comprises subsequent steps to provide for a homozygous breeding line.

The present invention also relates to a bird breeding line obtainable by a method of the invention and having commercially valuable characteristics, such as, but not limited to, large layers, resistance to diseases, rapid growth, color, character, plumage, quality of meat, size of bird, nutritional requirements, etc..

The present invention also provides a method of testing bird feed for its propensity to give rise to fishy taint eggs, said method comprising providing a female bird, said female bird being homozygous for at least one mutation in the genomic region encoding the FM03 enzyme, providing a test feed, feeding said bird with test feed and determining the presence or absence of a fishy taint in at least one egg obtained from said bird. Essentially standard bird feeds may be used as a test feed and in case a fishy taint is present in said egg, the composition of the test feed may be altered such that the fishy taint is absent. The mutation for which the bird is homozygous is preferably selected from the group of mutations consisting of the SNPs of Table 1, more preferably, the allelic mutation is selected from the missense mutation T329S in exon 7 and the missense mutation L30P in the predicted exon in intron 8 and is most preferably the missense mutation T329S in exon 7 (SNP25).

The present invention further provides a method of producing bird feed, comprising testing said bird feed according to a method of the invention, and selecting said feed which essentially gives no rise to fishy taint eggs.

The present invention still further provides a method for detecting a fishy taint egg, comprising
- isolating DNA from said egg and determining in said DNA the homozygous presence of at least one allelic mutation in the genomic region encoding the FM03 enzyme, wherein said mutation is selected from the group of mutations consisting of the SNPs of table 1, preferably the missense mutations T329S in exon 7 or the missense mutation L30P in the predicted exon in intron 8, or
- detecting in said egg the presence of a polypeptide of the invention, wherein said polypeptide is the mutant FM03 enzyme or a part thereof.

The present invention still further provides a method for detecting a bird with a predisposition for producing fishy taint eggs, comprising providing a female bird, obtaining a protein sample of said bird and determining in said protein sample the presence of a polypeptide of the invention, wherein said polypeptide is the mutant FM03 enzyme or a part thereof. A preferred polypeptide detected in methods of the invention has at least 85% identity, preferably at least 90% identity, more preferably at least 95% identity, even more preferably at least 98% identity with the polypeptide sequence shown in SEQ ID NO: 75 and most preferably comprises the T329S mutation caused by SNP25.

In preferred aspects and embodiments of the present invention described herein above, the bird referred to is a poultry bird, preferably a domestic bird of the order *Galliformes*, still more preferably a chicken. In a particularly preferred embodiment the bird is a chicken of a commercial pure line of brown layers.

Figure 1 shows a map of the FM03 genomic region on GGA8 of chicken as obtained by methods described in the Experimental part of this specification.

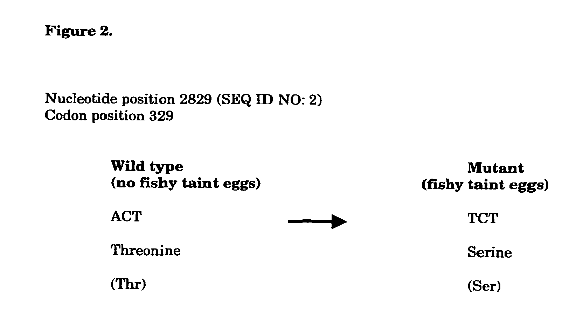

Figure 2 shows the effect of the missense mutation (T329S) in exon 7 of the chicken FM03 gene.

A "layer" is defined herein as a bird, preferably a chicken, which is kept for laying eggs. A "broiler" is defined herein as a young bird, preferably a chicken, which is kept and raised for food.

"Fowl" is defined herein as a group of birds including *Galliformes* (such as chicken, turkey, pheasant, quail, guinea fowl, partridge, grouse and peacock), and *Anserimorfes* (typically waterfowl such as duck, mallard, goose and swan).

"Poultry" is defined herein as the class of domesticated fowl used for food or for their eggs.

A "single nucleotide polymorphism" or "SNP" occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A single nucleotide polymorphism usually arises due to substitution of one nucleotide for another at the polymorphic site. Single nucleotide polymorphisms can also arise from a deletion of a nucleotide or an insertion of a nucleotide relative to a reference allele.

As used herein, the term "allele (s)" means any of one or more alternative forms of a gene, all of which alleles relate to at least one trait or characteristic. In a diploid cell or organism, the two alleles of a given gene occupy corresponding loci on a pair of homologous chromosomes. An allele is also used to describe a given version of a polymorphism.

As used herein, the term "heterozygous" means a genetic condition existing when different alleles reside at corresponding loci on homologous chromosomes.

As used herein, the term "homozygous" means a genetic condition existing when identical alleles reside at corresponding loci on homologous chromosomes.

The term "homozygous presence of at least one allelic mutation" is referred herein as the presence of an identical mutation, more specifically specified by the SNP, at both alleles.

A "haplotype" is a set of alleles for closely spaced polymorphisms along a chromosome that tend to be inherited together. Alternatively a haplotype can be thought of as a combination of alleles of closely linked loci that are found in a single chromosomal interval and tend to be inherited together. An individual SNP allele can be used to define a given haplotype.

The term, "genotype," is used herein to mean a specific allele or alleles an individual carries at a given locus. It can also be used to describe a set of alleles for multiple loci.

The term "linkage disequilibrium" is used herein to refer to the relationship that is said to exist between an allele found at a single polymorphic site and alleles found at nearby polymorphisms if the presence of one allele is strongly predictive of the alleles present at the nearby polymorphic sites. Thus, the existence of linkage disequilibrium (LD) enables an allele of one polymorphic marker (SNP) to be used as a surrogate for a specific allele of another. As an example, the presence of an SNP corresponding to a non-sense mutation that shows strong association with fishy taint eggs, may be indicative of the presence of a disabling mutation in that same haplotype block, e.g. SNP24 may be indicative of the presence of SNP25.

As used herein, the term "breeding line" or "line" means a group of similar animals that by phenotypic and genotypic characteristics can be identified from other breeding lines within the same species of animal.

As used herein, the term "pure breeding line" means a substantially homozygous line of birds, such as may be obtained by inbreeding.

As used herein, the term "chicken" means any species, variety, line, or population of *Gallus* spp., including but not limited to the domesticated chicken *Gallus gallus.*

A "probe" or "nucleic acid probe" is a nucleic acid capable of binding to a target nucleic acid of complementary sequence through one or more types of chemical bonds, usually through complementary base pairing, usually through hydrogen bond formation, thus forming a duplex structure. A probe may include natural bases (i.e., A, G, C, or T) or modified bases (e.g., 7-deazaguanosine, inosine, etc.). A probe can be an oligonucleotide which is a single-stranded DNA. Oligonucleotide probes can be synthesized or produced from naturally occurring polynucleotides. In addition, the bases in a probe can be joined by a linkage other than a phosphodiester bond, so long as it does not interfere with hybridization. Thus, probes may be peptide nucleic acids in which the constituent bases are joined by peptide bonds rather than phosphodiester linkages (see, for example, Nielsen et al., 1991). Some probes may have leading and/or trailing sequences of noncomplementarity flanking a region of complementarity.

"Hybridization" refers to binding between a nucleic acid probe and a target sequence via complementary base pairing; the resulting complex is referred to as a "hybridization complex". A hybridization complex may be either a complementary complex or a mismatch complex.

"Substantially complementary" means that the sequence of the target is not exactly complementary to the sequence of the probe; however, there is sufficient complementarity that a mismatch complex can form.

"Specific hybridization" refers to the binding, duplexing, or hybridizing of one nucleic acid molecule only to a particular nucleotide sequence under stringent conditions when that sequence is present in a complex mixture (e.g., total cellular) DNA or RNA.

"Stringent conditions" are conditions under which a probe will hybridize to its target subsequence, but to no other sequences. Stringent conditions are sequence-dependent and are different in different circumstances. A variety of factors may significantly affect the stringency of hybridization, including, among others, base composition, size of the complementary strands, the presence of organic solvents and the extent of base mismatching; the combination of parameters is more important than the absolute measure of any one. For a discussion of general factors influencing hybridization, see for example, WO 93/02216 and Watson et al., Recombinant DNA, 2.sup.nd Edition, Scientific American Books, NY 1992, each of which is incorporated herein by reference in its entirety. An extensive guide to hybridization of nucleic acids is found in Ausubel et al., Current Protocols in Molecular Biology, Greene Publishing Associates, Inc. and John Wiley and Sons, Inc. (supplemented through 1998), which is also incorporated herein by reference in its entirety. Generally, stringent conditions are selected to be about 5°C. lower than the thermal melting point (Tm) for the specific sequence at a defined ionic strength and pH. The Tm is the temperature (under defined ionic strength, pH, and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions include a salt concentration of at least about 0.01 to 1.0 M Na ion concentration (or other salts) at pH 7.0 to 8.3 and the temperature is at least about 30°C. for short probes (e.g., 10 to 50 nucleotides). Stringent conditions can also be achieved with the addition of destabilizing agents such as formamide or tetraalkyl ammonium salts. For example, conditions of 5xSSPE (750 M NaCl, 50 mM NaPhosphate, 5 mM EDTA, pH 7.4) and a temperature of 25-30° C. are typically suitable for allele-specific probe hybridizations. Stringency can be determined empirically by gradually increasing the stringency of the conditions (e.g., increasing salt, raising temperature, etc.) until the desired level of specificity is obtained.

By performing SNP studies in the FM03 enzyme-encoding sequences on the genome of chicken, the present inventors found that certain SNPs showed strong association with a fishy taint in eggs. Most of these SNPs comprised mutations that did not result in a change in the codon that would result in an amino acid substitution in the polypeptide sequence of the FM03 protein. Two SNPs however were found to result in an amino acid substitution in the predicted polypeptide sequence of the FM03 protein. Such amino acid substitutions will result in an aberrant protein, having reduced or impaired activity, and are termed herein a disabling mutation.

It is not only these disabling mutations that are useful in aspects of the present invention. Also the finding that certain SNPs show strong association with a fishy taint in eggs may be used in such aspects. For instance, there is no need to find the cause of a corrupted TMA metabolism by showing the presence of a disabling mutation in the FM03 gene or gene product in the genomic DNA or mRNA of a chicken. Rather the SNPs that correspond to non-sense mutation but show strong association with a fishy taint in eggs may be used to detect birds with a predisposition for producing fishy taint eggs, or at least an increased risk of having a predisposition. Due to the linkage disequilibrium, the presence of a non-sense SNP according to the present invention may be sufficient to exclude birds that are heterozygous or homozygous for the mutant allele from the breeding process since the chance that the such a bird gives rise to offspring having the mutant protein is significantly increased.

The present invention therefore relates to any genotypic characteristic that is useful in improving the breeding of birds. An essential element therein is the provision of an isolated nucleic acid sequence, including DNA or RNA, of a bird that comprises one or more mutations in the sequence that encodes the flavin-containing monooxygenase 3 enzyme, provided that the mutation shows a strong, preferably complete association with the predisposition of the bird to produce fishy taint eggs, and methods for detecting the presence of corresponding nucleic acid sequences in birds.

Depending on the diet and other environmental factors, a bird that homozygous for an allele with a disabling mutation does not necessarily produce eggs with fishy taint. Therefore, one should preferably refer to such a homozygous bird as having a predisposition for producing fishy taint eggs. The predisposition may for instance be determined by methods as described in the experimental part of this specification, such as by using trained individuals to judged the odour of eggs of selected birds. It should be noted that a predisposition of producing fishy taint eggs merely relates to female birds.

The presence of SNPs in the FMO3 gene-encoding sequences of the genome may be established by any method available. Very suitable methods are for instance disclosed in the experimental part of this specification. In short, a preferred method involves the isolation of genomic DNA from a part of the bird (e.g. an egg or a tissue biopt) or a blood sample, amplifying from said DNA the FMO3 genomic region using standard DNA amplification procedures such as PCR, sequencing the PCR product and comparing the obtained sequence with the reference sequence. Preferably, primers used are designed such that both exonic and intronic regions of the FM03 gene are amplified. The heterozygous presence of a mutant allele in a phenotypically normal bird, or its homozygous presence or absence, may also be determined by probing with an allele specific probe complementary only to the mutant allele (See, e.g., Guo et al., 1994).

Any method known in the art can be used to identify the nucleotide present at one of the disclosed FM03 polymorphic sites. Since the SNPs and haplotypes to be detected have been identified and specified in the present invention, detection will prove simple for one of ordinary skill in the art. Any number of techniques to detect the haplotype of an individual by genotyping the individual at certain polymorphic sites can be used, including, but not limited to, the following.

The nucleotide can be determined by sequencing analysis after DNA samples are subjected to PCR amplification. Preferably, the amplified DNA is subjected to automated dideoxy terminator sequencing reactions using a dye-primer cycle sequencing protocol. The sequencing reactions are then sequenced using any number of commercially available sequencing machines such as the ABI 377 or 3700 Sequence Analyzer (Applied Biosystems, Foster City, Calif.).

Techniques and methods of synthesizing and amplifying polynucleotides by ligation of multiple oligomers (LMO) onto a template-bound primer are also described in U.S. Pat. Nos. 5,998,175; 6,001,614; 6,013,456; and 6,020,138, which are hereby incorporated by reference in their entirety. Short polynucleotides, 5 to 10 bases long, can be supplied as a library of oligonucleotides and are simultaneously ligated, using a suitable ligase enzyme, to a template-bound primer in a contiguous manner to produce a complementary strand of template polynucleotide. If the sequence to be synthesized is known, a set containing the minimum number of oligomers can be used and are then ligated by DNA Ligase in the correct order starting from the primer, uni-or bi-directionally, to produce the complementary strand of a single-stranded template sequence.

A preferred method is to use sequence detection/amplification assays such as the INVADER assays which are commercially available from Third Wave Technologies (Madison, Wis.) to genotype samples. Such systems rely on an enzyme-substrate reaction to amplify signal generated when a perfect match with an (mutant) allele of FM03 is detected. See U.S. Pat. Nos. 5,846,717 and 5,888,780.

A third preferred method is using methods that have been developed for examining single base changes without direct sequencing. For example, if a mutation of interest happens to fall within a restriction recognition sequence, a change in the pattern of digestion can be used as a diagnostic tool (e.g., restriction fragment length polymorphism [RFLP] analysis) See U.S. Pat. Nos. 5,547,835; 6,221,601; 6,194,144 which are hereby incorporated by reference in their entirety. Other methods of SNP analysis are performed by companies such as Sequenom (San Diego, Calif.), which can genotype many samples very quickly and with great accuracy, non-sequencing methods such as MALDI-TOF, miniaturized chip-based array formats and mass spectrometry.

Other genotyping methods suited for detection of SNPs include, but are in no way limited to, LCR (ligase chain reaction), Gap LCR (GLCR), using allele-specific primers, mismatch detection assays, microsequencing assays, and hybridization assay methods.

Various methods for screening for genetic FM03 abnormalities in birds can be employed. Polynucleotides according to SEQ ID NOs: 1, 2 and 4-69 can also be used in gene marker assays, probes, primers for uses including, but not limited to, PCR, sequencing, hybridization assays and probes. Variations that are at least 85% or more homologous to the sequences of mutant FM03 polynucleotides (e.g. SEQ ID NOs: 1 and 2, 62, and 69) may also be used for comparison studies and in any of the above listed types of assays. In another embodiment, polypeptides that are at least 85%, preferably at least 95% of more homologous to FM03 protein (e.g. SEQ ID NO: 3) or to the protein generated from polynucleotide sequences selected from the group consisting of SEQ ID NO: 2, 62, and 69 may be used for allele detection.

The preferred embodiment also encompasses FM03 oligonucleotide primers made from SEQ ID NO: 1-47 and capable of amplifying the DNA sequence of and surrounding each of SNPs 1-39. Basic primer design considerations such as annealing/melting temperature, length, repetitive DNA, proximity to the SNP, and specificity will be appreciated and addressed by one skilled in the art. Many programs that enable one to pick and design custom primers address these considerations, such as PRIMER3 (Rozen and Skaletsky, 2000).

Suitable primers such as those disclosed in SEQ ID NOs: 4-23 and primers made from sequence up to about 500 base pairs away from the SNP, may be used to assay SNP25 (position 2829, SEQ ID NO: 2) and/or for sequencing this SNP. For example, SEQ ID NO: 16 (forward primer) and SEQ ID NO: 19 (reverse primer) which span exon 7 of the gene may be used. Furthermore, depending upon the genotyping strategy used, other probes and primers can be designed from SEQ ID NOs: 1,2 and 4-69 for use in such assays as PCR-RFLP and PCR INVADER assays (Third Wave Technologies, Madison, Wis.).

The tables in the Experimental part of this specification show primers that can be used to amplify genomic DNA surrounding SNPs 1-39 by such methods as PCR. The examples of forward and reverse primers can also be used to amplify sequence containing each of the described SNPs 1-39. The resulting amplified product can be genotyped by methods including, but not limited to, sequencing, mass spectrometry, RFLP, and INVADER assays. Primers such as those disclosed in Tables 2-5 and 8, SEQ ID NOS: 4-48 and primers made from sequences up to about 500 base pairs away from the SNP can be used for amplification and/or sequencing these SNPs.

In one embodiment of the invention, fragments of various lengths of mutant FMO3 DNA (preferably cDNA) may be placed onto solid supports for use in gene chips or other parallel formats for assay purposes. In general, these methods employ arrays of oligonucleotide probes that are complementary to targeted nucleic acid sequences, and allow for detection when the sample hybridizes to a probe on the array. In preferred embodiments, the nucleic acid sequences are mutant FMO3 fragments of about 15-30 nucleotides in length, specifically sequences containing FMO3 SNPs 1-39. In further embodiments, the chip may comprise an array including at least one of the sequences selected from the group consisting of amplification primers with SEQ ID NOs 4-48 as capture ligands in sandwich hybridization assays, well known in the art. See e.g. Lockhart, et al. (1996), for useful methods and heuristics in designing oligonucleotide probes from FMO3 fragments.

Chips, or microarrays of various formats from companies such as Agilent Technologies (Palo Alto, Calif.) and Affymetrix (Santa Clara, Calif.) can be produced on a customized basis by various methods. Alternatively, DNA microarray chips are fairly inexpensive to make and assemble. Individual samples to be tested are then contacted with the oligonucleotide probes and the genotype and/or haplotype of the sample can be determined based on detection of the hybridization between the probes and the sample. A suitable DNA micro-array is disclosed in Brown et al. U.S. Pat. No. 5,807,522

Instead of detecting the nucleic acid sequences, an alternative embodiment enables testing for the polymorphic FMO3 polypeptides of the invention and association with fishy taint eggs in birds having polymorphisms deviating from the normal or "wild type" phenotype. Genetic testing may be carried out on a bird's protein, provided that antibodies capable of distinguishing mutant from wild type FMO3 protein are available. The present invention therefore also relates to a method for detecting a bird with a predisposition for producing fishy taint eggs, comprising providing a female bird, obtaining a protein sample of said bird and determining in said protein sample the presence of a mutated FMO3 enzyme, wherein said mutated FMO3 enzyme comprises a threonine residue at position 329.

Antibodies including both polyclonal and monoclonal antibodies to the polymorphic FMO3 polypeptides of the invention possess certain diagnostic applications and may, for example, be utilized for the purpose of detecting the identity of the haplotype of birds. For example, wild type FMO3 and its variants may be used to produce both polyclonal and monoclonal antibodies in a variety of cellular media, by known techniques such as the hybridoma technique utilizing, for example, fused mouse spleen lymphocytes and myeloma cells.

The general methodology for making monoclonal antibodies by hybridomas is well known. Immortal, antibody-producing cell lines can be created by techniques other than fusion, such as direct transformation of B lymphocytes with oncogenic DNA, or transfection with Epstein-Barr virus. See, e.g., Schreier, M., G. Köhler, H. Hengartner, C. Berek, M. Trucco, L. Forni. 1980. Hybridoma Techniques Cold Spring Harbor Lab. Press, Cold Spring Harbor, NY.; Hammerling, G. J., Hammerling, U. and Kearney, J. F. eds., 1981. " Monoclonal Antibodies and T Cell Hybridomas". Elsevier Publishing Co. PP. 509-515, 1981; see also U.S. Pat. Nos. 4,341,761; 4,399,121; 4,427,783; 4,444,887; 4,451,570; 4,466,917; 4,472,500; 4,491,632; 4,493,890.

Panels of monoclonal antibodies produced against FM03 peptides (i.e. part of the mutant protein) can be screened for various properties; i.e., isotype, epitope, affinity, etc. Of particular interest are monoclonal antibodies that specifically bind and identify the alleles of FMO3, and can distinguish between the mutant and the normal alleles of FMO3. In one preferred embodiment, a monoclonal antibody can be generated that specifically binds to the position of the FMO3 protein that results from the SNP25 allele, i.e. the region comprising the threonine residue on residue number 329. Such monoclonals can be readily identified in, for example, gel-shift assays. High affinity antibodies are also useful when immunoaffinity purification of native or mutant FMO3 is possible.

A preferred method of generating these FMO3 allele-specific antibodies is by first synthesizing peptide fragments. These peptide fragments should cover at least SNP25 and the adjacent amino acid sequence. Subsequent antibodies should be screened for their ability to distinguish the two protein variants. Since synthesized peptides are not always immunogenic on their own, the mutant FMO3 peptides should be conjugated to a carrier protein before use. Appropriate carrier proteins include but are not limited to Keyhole limpet hemacyanin (KLH). The conjugated peptides should then be mixed with adjuvant and injected into a mammal, preferably a rabbit through intradermal injection, to elicit an immunogenic response. Samples of serum can be collected and tested by ELISA assay to determine the titer of the antibodies and then harvested.

Polyclonal FMO3 allele-specific antibodies can be purified by passing the harvested antibodies through an affinity column. Monoclonal antibodies are preferred over polyclonal antibodies and can be generated according to standard methods known in the art of creating an immortal cell line which expresses the antibody.

Additionally, spleen cells can be harvested from the immunized animal (typically rat or mouse) and fused to myeloma cells to produce a bank of monoclonal antibody-secreting hybridoma cells. The bank of hybridomas can be screened for clones that secrete immunoglobulins that bind the protein of interest specifically, i.e., with an affinity of at least 1 x 10⁷ M⁻¹. Animals other than mice and rats may be used to raise antibodies; for example, goats, rabbits, sheep, and chickens may also be employed to raise antibodies reactive with an FMO3 protein.

Bacteriophage antibody display libraries may also be screened for phage able to bind peptides and proteins specifically. Combinatorial libraries of antibodies have been generated in bacteriophage lambda expression systems and may be screened as bacteriophage plaques or as colonies of lysogens. For general methods to prepare antibodies, see Antibodies: A Laboratory Manual (1988), E. Harlow and D. Lane, Cold Spring Harbor Laboratory, Cold Spring Harbor, N.Y., incorporated herein by reference.

In a method for detecting a bird with a predisposition for producing fishy taint eggs and in a method for selecting a candidate breeder bird for use in bird breeding essentially the same methods for detection and/or screening may be applied as described above.

In the method for selecting a candidate breeder bird, the screening and exclusion process may be applied in all pure breeding lines of a bird that contribute to a certain hybrid line cross that is marketed and used for the production of eggs. The screening and exclusion process may for instance be applied to a pure breeding line in this way to exclude the fishy taint phenotype from the eggs produced by that breeding line itself. Alternatively, only the pure breeding line or pure breeding lines that constitute the male parent line and/or the female parent line of the final cross is/are subjected to the screening and exclusion process. This is attractive because fewer birds have to be tested and excluded and the absence of homozygotes for the mutation is guaranteed when one of the parents is not carrying a mutation. Preferably, the line or lines to be chosen for the screening and exclusion process are the lines with the lowest frequency of the mutation. The invention is particularly relevant in the breeding of chickens for the production of brown shelled eggs as the mutation is especially known to occur in these breeds of chickens. The invention may also be used in any other bird, any other fowl or any other breed of chicken that carries the same mutation. For instance, other breeds of chicken different from the ones described in the Experimental part herein are known to share (part of) the same genes as the chicken breeds presently known to carry the mutation and these other breeds may also benefit from the invention. The phenotype of fishy taint has also been described in broiler breeding lines.

The screening and exclusion process of may be applied to all breeding candidates of a certain pure breeding line or, alternatively, to save costs, it may be applied to male breeding candidates only,

The invention may also be used to screen birds used for production of eggs themselves. The exclusion of homozygous carriers will then suffice to make sure that no eggs with fishy taint are laid by this group of birds.

The invention may also be used to verify presence or absence of the mutation in a particular egg by isolating and testing DNA from the egg. This could be useful to prove the identity of the egg as an egg that does not carry the mutation and therefore cannot show the fishy taint phenotype,

The invention may also be used to create populations and/or pure breeding lines that are fully homozygous for the mutation. Such populations may be used for the evaluation of existing and novel materials and feeds to verify if these give rise to fishy taint in eggs of homozygous carriers of the mutation.

In a method of producing bird feed according to the present invention, a bird feed may be manufactured by mixing suitable ingredients in the conventional manner and provided to a test panel of birds. Such a test panel comprising birds that are homozygous for an allele with a disabling mutation (e.g. SNP T329S), and preferably also comprising reference birds that are either heterozygous and/or that are homozygous for the normal wild type allele. The latter type of birds will not produce fishy taint eggs and may serve as a control group. Upon testing of the eggs for the presence of fishy taint, either by judging the odour of an egg itself of by testing for the presence and optionally quantity of TMA in said eggs, those feeds that give rise to eggs that are essentially free of fishy taint may be used as a feed for commercial layers that have a predisposition for producing fishy taint eggs. By such feeding, the economic value of birds that have a predisposition for producing fishy taint eggs may be effectively increased.

In one aspect the present invention relates to methods of producing transgenic birds having commercially or otherwise favourable characteristics such as no predisposition to produce fishy taint eggs.

The present invention in one aspect also relates to a kit of parts comprising primers and/or probes according to the present invention and suitable for performing a method of the present invention.

### Experimental part

### Materials and Methods

### Animals

In a commercial pure line for the production of brown layers known to produce some eggs with fishy taint, all hens (250 animals) of one age group were phenotyped for odour of the eggs and blood samples were taken. These animals originate from 21 hens and 7 cocks. Thus, 7 paternal half sib or 21 maternal half sib families were included. From these animals also blood samples were taken. Genomic DNA was isolated from these blood samples following standard protocols.

### Phenotypes.

Phenotypes were collected for all 250 hens. Two trained individuals each judged the odour of an egg from the hens. Presence or absence of fishy smell was recorded. The observations of the two different persons were combined to create three classes: class 0 implies that both persons observed no fishy smell, class1 implies that one of the two persons observed a fishy smell and class 2 indicates that both persons observed fishy smell of the egg.

### Amplification and sequencing of the chicken FMO3 gene and loci homologous to human FMO genes located on GGA8.

Primers for chicken FMO3 were designed based on the published mRNA sequence (accession AJ431390) from NCBI Entrez Nucleotide database (www.ncbi.nlm.nih.gov/entrez/) for detection of SNPs in these sequences. The genomic position of this sequence (4.77 Mb on GGA8) was derived from the first draft of the chicken genome assembly by Ensembl (www.ensembl.org). Primers were designed in such a way that both exonic and intronic regions could be amplified (Table 2).

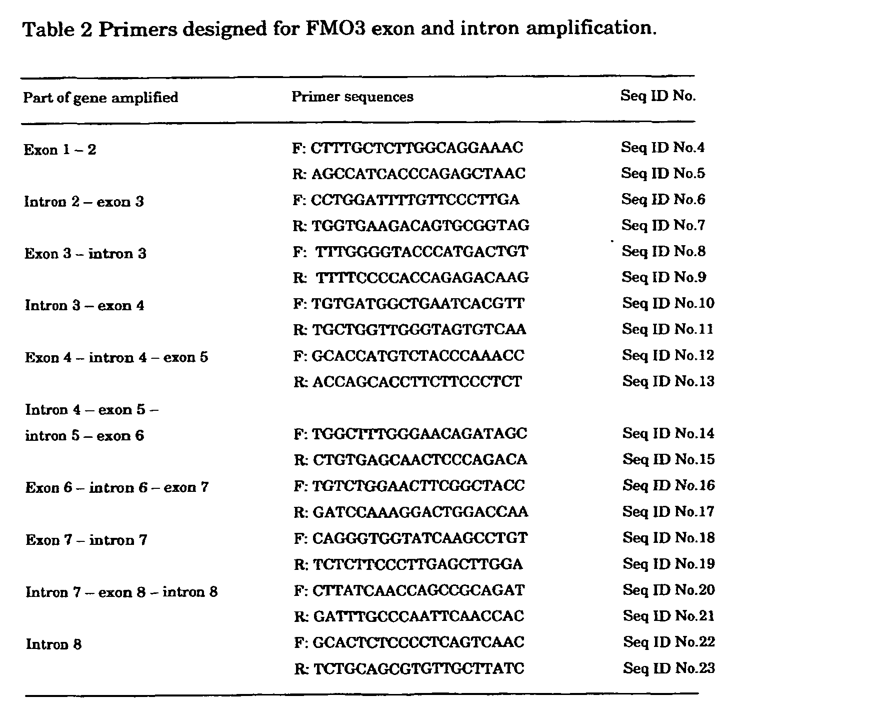

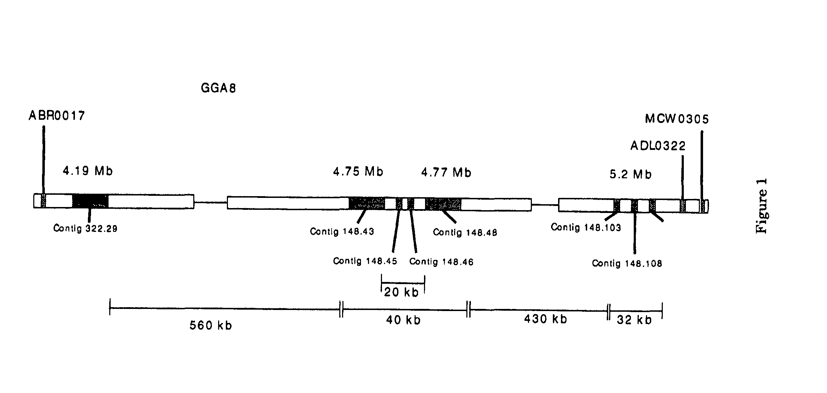

in addition, human FMO sequences were used in a BLAST search against the Chicken Genome Sequence from the Ensembl database (www.ensembl.org/Gallus_gallus/). This search lead to the detection of additional chicken sequences homologous to human FMO sequences located on the same chromosomal region on GGA8 as chicken FMO3 is located on (Figure 1). For several putative chicken FMO sequences primers were designed for SNP detection in these sequences (Table 3).

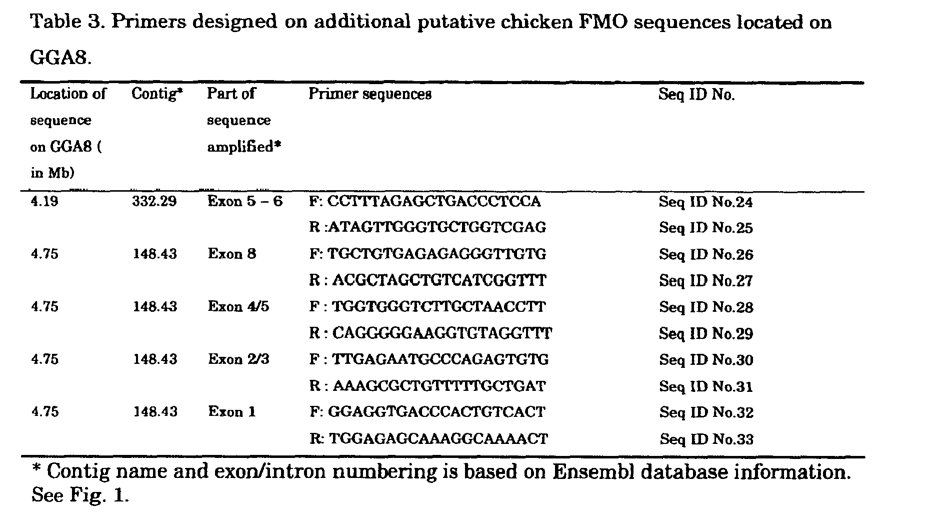

The PCR reactions were carried out in 24 µl volumes. Reactions contained 6 µl DNA (10 ng/µl), 12 µl AB-gene Master Mix, 2 µl primermix (both primers in a conc. of 30 ng/µl) and 4 µl MQ. Reactions were performed in a PCR machine with heated lid, e.g. PTC100 thermocycler (MJ Research, Watertown, MA, USA) with a 5 min initial denaturation at 95°C followed by 36 cycles of 30 s at 95°C, 45 s at annealing temperature and 90 s at 72°C, and a final extension for 10 min at 72°C. The annealing temperatures were optimized for each primer pair and varied between 50°C and 62°C.

After PCR the excess of primers was removed by running the samples over MANU030 PCR columns in 96-well format (Millipore, USA). From each sample, 2 µl was checked on agarose gel to estimate the DNA concentration. Sequencing reactions were performed with either the forward or the reverse amplification primer. Cycle sequencing reactions contained 100-400 ng of purified PCR product, 1 µl of Big Dye Terminator Rtmix version 2.0 (PE, Foster City, CA, USA), 3 µl of Half Big Dye Buffer (Genetix, New Milton, UK) and 0.8 pmol of either primer in a final volume of 10 µl. Excess dye terminator was removed by purification of the samples on Montage SEQ96 columns in 96-well format (Millipore, USA). After purification, the DNA is held in 15 µl Injection Solution (Millipore, Bedford, MA, USA). 2 µl is mixed with 10 µl formamide and denatured for 2 min. at 95°C before analysis on an ABI 3100 automated sequencer (ABI, Foster City, CA, USA).

### SNP detection

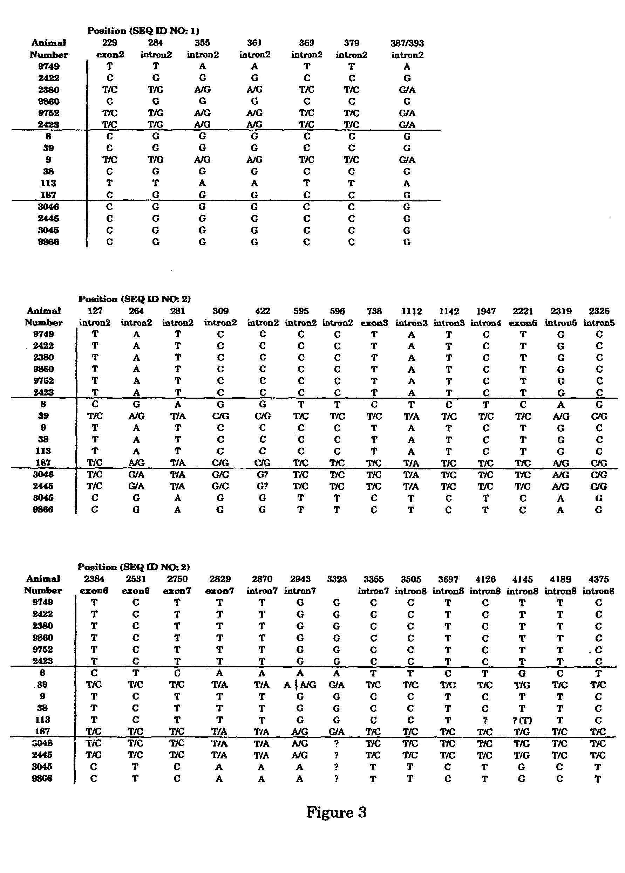

The SNP discovery panel consisted of 12 individuals: 4 hens with tainted eggs, 2 hens without fishy taint eggs and 6 animals representing the parents of the hens (2 males and 4 females). After high association was detected for the SNPs in the chicken FM03 gene, 4 animals were added to the SNP discovery panel. Two hens without tainted eggs and 2 hens that were expected to be heterozygous for fishy taint in their eggs were added to the panel. In Table 9 and Figure 3, Animal nos. 9749, 2422, 2380, 9860, 9752 and 2423 represent the ss phenotype (homozygotes, tainted egg producers); animals 8, 39, 9, 38, 113, and 187 represent the parents of the tainted egg producers; animals 3046 and 2445 are heterozygotes Ss phenotype (no tainted eggs) and animals 3045 and 9866 are SS phenotype (homozygotes, no tainted eggs).

Trace files were analyzed using the Pregap4 program of the Staden software package (Staden, 1996; www.mrc-lmb.cam.ac.uk/pubseq).

Using Pregap4, all chromatograms were analyzed: bases were called with Phred (Ewing et al., 1998) and low quality regions were masked. Next, all sequences passing Pregap4 were entered in a Gap4 database. In the Gap4 a normal shotgun assembly was performed with a minimal initial match of 20 bp, a maximum of 25 pads per read and a maximum of 5 % mismatches. If necessary, the assembly was finished manually using the Gap4 *Join contig* interface. Positions at which a disagreement occurred were highlighted in the Gap4 contig editor window using the *Highlight disagreements* option. All nucleotide positions at which a disagreement occurred were tagged as a putative SNP position.

### Design of SBE primers

Single-base extension (SBE) primers were designed to have a specific 3'end 18-25 bp in length. A non-specific 5'tail was used to create primers 25-120 bp in length, with 5 bp intervals. Two sets of SBE primers were designed; one for SNPs detected in chicken FMO3 gene (Table 4) and one set for SNPs detected in additional chicken sequences homologous to human FMO sequences (Table 5).

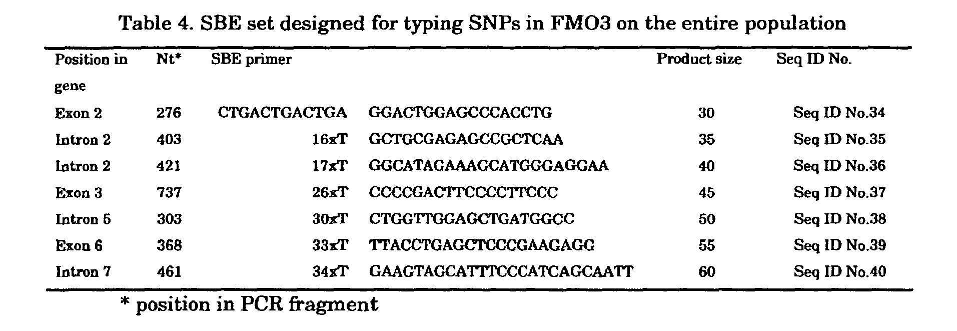

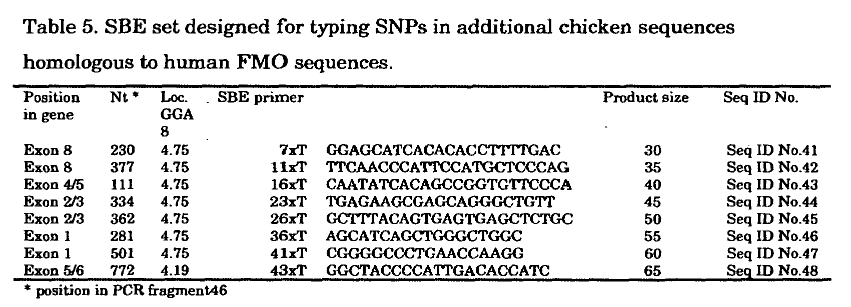

### Typing of SNPs on population

After identification of SNPs from the 16 individuals, the entire population of parents and their 250 offspring were genotyped for these SNPs by single-base extension (SBE). SBE genotyping was performed using the multiplex SNaPshot kit (ABI, Foster City, CA, USA) following the protocol provided by the manufacturer. SNaPshot products were analyzed on an ABI3100 automated sequencer. Resulting data were analyzed using the GeneMapper software package (v3.0 ; ABI) and checked by two independent persons.

### Association studies

SNPs and microsatellite markers typed on the entire population of chickens from the commercial pure line known to produce some eggs with fishy taint were analysed using the LDLA (Linkage Disequilibrium / Linkage Analysis) program by Meuwissen et al. (2002). In this program, in the first step linkage phases of the marker alleles are inferred to construct their paternally and maternally inherited marker haplotypes. In the second step, IBD probabilities of pairs of haplotypes at putative mutation or QTL positions are predicted. The third step involves the calculation of the LogLikelihood at putative mutation or QTL positions using variance component methods. After that twice the reduction in LogLikelihood was tested, resulting from dropping *t* random terms as a Chi-quare statistic. The 5% significance threshold for this test statistic is 3.9.

### Results

### Amplification of the chicken FMO3 gene and loci homologous to human FMO genes located on GGA8

Sequences for chicken FMO3 gene corresponding to exon 1 (SEQ ID NO: 49), intron 1 (SEQ ID NO: 50), exon 2 (SEQ ID NO: 51), partial intron 2A (SEQ ID NO: 52), partial intron 2B (SEQ ID NO: 53), exon 3 (SEQ ID NO: 54), intron 3 (SEQ ID NO: 55), exon 4 (SEQ ID NO: 56), intron 4 (SEQ ID NO: 57), exon 5 (SEQ ID NO: 58), intron 5 (SEQ ID NO: 59), exon 6 (SEQ ID NO: 60), intron 6 (SEQ ID NO: 61), exon 7 (SEQ ID NO: 62), intron 7 (SEQ ID NO: 63), exon 8 (SEQ ID NO: 64) and intron 8 (SEQ ID NO: 65) were generated and the amino acid sequence comprised in the exonic regions was generated using the XXX program. The exonic sequences showed very high percentage identity with the sequence that was used to design the primers (AJ431390).

### Sequencing and SNP detection in the chicken FMO3 gene

Sequencing of the PCR fragments, covering a very large part of the chicken FMO3 gene on the sequencing-test panel of 12 animals from a commercial pure line brown layers known to produce some eggs with fishy taint, resulted in a large number ef SNPs between these animals (Figure 3).

SNPs were detected in both intronic (28 SNPs) and exonic (7 SNPs) sequences.

As can be seen from Figure 3, all SNPs can be grouped in two haplotype blocks. One small haplotype block covers exon 1, intron 1, exon 2 and a part of intron 2. One large haplotype block covers the remaining part of intron 2 through intron 8. Initially, based on the 12 animals in the SNP discovery panel, none of the detected SNPs was completely associated with the phenotype.

### Typing of SNPs on population

Two SNPs from the small haplotype block and five SNPs representing the large haplotype block were typed on the complete population. The test statistics resulting from the LDLA analysis are given in Table 6. It can be seen from this table that significant association was detected between the SNPs in the large haplotype block and the phenotype of tainted eggs. The 5% significance threshold for the test statistic is 3.9

**Table 6. LDLA analysis of SNPs in chicken FMO3**

| SNP | Nt* | Test statistic |
|---|---|---|
| Exon 2 | 276 | |
| | | 1.56 |
| Intron 2 | 403 | |
| | | 5.50 |
| Intron 2 | 421 | |
| | | 8.34 |
| Exon 3 | 737 | |
| | | 9.24 |
| Intron 5 | 303 | |
| | | 4.96 |
| Exon 6 | 368 | |
| | | 8.24 |
| Intron 7 | 461 | |

| | | |
|---|---|---|
| * position in PCR fragment | | |

### Sequencing and SNP detection in the additional FMO sequences

To test whether any sequences or genes located between chicken FMO3 and a microsatellite marker identified as ABR0017 could be associated with tainted eggs, several chicken sequences homologous to human FMO sequences located on the same chromosomal region on GGA8 as chicken FMO3 is located on were generated (Figure 4). Sequencing of the PCR fragments on the selected 12 animals resulted in 19 SNPs between these animals (Figure 5). Seven SNPs were selected to be genotyped on the entire population. The results of the LDLA analysis are presented in Table 7. Based on the results from Table 6 and Table 7, the highest association was detected in the chicken FMO3 gene, with the SNPs from the large haplotype block.

**Tabel 7. LDLA analysis of SNPs in putative chicken FMO sequences.**

| SNP | Nt* | Loc. on GGA8 | Test statistic** |
|---|---|---|---|
| Exon 8 | 230 | 4.75 | |
| | | | 3.10 |
| Exon 8 | 377 | 4.75 | |
| | | | 4.12 |
| Exon 4/5 | 111 | 4.75 | |
| | | | 3.52 |
| Exon 2/3 | 334 | 4.75 | |
| | | | NG |
| Exon 2/3 | 362 | 4.75 | |
| | | | NG |
| Exon 1 | 281 | 4.75 | |
| | | | NG |
| Exon 1 | 501 | 4.75 | |
| | | | NG |
| Exon 5/6 | 772 | 4.19 | |

| | | | |
|---|---|---|---|
| * position in PCR fragment | | | |
| ** NG means not significant | | | |

Based on both the phenotypic information and the genotypic information derived from the LDLA analysis on the SNP discovery panel containing 12 animals, it became clear that the 2 hens without tainted eggs in the SNP discovery panel had the same haplotype as the 4 animals with fishy taint eggs. This would indicate that no animals without tainted eggs were represented in the SNP discovery panel. Thus, two hens that were expected to be heterozygous for fishy taint in their eggs and 2 animals that were expected to homozygous for the absence of fishy taint were added to the panel. All PCR fragments for chicken FMO3 (Table 2) were sequenced for these animals.

### Identification of a missense mutation (T329S) in exon 7 of the chicken FMO3 gene

All previously detected SNPs in both intronic (a total of 28 SNPs) and exonic (a total of 7 SNPs) sequences were also detected in the four additional animals. Of the six SNPs found in exons, six were silent mutations, and only one in exon 7 (at nucleotide position 2829 in SEQ ID NO: 2) was a missense mutation resulting in an altered amino acid (T329S).

The A>T nucleotide substitution located at nucleotide position 2829 changes the codon for Threonine (T) at amino acid position 329 to Serine (S). For the T329S missense mutation and all five nonsense mutations in chicken FM03, the 16 animals showed complete association between the mutations and the presence of fishy taint eggs.

### Predicted exon in intron 8 with missense mutation associated with fishy taint eggs

In addition to the detection of additional chicken sequences on GGA8 based on sequence homology to human FMO sequence, homology to a sequence contig (Contig 24165.1) with unknown genomic location was detected. In this contig, the Ensembl automatic analysis pipeline predicted two exons and intronic sequence in between these exons (http://www.ensembl.org/Gallus_gallus/).

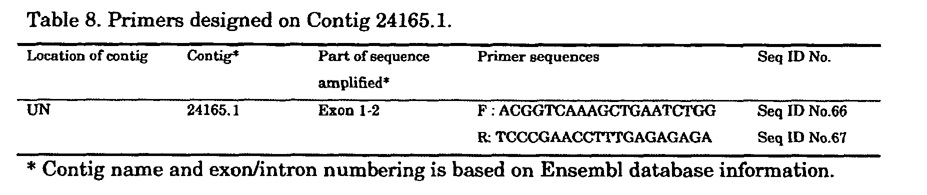

Primers were designed on the sequence of this contig (Table 8). The PCR product was sequenced for the 12 animals in the SNP discovery panel resulting in Contig 24165.1 (SEQ ID NO: 68). Four SNPs were detected. Two SNPs are located in the first exon and two SNPs are located in the intronic region (Table 9).

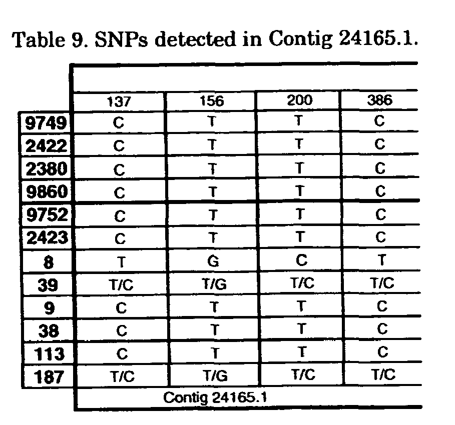

One SNP in the first exon is a nonsense mutation, and the other SNP in the first exon is a missense mutation (L30P) changing a Proline to a Leucine at nucleotide position 137 of the Contig 24165.1 (Table 10). All four SNPs are completely associated with tainted eggs within the SNP discovery panel.

**Table 10. A missense mutation (L30P) in Contig 24165.1 (SEQ ID NO: 68) is associated with fishy taint eggs.**

| Wild type (no fishy taint eggs) | Mutant (fishy taint eggs) |
|---|---|
| CTA | CCA |
| Leucine (L) | Proline (P) |
| Nucleotide position 137 | |
| Codon position 30 | |

To determine the location of this unmapped contig, the sequence of Contig 24165.1 was used in a BLAST search against the complete chicken sequence database (Ensembl). One hundred percent identity was detected with a part of intron 8 and exon 9 of the chicken FM03 gene on GGA8. The second exon of Contig 24165.1 was 100% identical to exon 9 of the FMO3 gene. The first exon and the intron of Contig 24165.1 were identical to the sequence of intron 8 of the chicken FMO3 gene.

A BLAST comparison of the Contig 24165.1 and the genome sequence of chicken FMO3 gene indicate the presence of an additional exon in intron 8 of chicken FMO3. In this predicted exon, a nonsense and a missense mutation are detected. Both the missense mutation (L30P) and the nonsense mutation are associated with fishy taint eggs in a commercial pure line brown layers.

### REFERENCES

Ewing B, Hillier L, Wendl MC, Green P. 1998. Base-calling of automated sequencer traces using phred. I. Accuracy assessment. Genome Res. 8(3):175-85.
Guo, Z. Guilfoyle, R.A., Thiel, A.J., Wang, R., and Smith, L.M. 1994. Direct fluorescence analysis of genetic polymorphisms by hybridization with oligonucleotide arrays on glass supports. Nucleic Acids Res 22:5456-65.
Hobson-Frohock, A., Land, D.G., Griffiths, N.M. and Curtis, E.F. 1973. Egg taints: association with trimethylamine. Nature 243:304-305.
Lockhart, D.J. et al., 1996. "Expression monitoring by hybridization to high-density oligonucleotide arrays," Nature Biotechnology, 14:1675-1680.
Meuwissen, T., A. Karlsen, S. Lien, I. Olsaker, et M. Goddard. 2002 Fine mapping of a quantitative trait locus for twinning rate using combined linkage and linkage disequilibrium mapping. Genetics 161(1) : 373-9.
Nielsen, P.E., Egholm, M., Berg, R.H., and Buchardt, O. 1991. Sequence selective recognition of DNA by strand displacement with a thyrnine-substituted polyamide. Science 254: 1497-1500.
Rozen, S. and Skaletsky, H.J. (2000) Primer3 on the WWW for general users and for biologist programmers. In Krawetz, S. and Misener, S. (eds), Bioinformatics Methods and Protocols: Methods in Molecular Biology. Humana Press, Totowa, NJ, pp. 365-386.
Staden, R. 1996. The Staden Sequence Analysis Package. Molecular Biotechnology 5, 233-241.
Treacy E.P. et al., 1998. Mutations of the flavin-containing monooxygenase gene (FM03) cause trimethylaminuria, a defect in detoxification. Hum Mol Genet 7:839-845.
Vondell, J.H. 1948. Detection of chickens laying "fishy eggs." Poult. Sci. 27:244.

ANNEX TO SEQUENCE LISTING

Application number: EP 04077506.6
Applicant: Hendrix Poultry Breeders BV
Title: "FMO Gene Polymorphisms"
Attorney reference: FS/P70434EP00

The EPO formalities Examiner noticed a difference in the number of sequences in the description and
the number of sequences in the sequence listing. For one CDS-sequence (protein-coding sequence) in
the description, PatentIn generates two sequences in the sequence listing (i.e. the complete CDS-
sequence and a corresponding amino acid sequence). This results in a discrepancy in the number of
sequences (and the corresponding sequence-numbering) between the description and PatentIn-
generated list. For the above-mentioned application the sequence numbers in the PatentIn sequence
listing correspond to the following numbers in the description:

Sequence no. 1 of the sequence listing corresponds to sequence no. 1 in the description.
Sequence no. 2 of the sequence listing corresponds to sequence no. 2 in the description.
Sequence no. 3 of the sequence listing corresponds to sequence no. 3 in the description.
Sequence no. 4 of the sequence listing corresponds to sequence no. 3 in the description because
this is the amino acid sequence attached to seq. no 3.
Sequence no. 5 of the sequence listing corresponds to sequence no. 4 in the description.
Sequence no. 6 of the sequence listing corresponds to sequence no. 5 in the description.
Sequence no. 7 of the sequence listing corresponds to sequence no. 6 in the description.
Sequence no. 8 of the sequence listing corresponds to sequence no. 7 in the description.
Sequence no. 9 of the sequence listing corresponds to sequence no. 8 in the description.
Sequence no. 10 of the sequence listing corresponds to sequence no. 9 in the description.
Sequence no. 11 of the sequence listing corresponds to sequence no. 10 in the description.
Sequence no. 12 of the sequence listing corresponds to sequence no. 11 in the description.
Sequence no. 13 of the sequence listing corresponds to sequence no. 12 in the description.
Sequence no. 14 of the sequence listing corresponds to sequence no. 13 in the description.
Sequence no. 15 of the sequence listing corresponds to sequence no. 14 in the description.
Sequence no. 16 of the sequence listing corresponds to sequence no. 15 in the description.
Sequence no. 17 of the sequence listing corresponds to sequence no. 16 in the description.
Sequence no. 18 of the sequence listing corresponds to sequence no. 17 in the description.
Sequence no. 19 of the sequence listing corresponds to sequence no. 18 in the description.
Sequence no. 20 of the sequence listing corresponds to sequence no. 19 in the description.
Sequence no. 21 of the sequence listing corresponds to sequence no. 20 in the description.
Sequence no. 22 of the sequence listing corresponds to sequence no. 21 in the description.
Sequence no. 23 of the sequence listing corresponds to sequence no. 22 in the description.
Sequence no. 24 of the sequence listing corresponds to sequence no. 23 in the description.
Sequence no. 25 of the sequence listing corresponds to sequence no. 24 in the description.
Sequence no. 26 of the sequence listing corresponds to sequence no. 25 in the description.
Sequence no. 27 of the sequence listing corresponds to sequence no. 26 in the description.
Sequence no. 28 of the sequence listing corresponds to sequence no. 27 in the description.
Sequence no. 29 of the sequence listing corresponds to sequence no. 28 in the description.
Sequence no. 30 of the sequence listing corresponds to sequence no. 29 in the description.
Sequence no. 31 of the sequence listing corresponds to sequence no. 30 in the description.
Sequence no. 32 of the sequence listing corresponds to sequence no. 31 in the description.
Sequence no. 33 of the sequence listing corresponds to sequence no. 32 in the description.
Sequence no. 34 of the sequence listing corresponds to sequence no. 33 in the description.
Sequence no. 35 of the sequence listing corresponds to sequence no. 34 in the description.
Sequence no. 36 of the sequence listing corresponds to sequence no. 35 in the description.
Sequence no. 37 of the sequence listing corresponds to sequence no. 36 in the description.
Sequence no. 38 of the sequence listing corresponds to sequence no. 37 in the description.
Sequence no. 39 of the sequence listing corresponds to sequence no. 38 in the description.

Sequence no. 40 of the sequence listing corresponds to sequence no. 39 in the description.
Sequence no. 41 of the sequence listing corresponds to sequence no. 40 in the description.
Sequence no. 42 of the sequence listing corresponds to sequence no. 41 in the description.
Sequence no. 43 of the sequence listing corresponds to sequence no. 42 in the description.
Sequence no. 44 of the sequence listing corresponds to sequence no. 43 in the description.
Sequence no. 45 of the sequence listing corresponds to sequence no. 44 in the description.
Sequence no. 46 of the sequence listing corresponds to sequence no. 45 in the description.
Sequence no. 47 of the sequence listing corresponds to sequence no. 46 in the description.
Sequence no. 48 of the sequence listing corresponds to sequence no. 47 in the description.
Sequence no. 49 of the sequence listing corresponds to sequence no. 48 in the description.
Sequence no. 50 of the sequence listing corresponds to sequence no. 49 in the description.
Sequence no. 51 of the sequence listing corresponds to sequence no. 50 in the description.
Sequence no. 52 of the sequence listing corresponds to sequence no. 51 in the description.
Sequence no. 53 of the sequence listing corresponds to sequence no. 52 in the description.
Sequence no. 54 of the sequence listing corresponds to sequence no. 53 in the description.
Sequence no. 55 of the sequence listing corresponds to sequence no. 54 in the description.
Sequence no. 56 of the sequence listing corresponds to sequence no. 55 in the description.
Sequence no. 57 of the sequence listing corresponds to sequence no. 56 in the description.
Sequence no. 58 of the sequence listing corresponds to sequence no. 57 in the description.
Sequence no. 59 of the sequence listing corresponds to sequence no. 58 in the description.
Sequence no. 60 of the sequence listing corresponds to sequence no. 59 in the description.
Sequence no. 61 of the sequence listing corresponds to sequence no. 60 in the description.
Sequence no. 62 of the sequence listing corresponds to sequence no. 61 in the description.
Sequence no. 63 of the sequence listing corresponds to sequence no. 62 in the description.
Sequence no. 64 of the sequence listing corresponds to sequence no. 63 in the description.
Sequence no. 65 of the sequence listing corresponds to sequence no. 64 in the description.
Sequence no. 66 of the sequence listing corresponds to sequence no. 65 in the description.
Sequence no. 67 of the sequence listing corresponds to sequence no. 66 in the description.
Sequence no. 68 of the sequence listing corresponds to sequence no. 67 in the description.
Sequence no. 69 of the sequence listing corresponds to sequence no. 68 in the description.
Sequence no. 70 of the sequence listing corresponds to sequence no. 69 in the description.
Sequence no. 71 of the sequence listing corresponds to sequence no. 70 in the description.
Sequence no. 72 of the sequence listing corresponds to sequence no. 70 in the description because
this is the amino acid sequence attached to seq. no 70.
Sequence no. 73 of the sequence listing corresponds to sequence no. 71 in the description.
Sequence no. 74 of the sequence listing corresponds to sequence no. 71 in the description because
this is the amino acid sequence attached to seq. no 71.
Sequence no. 75 of the sequence listing corresponds to sequence no. 72 in the description.
Sequence no. 76 of the sequence listing corresponds to sequence no. 72 in the description because
this is the amino acid sequence attached to seq. no 72.
Sequence no. 77 of the sequence listing corresponds to sequence no. 73 in the description.
Sequence no. 78 of the sequence listing corresponds to sequence no. 73 in the description because
this is the amino acid sequence attached to seq. no 73.
Sequence no. 79 of the sequence listing corresponds to sequence no. 74 in the description.
Sequence no. 80 of the sequence listing corresponds to sequence no. 74 in the description because
this is the amino acid sequence attached to seq. no 74.
Sequence no. 81 of the sequence listing corresponds to sequence no. 75 in the description.
Sequence no. 82 of the sequence listing corresponds to sequence no. 75 in the description because
this is the amino acid sequence attached to seq. no 75.
Sequence no. 83 of the sequence listing corresponds to sequence no. 76 in the description.
Sequence no. 84 of the sequence listing corresponds to sequence no. 76 in the description because
this is the amino acid sequence attached to seq. no 76.

SEQUENCE LISTING

SEQ ID NO: 1

Part 1 (exon1 - partial intron2)
1 CCACTCCAAGCTCTGAGGCACCACAGGCTGCACAGGTAAGCGAGGACAGATGAAAAAGCC
61 CCAGGTGTTGCATGGGGCCAGACTGGGCCTAGGAGCGCAACATGGCGCCATGACGGCTGC
121 TCTCCCACCACAGGACACCATGGTGCGACGCGTGGCTGTGGTGGGCGCGGGCATCAGCGG
181 GTTGGCGGCCACCAAGTGCTGCCTGGAAGAGGGACTGGAGCCCACCTGCTTTGAGCAGAG
241 CGAGGACATCGGGGGGCTCTGGCGCTACACGTGAGGGGTTTGGGCTGCGTGGTCTTCTGG
301 CAATCTGGGGCTAGGGGAGAATCTGGGGGGCGGTGGCTGCGAGAGCCGCTCAAGTGGAG
361 GAACTGACCGTGTTAAAACTCATGATGAGGGGCAAAGACATCAGGTTAGCTCTGGGTGAT
421 GGCT

SEQ ID NO: 2

Part 2 (partial intron2 - intron8)
1 CCTTGAGAGCAATAAGCCTGCAATGTCCCCTGTGTGCTCCAGCTAAGCTGAGAAAAATAT
61 CACTTTGAGCTCTGCAAGTGATGTTTATAAGAAGCGAGGCTTGAAGTTGAAAATAACAGC
121 TGTGTGTGTGCCCAAGGTTAAGCCCAGACAACAACAGAACTGATCTTTCTACAGTGGTAA
181 ATGTAGAAGACATTATGCCAAGTAGAGGACTCTGAGGTTATTACAGGGATGTACTGTGTA
241 TGGCTCTTCAGGATGAGCCCACAAAGCCTGTGAGCACATGTGGAAGGTAAGCAGTGGGCT
301 GGTGATGACCCTCTGGGGTGCTCAGTGCCCCTTTTTCTGGGCTTGGTTGGTGAAGCTGAG
361 TGGAAGGACGGGCTGGGAATTACCTGGCCAGACAAGGAGGGCATAGAAAGCATGGAGGA
421 ACTGGGTTGCAAGTGAAGACATTGCTCTGAGAGCCAGACATTGTGGTTCAGGTCCCCAGA
481 ACTGAGGTTCTCCTAGTCGTGTTCTTGGGTCAGTTCTCCTGACCCAAAGTCATGACATTG
541 GGTGAGGTTATCTCCAGATTGGCTCAGCATCTTCTTGCCTGTGGCTTATGACTTTTTGGG
601 GTACCCATGACTGTGACCCCTCTAACCGAAGCTCTCTTCCTCCAGGAAAAGCCAGAGGAA
661 GGCAGAGCTAGCATCTACCGCACTGTCTTCACCAATTCCTGCAAGGAGATGATGTGCTAC
721 CCCGACTTCCCCTTCCCTGATGACTACCCCAACTACATACACAACGCACGGCTCCATAAG
781 TACATCCGTGACTACGCACAGCATTTTGACCTCCTCCGGCACATCCGCTTCAAGGTAGGA
841 GGGATGGACTTTGCTGCTGTGTCTCAGAGAAAATCCTGCGGCAGACATCCTGAGGGAAAG
901 TCTTGTTGGTTTTGTTAAATTGGGTCTCACCAGCTGCTGGTTGGGTAGTGTCAAATGATC
961 AGTCATCACGGGGAAACCATTGCTTCTGAGGGTGGAAAATCACGTGGAATGGAGGACAC
1021 CAGTGGGTTTTCTCTATCCAAAATAATTGCCAAGTCTGTGACCTTGTCTCTGGTGGGGAA
1081 AAGGACAGAGCCTGCCTGGCTGCAGTGGGGTACCCGGAGCACTTCCCAAACCTTTATCCC
1141 TTTCCTTTAAGACCACAGTCACCAAGATAAGGAAGCGCCCTGACTTCAGTGCTACGGGGC
1201 AGTGGGAGGTGGTTACACGGAGTGACGGGAAGGAAGAGGCAGCGGTTTTTGATGCTGTTA
1261 TGGTTTGCACTGGGCACCATGTCTACCCAAACCTCCCCCTTGCCCACTTCCCAGTAAGCT
1321 ATATGAACGTGATTCAGCCATCACACAGCCAGGTGTGCACTTTACATTCAAATGTTGGGT
1381 TTTGGTTTTTTTTTCAGCATCAAAGATTGGTTTTAGGGGTGAGCTCTGCTCAGGAAATC
1441 AAAACTTTTATTTAAGACAAAAGACTGTATCCAAACCATGTCAAAGCAGGGACATTCTTA
1501 CTGGAGGCAATGCTAATATTTCCTGGAAAAATTATTGCAGGGTTTCCATTTAGTAGAAGT
1561 TTCTTCACTAAAGGGTTTCGTAAAGCACTTCTTTTGGCATTTTTTCCATGGTTTCTAGTG
1621 CAAATGCTGTGCGCAAACTTTGCTCCTGCTTACAGGATCAGCAGTGGGAAAACTTCCTAT
1681 GATAAGAAGTATTTGGTGCTGGGGGTCTGTTTGGAGGAGGTATGGTCTTGGGACACTGAC
1741 TGGCTGGGAAAACTCCCATTGTGTCCTTTGTCACCTTCTGTTTTAGAAATTAAAGTTGCT
1801 TTCCCATCACTTGCTGGCGCAAAGGCACGAAGACACACCAAGCGGATGGAACGTGGCTGG
1861 TCTGGTGTCTCAGGGTGATGATGCTGAGTTGTATCTTTAAGGCACACAAATACCACACAA
1921 AGTTATCCTGTTCTCTCATGCTGGGGCTCCCCTCTGTTTAAATATTGAATGAATCCAGAG
1981 ACAAAAACCCATCTACAGGTTTGCAGCATCCCTCTGCTGTGAGCAACTCCCAGACATGCT
2041 CCAGCAGCGCCTGCAGGAATGGGAGCAGCCAAGGTTAACGCAGCCACACTTCTTCACTGA
2101 TGACTTACATCCCTTTCTGTCTTCCTGCTCCCAGGGGATAGAGAAGTTTAAAGGTTGCTA
2161 CTTACACAGCCGGGACTACAAGGAGCCAGAAAAGTTCAGAGGGAAGAAGGTGCTGGTGGT
2221 TGGCTTGGGCAACTCCGGCTGTGACATTGCTGTGGAGCTCAGCACCGTGGCCTCGCAGGT
2281 ATGGGCACCTGAACCAGGGCTGGTTGGAGCTGATGGCCGCCACGACGGTGGCTCAACCCT
2341 GCCTCACCATCCCCCCCCAGGTTTACCTGAGCTCCCGAAGAGGTTCCTGGGTGATGAGCC

2401 GTGTCTGGAACTTCGGCTACCCCTGGGACATGCTGCTCATCACCCGCTTCTGGACGTGGC
2461 TGGACAACTTTCTCCCCAAGGCAGTCAGTGACTGGCTCTATGTCAGGAGCATGAACCAGC
2521 AGTACAAACACGAGGATTTTGGCCTAATGCCGGTGGATGGAGTAAGCATGAGGCTATCTG
2581 TTCCCAAAGCCAGAAGTGCTGGTCCCAGGCAGGGTGGTATCAAGCCTGTACAGGGCCAGC
2641 AGCAGCAGCCAACTTAGGCTTTTTCCTTGCAGTCATCCCGCAGGGAACCGGTGTTGAATG
2701 ATGACATCCTGAGCCGCATCACCTGTGGTGTGGTGCTGATAAAGCCAAATGTGAAGGAAT
2761 TCAGAGAAACATCAGTCTTGTTTCAAGATGGGACTGTGCAGGACGACCTCGATGCGGTTA
2821 TCTTTGCCYCTGGTTACTCTCACTCCTTTCCTTTCATGGAGGATGAATCCATCATCGAAA
2881 GCAAGAACAACGAGGCTACCCTCTACAAATGCATTGTTCCTCCTAAGTTGGAGAAGCCAA
2941 CCATGGCAGTCATTGGATTGGTCCAGTCCTTTGGATCCGCCATCCCGACAGCAGATGTCC
3001 AGTGCCGCTGGGCAGTCAAGGTGTTTCAGGTAGGTGAATGAGCTAAGGTCCCTAAATGGG
3061 AAGTAGCATTTCCCATCAGCAATTTCACAGATTCCAACTAAAAATGAGGGCAGTGCATGA
3121 GTGCTGGAAATACCAGCAACGGGTTCTCCCCGGATGGGCTGCCTTGTGCCATGGAGAAT
3181 AGGTCACTTATCAACCAGCCGCAGATGTCAGTGAGGTCCCTGTGGTGAGGCAGCTATCTG
3241 GGCATCGCCACACATCCTAAAGGACAGGGGGATCTGTTTTATCGCAACCCTGATGCCCAG
3301 TTTGATCCAAGCTCAAGGGAAGAGAAGTGCCCAAGGTGATCCCTACAGACCCCACCTGCT
3361 GCAGGAGGGATCTCCTGGGCATCATCACCCTTTGCCGACACTCTTCCTGTTCTTTCCAGG
3421 GGGCTCTGCACTCTCCCCTCAGTCAACGAGATGCTGGAGGACATAGAGGAGAAGAAGAGG
3481 AATAAGATCAGATGGTAGGTGCAGCTCATGGGACGCTGCCCAGGAAGGAGGAGGAAAAAC
3541 CTTGCTGTCTTCTCAGCCTGGGTGGGCTGGATGTGGGCATGGGGAGGGCTGCAGCAGGGG
3601 CAAGCTCCTAGCAAGGAGACCATTCTGAACTGCTCCGTTCATGACCCAGATGAGCAAACC
3661 CAATGGACCAACACAGGAAGGTCATACCCAGCTTCTTTGGTGTCAGTTCTCAGGTTTCC
3721 ACCCGCCCCAGGGTGGTTGAATTGGGCAAATCTGTGCAGAAAATCTGTGATGGTAAGATT
3781 TCCTGTCTGAAAATGCTGCTTTGCAGCATTTGTAGAGTGTTATTGGTCTGGAAAAAACAC
3841 AGAAATTGAGGCCCCAGCAAAAACTGAACATTCCCTCATTCTTATTCTTCAGTAACATTT
3901 TTTTGTTTCATAAAGTACACAGAGGTCTTGTTTACCCCTCTTTGCTCGTATCTCACATTA
3961 TAGTGAAGCAATACTTTGCCAATATGAGGACGGTCAAAGCTGAATCTGGCCCAAGCAATG
4021 TTTTCTGTTGTTTTTAGAAACTGTTGCGTTGCAGGAAGTTCCAAAGCACAGCGCTGCTGT
4081 GGGAGCCAGATCCCCTCACCACCACCAGCAGCAGTGATGTGTGGCCATGCCCCAGTCCAG
4141 CACCTCTCCCCAGTTTTAGGGGACAGCAGGGACAGGTGCCACCTTCCCTAGGCTGACAAG
4201 GGGCACATCATCCCCTTCTCAAGGCTCTGTGGGGGAGCAGATTTCCTGTCCCTTGCATTA
4261 ATGTCATGGGAAAAGATGGCTGCCAAGCACAGAAAGCTGTCTGTGTTCTCTGCCAAATCC
4321 TCGTGCTATTTTGACCCTGCACTGAATGTGTCTGAATTTAAGGAGGATTTCCTCCCTGCC
4381 CATCCTGGCTGCTGCTACAGGGTCCCCTCAAGCAGCAAGAACACAAGATGATGTCATGTC
4441 GAGAGAAATAAGGAGATCTAAAGCAGTCCCTCTCTTCTTCTACCCTCTCTCTCAAAG

SEQ ID NO: 3
AMINO ACID SEQUENCE

1 M V R R V A V V G A G I S G L A A T K C
1 ATGGTGCGACGCGTGGCTGTGGTGGGCGCGGGCATCAGCGGGTTGGCGGCCACCAAGTGC
21 C L E E G L E P T C F E Q S E D I G G L
61 TGCCTGGAAGAGGGACTGGAGCCCACCTGCTTTGAGCAGAGCGAGGACATCGGGGGGCTC
41 W R Y T E K P E E G R A S I Y R T V F T
121 TGGCGCTACACGGAAAAGCCAGAGGAAGGCAGAGCTAGCATCTACCGCACTGTCTTCACC
61 N S C K E M M C Y P D F P F P D D Y P N
181 AATTCCTGCAAGGAGATGATGTGCTACCCCGACTTCCCCTTCCCTGATGACTACCCCAAC
81 Y I H N A R L H K Y I R D Y A Q H F D L
241 TACATACACAACGCACGGCTCCATAAGTACATCCGTGACTACGCACAGCATTTTGACCTC
101 L R H I R F K T T V T K I R K R P D F S
301 CTCCGGCACATCCGCTTCAAGACCACAGTCACCAAGATAAGGAAGCGCCCTGACTTCAGT
121 A T G Q W E V V T R S D G K E E A A V F
361 GCTACGGGGCAGTGGGAGGTGGTTACACGGAGTGACGGGAAGGAAGAGGCAGCGGTTTTT
141 D A V M V C T G H H V Y P N L P L A H F
421 GATGCTGTTATGGTTTGCACTGGGCACCATGTCTACCCAAACCTCCCCCTTGCCCACTTC
161 P G I E K F K G C Y L H S R D Y K E P E

481 CCAGGGATAGAGAAGTTTAAAGGTTGCTACTTACACAGCCGGGACTACAAGGAGCCAGAA
181 K F R G K K V L V V G L G N S G C D I A
541 AAGTTCAGAGGGAAGAAGGTGCTGGTGGTTGGCTTGGGCAACTCCGGCTGTGACATTGCT
201 V E L S T V A S Q V Y L S S R R G S W V
601 GTGGAGCTCAGCACCGTGGCCTCGCAGGTTTACCTGAGCTCCCGAAGAGGTTCCTGGGTG
221 M S R V W N F G Y P W D M L L I T R F W
661 ATGAGCCGTGTCTGGAACTTCGGCTACCCCTGGGACATGCTGCTCATCACCCGCTTCTGG
241 T W L D N F L P K A V S D W L Y V R S M
721 ACGTGGCTGGACAACTTTCTCCCCAAGGCAGTCAGTGACTGGCTCTATGTCAGGAGCATG
261 N Q Q Y K H E D F G L M P V D G S S R R
781 AACCAGCAGTACAAACACGAGGATTTTGGCCTAATGCCGGTGGATGGATCATCCCGCAGG
281 E P V L N D D I L S R I T C G V V L I K
841 GAACCGGTGTTGAATGATGACATCCTGAGCCGCATCACCTGTGGTGTGGTGCTGATAAAG
301 P N V K E F R E T S V L F Q D G T V Q D
901 CCAAATGTGAAGGAATTCAGAGAAACATCAGTCTTGTTTCAAGATGGGACTGTGCAGGAC
321 D L D A V I F A S G Y S H S F P F M E D
961 GACCTCGATGCGGTTATCTTTGCCTCTGGTTACTCTCACTCCTTTCCTTTCATGGAGGAT
341 E S I I E S K N N E A T L Y K C I V P P
1021 GAATCCATCATCGAAAGCAAGAACAACGAGGCTACCCTCTACAAATGCATTGTTCCTCCT
361 K L E K P T M A V I G L V Q S F G S A I
1081 AAGTTGGAGAAGCCAACCATGGCAGTCATTGGATTGGTCCAGTCCTTTGGATCCGCCATC
381 P T A D V Q C R W A V K V F Q G L C T L
1141 CCGACAGCAGATGTCCAGTGCCGCTGGGCAGTCAAGGTGTTTCAGGGGCTCTGCACTCTC
401 P S V N E M L E D I E E K K R N K I R W
1201 CCCTCAGTCAACGAGATGCTGGAGGACATAGAGGAGAAGAAGAGGAATAAGATCAGATGG

SEQ ID NO: 4
CTTTGCTCTTGGCAGGAAAC

SEQ ID NO: 5
AGCCATCACCCAGAGCTAAC

SEQ ID NO: 6
CCTGGATTTTGTTCCCTTGA

SEQ ID NO: 7
TGGTGAAGACAGTGCGGTAG

SEQ ID NO: 8
TTTGGGGTACCCATGACTGT

SEQ ID NO: 9
TTTTCCCCACCAGAGACAAG

SEQ ID NO: 10
TGTGATGGCTGAATCACGTT

SEQ ID NO: 11
TGCTGGTTGGGTAGTGTCAA

SEQ ID NO: 12
GCACCATGTCTACCCAAACC

SEQ ID NO: 13
ACCAGCACCTTCTTCCCTCT

SEQ ID NO: 14
TGGCTTTGGGAACAGATAGC

SEQ ID NO: 15
CTGTGAGCAACTCCCAGACA

SEQ ID NO: 16
TGTCTGGAACTTCGGCTACC

SEQ ID NO: 17
GATCCAAAGGACTGGACCAA

SEQ ID NO: 18
CAGGGTGGTATCAAGCCTGT

SEQ ID NO: 19
TCTCTTCCCTTGAGCTTGGA

SEQ ID NO: 20
CTTATCAACCAGCCGCAGAT

SEQ ID NO: 21
GATTTGCCCAATTCAACCAC

SEQ ID NO: 22
GCACTCTCCCCTCAGTCAAC

SEQ ID NO: 23
TCTGCAGCGTGTTGCTTATC

SEQ ID NO: 24
CCTTTAGAGCTGACCCTCCA

SEQ ID NO: 25
ATAGTTGGGTGCTGGTCGAG

SEQ ID NO: 26
TGCTGTGAGAGAGGGTTGTG

SEQ ID NO: 27
ACGCTAGCTGTCATCGGTTT

SEQ ID NO: 28
TGGTGGGTCTTGCTAACCTT

SEQ ID NO: 29
CAGGGGGAAGGTGTAGGTTT

SEQ ID NO: 30
TTGAGAATGCCCAGAGTGTG

SEQ ID NO: 31
AAAGCGCTGTTTTGCTGAT

SEQ ID NO: 32
GGAGGTGACCCACTGTCACT

SEQ ID NO: 33
TGGAGAGCAAAGGCAAAACT

SEQ ID NO: 34
CTGACTGACTGA- GGACTGGAGCCCACCTG

SEQ ID NO: 35
TTTTTTTTTTTTTTTT-GCTGCGAGAGCCGCTCAA

SEQ ID NO: 36
TTTTTTTTTTTTTTTTT- GGCATAGAAAGCATGGGAGGAA

SEQ ID NO: 37
TTTTTTTTTTTTTTTTTTTTTTTTT-CCCCGACTTCCCCTTCCC

SEQ ID NO: 38
TTTTTTTTTTTTTTTTTTTTTTTTTTTTT-CTGGTTGGAGCTGATGGCC

SEQ ID NO: 39
TTTTTTTTTTTTTTTTTTTTTTTTTTTTTTTT-TTACCTGAGCTCCCGAAGAGG

SEQ ID NO: 40
TTTTTTTTTTTTTTTTTTTTTTTTTTTTTTTTTT-GAAGTAGCATTTCCCATCAGCAATT

SEQ ID NO: 41
TTTTTTT-GGAGCATCACACACCTTTTGAC

SEQ ID NO: 42
TTTTTTTTTTT-TTCAACCCATTCCATGCTCCCAG

SEQ ID NO: 43
TTTTTTTTTTTTTTTT-CAATATCACAGCCGGTGTTCCCA

SEQ ID NO: 44
TTTTTTTTTTTTTTTTTTTTTTT-TGAGAAGCGAGCAGGGCTGTT

SEQ ID NO: 45
TTTTTTTTTTTTTTTTTTTTTTTTTT-GCTTTACAGTGAGTGAGCTCTGC

SEQ ID NO: 46
TTTTTTTTTTTTTTTTTTTTTTTTTTTTTTTTTTTT-AGCATCAGCTGGGCTGGC

SEQ ID NO: 47
TTTTTTTTTTTTTTTTTTTTTTTTTTTTTTTTTTTTTTTTTT-CGGGGCCCTGAACCAAGG

SEQ ID NO: 48
TTTTTTTTTTTTTTTTTTTTTTTTTTTTTTTTTTTTTTTTTTTTT-GGCTACCCCATTGACACCATC

SEQ ID NO: 49
>exon1
CCACTCCAAGCTCTGAGGCACCACAGGCTGCACAGG

SEQ ID NO: 50
>intron1
TAAGCGAGGACAGATGAAAAAGCCCCAGGTGTTGCATGGGGCCAGACTGGGCCTAGGAGC
GCAACATGGCGCCATGACGGCTGCTCTCCCAC

SEQ ID NO: 51
>exon2

CACAGGACACCATGGTGCGACGCGTGGCTGTGGTGGGCGCGGGCATCAGCGGTTGGCGG
CCACCAAGTGCTGCCTGGAAGAGGGACTGGAGCCCACCTGCTTTGAGCAGAGCGAGGACA
TCGGGGGGCTCTGGCGCTACACG

SEQ ID NO: 52
>intron2A (partly)
TGAGGGGTTTGGGCTGCGTGGTCTTCTGGCAATCTGGGGCTAGGGGAGAATCTGGGGGGG
CGGTGGCTGCGAGAGCCGCTCAAGTGGAGGAACTGACCGTGTTAAAACTCATGATGAGGG
GCAAAGACATCAGGTTAGCTCTGGGTGATGGCT

SEQ ID NO: 53
>intron2B (partly)
CCTTGAGAGCAATAAGCCTGCAATGTCCCCTGTGTGCTCCAGCTAAGCTGAGAAAAATAT
CACTTTGAGCTCTGCAAGTGATGTTTATAAGAAGCGAGGCTTGAAGTTGAAAATAACAGC
TGTGTGTGTGCCCAAGGTTAAGCCCAGACAACAACAGAACTGATCTTTCTACAGTGGTAA
ATGTAGAAGACATTATGCCAAGTAGAGGACTCTGAGGTTATTACAGGGATGTACTGTGTA
TGGCTCTTCAGGATGAGCCCACAAAGCCTGTGAGCACATGTGGAAGGTAAGCAGTGGGCT
GGTGATGACCCTCTGGGGTGCTCAGTGCCCCTTTTTCTGGGCTTGGTTGGTGAAGCTGAG
TGGAAGGACGGGCTGGGAATTACCTGGCCAGACAAGGAGGGCATAGAAAGCATGGGAGGA
ACTGGGTTGCAAGTGAAGACATTGCTCTGAGAGCCAGACATTGTGGTTCAGGTCCCCAGA
ACTGAGGTTCTCCTAGTCGTGTTCTTGGGTCAGTTCTCCTGACCCAAAGTCATGACATTG
GGTGAGGTTATCTCCAGATTGGCTCAGCATCTTCTTGCCTGTGGCTTATGACTTTTTGGG
GTACCCATGACTGTGACCCCTCTAACCGAAGCTCTCTTCCTCCAG

SEQ ID NO: 54
>exon3
GAAAAGCCAGAGGAAGGCAGAGCTAGCATCTACCGCACTGTCTTCACCAATTCCTGCAAGG
AGATGATGTGCTACCCCGACTTCCCCTTCCCTGATGACTACCCCAACTACATACACAACGC
ACGGCTCCATAAGTACATCCGTGACTACGCACAGCATTTTGACCTCCTCCGGCACATCCGC
TTCAAG

SEQ ID NO: 55
>intron3
GTAGGAGGGATGGACTTTGCTGCTGTGTCTCAGAGAAAATCCTGCGGCAGACATCCTGAGG
GAAAGTCTTGTTGGTTTTGTTAAATTGGGTCTCACCAGCTGCTGGTTGGGTAGTGTCAAAT
GATCAGTCATCACGGGGAAACCATTGCTTCTGAGGGTGGAAAATCACGTGGAATGGGAGGA
CACCAGTGGGTTTTCTCTATCCAAAATAATTGCCAAGTCTGTGACCTTGTCTCTGGTGGGG
AAAAGGACAGAGCCTGCCTGGCTGCAGTGGGGTACCCGGAGCACTTCCCAAACCTTTATCC
CTTTCCTTTAAG

SEQ ID NO: 56
>exon4
ACCACAGTCACCAAGATAAGGAAGCGCCCTGACTTCAGTGCTACGGGGCAGTGGGAGGTGG

TTACACGGAGTGACGGGAAGGAAGAGGCAGCGGTTTTTGATGCTGTTATGGTTTGCACTGG
GCACCATGTCTACCCAAACCTCCCCCTTGCCCACTTCCCA

SEQ ID NO: 57
>intron4
GTAAGCTATATGAACGTGATTCAGCCATCACACAGCCAGGTGTGCACTTTACATTCAAATG
TTGGGTTTTGGTTTTTTTTTCAGCATCAAAGATTGGTTTAGGGGTGAGCTCTGCTCAGG
AAATCAAAACTTTTATTTAAGACAAAAGACTGTATCCAAACCATGTCAAAGCAGGGACATT
CTTACTGGAGGCAATGCTAATATTTCCTGGAAAAATTATTGCAGGGTTTCCATTTAGTAGA
AGTTTCTTCACTAAAGGGTTTCGTAAAGCACTTCTTTTGGCATTTTTTCCATGGTTTCTAG
TGCAAATGCTGTGCGCAAACTTTGCTCCTGCTTACAGGATCAGCAGTGGGAAAACTTCCTA
TGATAAGAAGTATTTGGTGCTGGGGGTCTGTTTGGAGGAGGTATGGTCTTGGGACACTGAC
TGGCTGGGAAAACTCCCATTGTGTCCTTTGTCACCTTCTGTTTTAGAAATTAAAGTTGCTT
TCCCATCACTTGCTGGCGCAAAGGCACGAAGACACACCAAGCGGATGGAACGTGGCTGGTC
TGGTGTCTCAGGGTGATGATGCTGAGTTGTATCTTTAAGGCACACAAATACCACACAAAGT
TATCCTGTTCTCTCATGCTGGGGCTCCCCTCTGTTTAAATATTGAATGAATCCAGAGACAA
AAACCCATCTACAGGTTTGCAGCATCCCTCTGCTGTGAGCAACTCCCAGACATGCTCCAGC
AGCGCCTGCAGGAATGGGAGCAGCCAAGGTTAACGCAGCCACACTTCTTCACTGATGACTT
ACATCCCTTTCTGTCTTCCTGCTCCCAG

SEQ ID NO: 58
>exon5
GGGATAGAGAAGTTTAAAGGTTGCTACTTACACAGCCGGGACTACAAGGAGCCAGAAAAGT
TCAGAGGGAAGAAGGTGCTGGTGGTTGGCTTGGGCAACTCCGGCTGTGACATTGCTGTGGA
GCTCAGCACCGTGGCCTCGCAG

SEQ ID NO: 59
>intron5
GTATGGGGACCTGAACCAGGGCTGGTTGGAGCTGATGGCCGCCACGACGGTGGCTCAACCCT
GCCTCACCATCCCCCCCCAG

SEQ ID NO: 60
>exon6
GTTTACCTGAGCTCCCGAAGAGGTTCCTGGGTGATGAGCCGTGTCTGGAACTTCGGCTACCC
CTGGGACATGCTGCTCATCACCCGCTTCTGGACGTGGCTGGACAACTTTCTCCCCAAGGCAG
TCAGTGACTGGCTCTATGTCAGGAGCATGAACCAGCAGTACAAACACGAGGATTTTGGCCTA
ATGCCGGTGGATGGA

SEQ ID NO: 61
>intron6
GTAAGCATGAGGCTATCTGTTCCCAAAGCCAGAAGTGCTGGTCCCAGGCAGGGTGGTATCAA
GCCTGTACAGGGCCAGCAGCAGCAGCCAACTTAGGCTTTTCCTTGCAG

SEQ ID NO: 62
>exon7
TCATCCCGCAGGGAACCGGTGTTGAATGATGACATCCTGAGCCGCATCACCTGTGGTGTGG
TGCTGATAAAGCCAAATGTGAAGGAATTCAGAGAAACATCAGTCTTGTTTCAAGATGGGACT
GTGCAGGACGACCTCGATGCGGTTATCTTTGCCYCTGGTTACTCTCACTCCTTTCCTTTCAT
GGAGGATGAATCCATCATCGAAAGCAAGAACAACGAGGCTACCCTCTACAAATGCATTGTTC
CTCCTAAGTTGGAGAAGCCAACCATGGCAGTCATTGGATTGGTCCAGTCCTTTGGATCCGCC
ATCCCGACAGCAGATGTCCAGTGCCGCTGGGCAGTCAAGGTGTTTCAG

SEQ ID NO: 63
>intron7
GTAGGTGAATGAGCTAAGGTCCCTAAATGGGAAGTAGCATTTCCCATCAGCAATTTCACAGA
TTCCAACTAAAAATGAGGGCAGTGCATGAGTGCTGGAAATACCAGCAACGGGTTCTCCCCGG
GATGGGCTGCCTTGTGCCATGGAGAATAGGTCACTTATCAACCAGCCGCAGATGTCAGTGAG

GTCCCTGTGGTGAGGCAGCTATCTGGGCATCGCCACACATCCTAAAGGACAGGGGGATCTGT
TTTATCGCAACCCTGATGCCCAGTTTGATCCAAGCTCAAGGGAAGAGAAGTGCCCAAGGTGA
TCCCTACAGACCCCACCTGCTGCAGGAGGGATCTCCTGGGCATCATCACCCTTTGCCGACAC
TCTTCCTGTTCTTTCCAGG

SEQ ID NO: 64
>exon8
GGGCTCTGCACTCTCCCCTCAGTCAACGAGATGCTGGAGGACATAGAGGAGAAGAAGAGGAA
TAAGATCAGATGG

SEQ ID NO: 65
>intron8
TAGGTGCAGCTCATGGGACGCTGCCCAGGAAGGAGGAGGAAAAACCTTGCTGTCTTCTCAG
CCTGGGTGGGCTGGATGTGGGCATGGGGAGGGCTGCAGCAGGGGCAAGCTCCTAGCAAGGAG
ACCATTCTGAACTGCTCCGTTCATGACCCAGATGAGCAAACCCAATGGGACCAACACAGGAA
GGTCATACCCAGCTTCTTTGGTGTCAGTTCTCAGGTTTCCACCCGCCCCAGGGTGGTTGAAT
TGGGCAAATCTGTGCAGAAAATCTGTGATGGTAAGATTTCCTGTCTGAAAATGCTGCTTTGC
AGCATTTGTAGAGTGTTATTGGTCTGGAAAAAACACAGAAATTGAGGCCCCAGCAAAAACTG
AACATTCCCTCATTCTTATTCTTCAGTAACATTTTTTTGTTTCATAAAGTACACAGAGGTCT
TGTTTACCCCTCTTTGCTCGTATCTCACATTATAGTGAAGCAATACTTTGCCAATATGAGGA
CGGTCAAAGCTGAATCTGGCCCAAGCAATGTTTTCTGTTGTTTTAGAAACTGTTGCGTTGC
AGGAAGTTCCAAAGCACAGCGCTGCTGTGGGAGCCAGATCCCCTCACCACCACCAGCAGCAG
TGATGTGTGGCCATGCCCCAGTCCAGCACCTCTCCCCAGTTTTAGGGGACAGCAGGGACAGG
TGCCACCTTCCCTAGGCTGACAAGGGGCACATCATCCCCTTCTCAAGGCTCTGTGGGGGAGC
AGATTTCCTGTCCCTTGCATTAATGTCATGGGAAAAGATGGCTGCCAAGCACAGAAAGCTGT
CTGTGTTCTCTGCCAAATCCTCGTGCTATTTTGACCCTGCACTGAATGTGTCTGAATTTAAG
GAGGATTTCCTCCCTGCCCATCCTGGCTGCTGCTACAGGGTCCCCTCAAGCAGCAAGAACAC
AAGATGATGTCATGTCGAGAGAAATAAGGAGATCTAAAGCAGTCCCTCTCTTCTTCTACCCT
CTCTCTCAAAG

SEQ ID NO: 66
ACGGTCAAAGCTGAATCTGG

SEQ ID NO: 67
TCCCGAACCTTTGAGAGAGA

SEQ ID NO: 68
ACGGTCAAAGCTGAATCTGGCCCAAGCAATGTTTTCTGTTGTTTTAGAAACTGTTGCGT 60
TGCAGGAAGTTCCAAAGCACAGCGCTGCTGTGGGAGCCAGATCCCCTCACCACCACCAGC 120
AGCAGTGATGTGTGGCCATGCCCCAGTCCAGCACCTCTCCCCAGTTTTAGGGGACAGCAG 180
GGACAGGTGCCACCTTCCCTAGGCTGACAAGGGGCACATCATCCCCTTCTCAAGGCTCTG 240
TGGGGGAGCAGATTTCCTGTCCCTTGCATTAATGTCATGGGAAAAGATGGCTGCCAAGCA 300
CAGAAAGCTGTCTGTGTTCTCTGCCAAATCCTCGTGCTATTTTGACCCTGCACTGAATGT 360
GTCTGAATTTAAGGAGGATTTCCTCCCTGCCCATCCTGGCTGCTGCTACAGGGTCCCCTC 420
AAGCAGCAAGAACACAAGATGATGTCATGTCGAGAGAAATAAGGAGATCTAAAGCAGTCC 480
CTCTCTTCTTCTACCCTCTCTCTCAAAGTTCGGG

SEQ ID NO: 69

EXON SEQUENCE

1 CCACTCCAAGCTCTGAGGCACCACAGGCTGCACAGGCACAGGACACCATGGTGCGACGCG
61 TGGCTGTGGTGGGCGCGGGCATCAGCGGGTTGGCGGCCACCAAGTGCTGCCTGGAAGAGG
121 GACTGGAGCCCACCTGCTTTGAGCAGAGCGAGGACATCGGGGGGCTCTGGCGCTACACGG
181 AAAAGCCAGAGGAAGGCAGAGCTAGCATCTACCGCACTGTCTTCACCAATTCCTGCAAGG
241 AGATGATGTGCTACCCCGACTTCCCCTTCCCTGATGACTACCCCAACTACATACACAACG

301 CACGGCTCCATAAGTACATCCGTGACTACGCACAGCATTTTGACCTCCTCCGGCACATCC
361 GCTTCAAGACCACAGTCACCAAGATAAGGAAGCGCCCTGACTTCAGTGCTACGGGGCAGT
421 GGGAGGTGGTTACACGGAGTGACGGGAAGGAAGAGGCAGCGGTTTTGATGCTGTTATGG
481 TTTGCACTGGGCACCATGTCTACCCAAACCTCCCCCTTGCCCACTTCCCAGGGATAGAGA
541 AGTTTAAAGGTTGCTACTTACACAGCCGGGACTACAAGGAGCCAGAAAAGTTCAGAGGGA
601 AGAAGGTGCTGGTGGTTGGCTTGGGCAACTCCGGCTGTGACATTGCTGTGGAGCTCAGCA
661 CCGTGGCCTCGCAGGTTTACCTGAGCTCCCGAAGAGGTTCCTGGGTGATGAGCCGTGTCT
721 GGAACTTCGGCTACCCCTGGGACATGCTGCTCATCACCCGCTTCTGGACGTGGCTGGACA
781 ACTTTCTCCCCAAGGCAGTCAGTGACTGGCTCTATGTCAGGAGCATGAACCAGCAGTACA
841 AACACGAGGATTTTGGCCTAATGCCGGTGGATGGATCATCCCGCAGGGAACCGGTGTTGA
901 ATGATGACATCCTGAGCCGCATCACCTGTGGTGTGGTGCTGATAAAGCCAAATGTGAAGG
961 AATTCAGAGAAACATCAGTCTTGTTTCAAGATGGACTGTGCAGGACGACCTCGATGCGG
1021 TTATCTTTGCCTCTGGTTACTCTCACTCCTTTCCTTTCATGGAGGATGAATCCATCATCG
1081 AAAGCAAGAACAACGAGGCTACCCTCTACAAATGCATTGTTCCTCCTAAGTTGGAGAAGC
1141 CAACCATGGCAGTCATTGGATTGGTCCAGTCCTTTGGATCCGCCATCCCGACAGCAGATG
1201 TCCAGTGCCGCTGGGCAGTCAAGGTGTTTCAGGGGCTCTGCACTCTCCCCTCAGTCAACG
1261 AGATGCTGGAGGACATAGAGGAGAAGAAGAGGAATAAGATCAGATGG

SEQ ID NO: 70
>exon2

1 M V R R V A V V G A G I S G L A A T K C
1 ATGGTGCGACGCGTGGCTGTGGTGGGCGCGGGCATCAGCGGGTTGGCGGCCACCAAGTGC

21 C L E E G L E P T C F E Q S E D I G G L
61 TGCCTGGAAGAGGGACTGGAGCCCACCTGCTTTGAGCAGAGCGAGGACATCGGGGGGCTC

41 W R Y T
121 TGGCGCTACACG

SEQ ID NO: 71
>exon3

45 E K P E E G R A S I Y R T V F T N S C K
133 GAAAAGCCAGAGGAAGGCAGAGCTAGCATCTACCGCACTGTCTTCACCAATTCCTGCAAG

65 E M M C Y P D F P F P D D Y P N Y I H N
193 GAGATGATGTGCTACCCCGACTTCCCCTTCCCTGATGACTACCCCAACTACATACACAAC

85 A R L H K Y I R D Y A Q H F D L L R H I
253 GCACGGCTCCATAAGTACATCCGTGACTACGCACAGCATTTTGACCTCCTCCGGCACATC

105 R F K
313 CGCTTCAAG

SEQ ID NO: 72
>exon4

108 T T V T K I R K R P D F S A T G Q W E V
323 ACCACAGTCACCAAGATAAGGAAGCGCCCTGACTTCAGTGCTACGGGGCAGTGGGAGGTG

128 V T R S D G K E E A A V F D A V M V C T
383 GTTACACGGAGTGACGGGAAGGAAGAGGCAGCGGTTTTTGATGCTGTTATGGTTTGCACT

148 G H H V Y P N L P L A H F P

443 GGGCACCATGTCTACCCAAACCTCCCCCTTGCCCACTTCCCA

SEQ ID NO: 73
>exon5

162 G I E K F K G C Y L H S R D Y K E P E K
485 GGGATAGAGAAGTTTAAAGGTTGCTACTTACACAGCCGGGACTACAAGGAGCCAGAAAAG

182 F R G K K V L V V G L G N S G C D I A V
545 TTCAGAGGGAAGAAGGTGCTGGTGGTTGGCTTGGGCAACTCCGGCTGTGACATTGCTGTG

202 E L S T V A S Q
605 GAGCTCAGCACCGTGGCCTCGCAG

SEQ ID NO: 74
>exon6

210 V Y L S S R R G S W V M S R V W N F G Y
629 GTTTACCTGAGCTCCCGAAGAGGTTCCTGGGTGATGAGCCGTGTCTGGAACTTCGGCTAC

230 P W D M L L I T R F W T W L D N F L P K
689 CCCTGGGACATGCTGCTCATCACCCGCTTCTGGACGTGGCTGGACAACTTTCTCCCCAAG

250 A V S D W L Y V R S M N Q Q Y K H E D F
749 GCAGTCAGTGACTGGCTCTATGTCAGGAGCATGAACCAGCAGTACAAACACGAGGATTTT

270 G L M P V D G
809 GGCCTAATGCCGGTGGATGGA

SEQ ID NO: 75
>exon7

277 S S R R E P V L N D D I L S R I T C G V
830 TCATCCCGCAGGGAACCGGTGTTGAATGATGACATCCTGAGCCGCATCACCTGTGGTGTG

297 V L I K P N V K E F R E T S V L F Q D G
890 GTGCTGATAAAGCCAAATGTGAAGGAATTCAGAGAAACATCAGTCTTGTTTCAAGATGGG

317 T V Q D D L D A V I F A S G Y S H S F P
950 ACTGTGCAGGACGACCTCGATGCGGTTATCTTTGCCTCTGGTTACTCTCACTCCTTTCCT

337 F M E D E S I I E S K N N E A T L Y K C
1010 TTCATGGAGGATGAATCCATCATCGAAAGCAAGAACAACGAGGCTACCCTCTACAAATGC

357 I V P P K L E K P T M A V I G L V Q S F
1070 ATTGTTCCTCCTAAGTTGGAGAAGCCAACCATGGCAGTCATTGGATTGGTCCAGTCCTTT

377 G S A I P T A D V Q C R W A V K V F Q
1160 GGATCCGCCATCCCGACAGCAGATGTCCAGTGCCGCTGGGCAGTCAAGGTGTTTCAG

SEQ ID NO: 76
>exon8

396 G L C T L P S V N E M L E D I E E K K R
1217 GGGCTCTGCACTCTCCCCTCAGTCAACGAGATGCTGGAGGACATAGAGGAGAAGAAGAGG

416 N K I R W
1277 AATAAGATCAGATGG

## Claims

1. An isolated nucleic acid sequence corresponding to the genomic region encoding the FM03 enzyme of a bird, or a complement thereof, said nucleic acid sequence further comprising at least one mutation resulting from a single nucleotide polymorphism (SNP) associated with the predisposition of said bird to produce fishy taint eggs, wherein said at least one single nucleotide polymorphism (SNP) is at least one of the group of single nucleotide polymorphisms (SNPs) SNP 1 to SNP 39 as shown in Table 1 or an equivalent thereof.

2. Nucleic acid sequence according to claim 1, wherein said at least one single nucleotide polymorphism (SNP) is SNP25 and/or SNP36 as shown in Table 1.

3. Nucleic acid sequence according to claim 1 or 2, wherein said nucleic acid sequence comprises essentially only exonic sequences of said genomic region.

4. Nucleic acid sequence according to any one of the preceding claims, wherein said nucleic acid is DNA.

5. Nucleic acid sequence according to any one of the preceding claims, wherein said nucleic acid sequence is selected from the group consisting of SEQ ID NOs: 1, 2, 51, 52, 53, 54, 55, 57, 58, 59, 60, 62, 63, 65, 68 and 69.

6. Nucleic acid sequence according to any one of the preceding claims, wherein said nucleic acid sequence is the sequence shown in SEQ ID NO: 69.

7. A polypeptide, or a part thereof, encoded by a nucleic acid sequence according to any one of the preceding claims.

8. A polypeptide comprising a sequence that has at least 85% identity with the polypeptide sequence shown in SEQ ID NO: 3.

9. Polypeptide according to claim 7 or 8, wherein said polypeptide is a functionally altered mutant of the FMO3 enzyme of chicken.

10. A polypeptide comprising a sequence that has at least 85% identity with the polypeptide sequence shown in SEQ ID NO: 75.

11. Polypeptide according to any one of claims 7 to 10, comprising the T329S mutation caused by SNP25.

12. A set of primers suitable for amplifying a nucleic acid sequence according to any one of claims 1 to 6, comprising at least one primer selected from the group consisting of:
- a specific fragment of a nucleic acid sequence shown in SEQ ID NO: 1 or 2;
- a nucleic acid fragment which specifically hybridizes under stringent conditions with a nucleic acid sequence shown in SEQ ID NO: 1 or 2, and
- SEQ ID NOs: 4 - 48, 66 and 67.

13. An oligonucleotide probe suitable for specifically detecting a nucleic acid sequence according to any one of claims 1 to 6, selected from the group consisting of:
- a specific fragment of a nucleic acid sequence shown in SEQ ID NO: 1 or 2, wherein said fragment extends over a region of said nucleic acid comprising at least one mutation resulting from a single nucleotide polymorphism (SNP) selected from the group consisting of the single nucleotide polymorphisms (SNPs) SNP 1 to SNP 39 as shown in Table 1, and
- a nucleic acid fragment which specifically hybridizes under stringent conditions to a target region of a nucleic acid sequence shown in SEQ ID NO: 1 or 2, wherein said target region comprises at least one mutation resulting from a single nucleotide polymorphism (SNP) selected from the group consisting of the single nucleotide polymorphisms (SNPs) SNP 1 to SNP 39 as shown in Table 1.

14. A method for detecting a bird with a predisposition for producing fishy taint eggs, comprising providing a female bird or an egg of said bird, obtaining a nucleic acid sample of said bird or of said egg and determining in said nucleic acid sample the presence of at least one mutation in the genomic region encoding the FM03 enzyme, wherein said mutation results from at least one single nucleotide polymorphism (SNP) of the group of single nucleotide polymorphisms (SNPs) SNP 1 to SNP 39 as shown in Table 1 or an equivalent thereof.

15. Method according to claim 14, wherein the homozygous presence of said at least one mutation is determined.

16. A method for selecting a candidate breeder bird for use in bird breeding, comprising providing a candidate breeder bird, preferably a male bird, obtaining a nucleic acid sample of said bird, and screening said nucleic acid sample for the absence of at least one mutation in the genomic region encoding the FM03 enzyme, wherein said mutation results from at least one single nucleotide polymorphism (SNP) of the group of single nucleotide polymorphisms (SNPs) SNP 1 to SNP 39 as shown in Table 1 or an equivalent thereof.

17. Method according to claim 5, wherein the homozygous absence of said at least one mutation is determined.

18. Method according to any one of claims 14 to 17, wherein said at least one mutation is the missense mutation T329S in exon 7 or the missense mutation L30P in the predicted exon of intron 8.

19. A method of producing a bird breeding line, said method comprising selecting a candidate breeder bird according to a method of any one of claims 16 to 18, and crossing said bird with an other bird.

20. Method according to claim 19, wherein said candidate breeder bird and/or said other bird is a bird from a pure breeding line.

21. Method according to claim 19 or 20, said method further comprising subsequent steps to provide for a homozygous breeding line.

22. Method according to anyone of claims 14 to 21, wherein said bird is a poultry bird, preferably a chicken.

23. A method of testing bird feed for its propensity to give rise to fishy taint eggs, said method comprising providing a female bird, said female bird being homozygous for at least one mutation in the genomic region encoding the FM03 enzyme, providing a test feed, feeding said bird with test feed and determining the presence or absence of a fishy taint in at least one egg obtained from said bird.

24. Method according to claim 23, wherein said bird is a poultry bird, preferably a chicken.

25. Method according to claim 23 or 24, wherein said mutation is the missense mutation T329S in exon 7 or the missense mutation L30P in the predicted exon of intron 8.

26. A method of producing bird feed, comprising testing said bird feed according to a method of any one of claims 12 to 14, and selecting said feed which essentially gives no rise to fishy taint eggs.

27. A method for detecting a fishy taint egg, said method comprising
- isolating DNA from said egg and determining in said DNA the presence of at least one mutation in the genomic region encoding the FM03 enzyme, wherein said mutation results from at least one single nucleotide polymorphism (SNP) of the group of single nucleotide polymorphisms (SNPs) SNP 1 to SNP 39 as shown in Table 1 or an equivalent, preferably the missense mutations T329S in exon 7 or the missense mutation L30P in the predicted exon in intron 8, or
- detecting in said egg the presence of a polypeptide according to claim 9.

28. A method for detecting a bird with a predisposition for producing fishy taint eggs, comprising providing a female bird, obtaining a protein sample of said bird and determining in said protein sample the presence of a polypeptide according to claim 9.


SEQUENCE LISTING

<110> Hendrix Poultry Breeders B.V.

<120> FMO gene polymorphisms

<130> P70434EP00

<140> 04077506.6
<141> 2004-09-10

<160> 84

<170> PatentIn Ver. 2.1

<210> 1
<211> 424
<212> DNA
<213> Gallus gallus

<220>
<223> /note="FMO3 gene exon 1 - partial intron 2"

<400> 1
ccactccaag ctctgaggca ccacaggctg cacaggtaag cgaggacaga tgaaaaagcc 60
ccaggtgttg catggggcca gactgggcct aggagcgcaa catggcgcca tgacggctgc 120
tctcccacca caggacacca tggtgcgacg cgtggctgtg gtgggcgcgg gcatcagcgg 180
gttggcggcc accaagtgct gcctggaaga gggactggag cccacctgct ttgagcagag 240
cgaggacatc ggggggctct ggcgctacac gtgaggggtt tgggctgcgt ggtcttctgg 300
caatctgggg ctaggggaga atctgggggg gcggtggctg cgagagccgc tcaagtggag 360
gaactgaccg tgttaaaact catgatgagg ggcaaagaca tcaggttagc tctgggtgat 420
ggct 424

<210> 2
<211> 4497
<212> DNA
<213> Gallus gallus

<220>
<223> /note="FMO3 gene partial intron 2 - intron 8"

<400> 2
ccttgagagc aataagcctg caatgtcccc tgtgtgctcc agctaagctg agaaaaatat 60
cactttgagc tctgcaagtg atgtttataa gaagcgaggc ttgaagttga aaataacagc 120
tgtgtgtgtg cccaaggtta agcccagaca acaacagaac tgatctttct acagtggtaa 180
atgtagaaga cattatgcca agtagaggac tctgaggtta ttacagggat gtactgtgta 240
tggctcttca ggatgagccc acaaagcctg tgagcacatg tggaaggtaa gcagtgggct 300
ggtgatgacc ctctggggtg ctcagtgccc ctttttctgg gcttggttgg tgaagctgag 360
tggaaggacg ggctgggaat tacctggcca gacaaggagg gcatagaaag catgggagga 420
actgggttgc aagtgaagac attgctctga gagccagaca ttgtggttca ggtccccaga 480
actgaggttc tcctagtcgt gttcttgggt cagttctcct gacccaaagt catgacattg 540
ggtgaggtta tctccagatt ggctcagcat cttcttgcct gtggcttatg actttttggg 600
gtacccatga ctgtgacccc tctaaccgaa gctctcttcc tccaggaaaa gccagaggaa 660
ggcagagcta gcatctaccg cactgtcttc accaattcct gcaaggagat gatgtgctac 720
cccgacttcc ccttccctga tgactacccc aactacatac acaacgcacg gctccataag 780
tacatccgtg actacgcaca gcattttgac ctcctccggc acatccgctt caaggtagga 840
gggatggact ttgctgctgt gtctcagaga aaatcctgcg gcagacatcc tgagggaaag 900
tcttgttggt tttgttaaat tgggtctcac cagctgctgg ttgggtagtg tcaaatgatc 960
agtcatcacg gggaaaccat tgcttctgag ggtggaaaat cacgtggaat gggaggacac 1020
cagtgggttt tctctatcca aaataattgc caagtctgtg accttgtctc tggtggggaa 1080
aaggacagag cctgcctggc tgcagtgggg tacccggagc acttcccaaa cctttatccc 1140
tttcctttaa gaccacagtc accaagataa ggaagcgccc tgacttcagt gctacggggc 1200
agtgggaggt ggttacacgg agtgacggga aggaagaggc agcggttttt gatgctgtta 1260
tggtttgcac tgggcaccat gtctacccaa acctccccct tgcccacttc ccagtaagct 1320
atatgaacgt gattcagcca tcacacagcc aggtgtgcac tttacattca aatgttgggt 1380
tttggttttt tttttcagca tcaaagattg gttttagggg tgagctctgc tcaggaaatc 1440
aaaactttta tttaagacaa aagactgtat ccaaaccatg tcaaagcagg gacattctta 1500

ctggaggcaa tgctaatatt tcctggaaaa attattgcag ggtttccatt tagtagaagt 1560
ttcttcacta aagggtttcg taaagcactt cttttggcat tttttccatg gtttctagtg 1620
caaatgctgt gcgcaaactt tgctcctgct tacaggatca gcagtgggaa aacttcctat 1680
gataagaagt atttggtgct gggggtctgt ttggaggagg tatggtcttg ggacactgac 1740
tggctgggaa aactcccatt gtgtcctttg tcaccttctg ttttagaaat taaagttgct 1800
ttcccatcac ttgctggcgc aaaggcacga agacacacca agcggatgga acgtggctgg 1860
tctggtgtct cagggtgatg atgctgagtt gtatctttaa ggcacacaaa taccacacaa 1920
agttatcctg ttctctcatg ctggggctcc cctctgttta aatattgaat gaatccagag 1980
acaaaaaccc atctacaggt ttgcagcatc cctctgctgt gagcaactcc cagacatgct 2040
ccagcagcgc ctgcaggaat gggagcagcc aaggttaacg cagccacact tcttcactga 2100
tgacttacat ccctttctgt cttcctgctc ccaggggata gagaagttta aaggttgcta 2160
cttacacagc cgggactaca aggagccaga aaagttcaga gggaagaagg tgctggtggt 2220
tggcttgggc aactccggct gtgacattgc tgtggagctc agcaccgtgg cctcgcaggt 2280
atggggacct gaaccagggc tggttggagc tgatggccgc cacgacggtg gctcaaccct 2340
gcctcaccat ccccccccag gtttacctga gctcccgaag aggttcctgg gtgatgagcc 2400
gtgtctggaa cttcggctac ccctgggaca tgctgctcat cacccgcttc tggacgtggc 2460
tggacaactt tctccccaag gcagtcagtg actggctcta tgtcaggagc atgaaccagc 2520
agtacaaaca cgaggatttt ggcctaatgc cggtggatgg agtaagcatg aggctatctg 2580
ttcccaaagc cagaagtgct ggtcccaggc agggtggtat caagcctgta cagggccagc 2640
agcagcagcc aacttaggct ttttccttgc agtcatcccg cagggaaccg gtgttgaatg 2700
atgacatcct gagccgcatc acctgtggtg tggtgctgat aaagccaaat gtgaaggaat 2760
tcagagaaac atcagtcttg tttcaagatg ggactgtgca ggacgacctc gatgcggtta 2820
tctttgccyc tggttactct cactcctttc ctttcatgga ggatgaatcc atcatcgaaa 2880
gcaagaacaa cgaggctacc ctctacaaat gcattgttcc tcctaagttg gagaagccaa 2940
ccatggcagt cattggattg gtccagtcct ttggatccgc catcccgaca gcagatgtcc 3000
agtgccgctg ggcagtcaag gtgtttcagg taggtgaatg agctaaggtc cctaaatggg 3060
aagtagcatt tcccatcagc aatttcacag attccaacta aaaatgaggg cagtgcatga 3120
gtgctggaaa taccagcaac gggttctccc cgggatgggc tgccttgtgc catggagaat 3180
aggtcactta tcaaccagcc gcagatgtca gtgaggtccc tgtggtgagg cagctatctg 3240
ggcatcgcca cacatcctaa aggacagggg gatctgtttt atcgcaaccc tgatgcccag 3300
tttgatccaa gctcaaggga agagaagtgc ccaaggtgat ccctacagac cccacctgct 3360
gcaggaggga tctcctgggc atcatcaccc tttgccgaca ctcttcctgt tctttccagg 3420
gggctctgca ctctcccctc agtcaacgag atgctggagg acatagagga gaagaagagg 3480
aataagatca gatggtaggt gcagctcatg ggacgctgcc caggaaggag gaggaaaaac 3540
cttgctgtct tctcagcctg ggtgggctgg atgtgggcat ggggagggct gcagcagggg 3600
caagctccta gcaaggagac cattctgaac tgctccgttc atgacccaga tgagcaaacc 3660
caatgggacc aacacaggaa ggtcataccc agcttctttg gtgtcagttc tcaggtttcc 3720
acccgcccca gggtggttga attgggcaaa tctgtgcaga aaatctgtga tggtaagatt 3780
tcctgtctga aaatgctgct ttgcagcatt tgtagagtgt tattggtctg gaaaaaacac 3840
agaaattgag gccccagcaa aaactgaaca ttccctcatt cttattcttc agtaacattt 3900
ttttgtttca taaagtacac agaggtcttg tttacccctc tttgctcgta tctcacatta 3960
tagtgaagca atactttgcc aatatgagga cggtcaaagc tgaatctggc ccaagcaatg 4020
ttttctgttg tttttagaaa ctgttgcgtt gcaggaagtt ccaaagcaca gcgctgctgt 4080
gggagccaga tcccctcacc accaccagca gcagtgatgt gtggccatgc cccagtccag 4140
cacctctccc cagttttagg ggacagcagg gacaggtgcc accttcccta ggctgacaag 4200
gggcacatca tccccttctc aaggctctgt gggggagcag atttcctgtc ccttgcatta 4260
atgtcatggg aaaagatggc tgccaagcac agaaagctgt ctgtgttctc tgccaaatcc 4320
tcgtgctatt ttgaccctgc actgaatgtg tctgaattta aggaggattt cctccctgcc 4380
catcctggct gctgctacag ggtcccctca agcagcaaga acacaagatg atgtcatgtc 4440
gagagaaata aggagatcta aagcagtccc tctcttcttc taccctctct ctcaaag 4497

<210> 3
<211> 1260
<212> DNA
<213> Gallus gallus

<220>
<223> Description of Combined DNA/RNA Molecule:
      Predicted FMO3 protein (complete codons) from
      compiled predicted exonic regions

<220>
<221> CDS
<222> (1)..(1260)

<400> 3
atg gtg cga cgc gtg gct gtg gtg ggc gcg ggc atc agc ggg ttg gcg 48

Met Val Arg Arg Val Ala Val Val Gly Ala Gly Ile Ser Gly Leu Ala
1               5                   10                  15

gcc acc aag tgc tgc ctg gaa gag gga ctg gag ccc acc tgc ttt gag     96
Ala Thr Lys Cys Cys Leu Glu Glu Gly Leu Glu Pro Thr Cys Phe Glu
            20                  25                  30

cag agc gag gac atc ggg ggg ctc tgg cgc tac acg gaa aag cca gag     144
Gln Ser Glu Asp Ile Gly Gly Leu Trp Arg Tyr Thr Glu Lys Pro Glu
        35                  40                  45

gaa ggc aga gct agc atc tac cgc act gtc ttc acc aat tcc tgc aag     192
Glu Gly Arg Ala Ser Ile Tyr Arg Thr Val Phe Thr Asn Ser Cys Lys
    50                  55                  60

gag atg atg tgc tac ccc gac ttc ccc ttc cct gat gac tac ccc aac     240
Glu Met Met Cys Tyr Pro Asp Phe Pro Phe Pro Asp Asp Tyr Pro Asn
65                  70                  75                  80

tac ata cac aac gca cgg ctc cat aag tac atc cgt gac tac gca cag     288
Tyr Ile His Asn Ala Arg Leu His Lys Tyr Ile Arg Asp Tyr Ala Gln
                85                  90                  95

cat ttt gac ctc ctc cgg cac atc cgc ttc aag acc aca gtc acc aag     336
His Phe Asp Leu Leu Arg His Ile Arg Phe Lys Thr Thr Val Thr Lys
            100                 105                 110

ata agg aag cgc cct gac ttc agt gct acg ggg cag tgg gag gtg gtt     384
Ile Arg Lys Arg Pro Asp Phe Ser Ala Thr Gly Gln Trp Glu Val Val
        115                 120                 125

aca cgg agt gac ggg aag gaa gag gca gcg gtt ttt gat gct gtt atg     432
Thr Arg Ser Asp Gly Lys Glu Glu Ala Ala Val Phe Asp Ala Val Met
    130                 135                 140

gtt tgc act ggg cac cat gtc tac cca aac ctc ccc ctt gcc cac ttc     480
Val Cys Thr Gly His His Val Tyr Pro Asn Leu Pro Leu Ala His Phe
145                 150                 155                 160

cca ggg ata gag aag ttt aaa ggt tgc tac tta cac agc cgg gac tac     528
Pro Gly Ile Glu Lys Phe Lys Gly Cys Tyr Leu His Ser Arg Asp Tyr
                165                 170                 175

aag gag cca gaa aag ttc aga ggg aag aag gtg ctg gtg gtt ggc ttg     576
Lys Glu Pro Glu Lys Phe Arg Gly Lys Lys Val Leu Val Val Gly Leu
            180                 185                 190

ggc aac tcc ggc tgt gac att gct gtg gag ctc agc acc gtg gcc tcg     624
Gly Asn Ser Gly Cys Asp Ile Ala Val Glu Leu Ser Thr Val Ala Ser
        195                 200                 205

cag gtt tac ctg agc tcc cga aga ggt tcc tgg gtg atg agc cgt gtc     672
Gln Val Tyr Leu Ser Ser Arg Arg Gly Ser Trp Val Met Ser Arg Val
    210                 215                 220

tgg aac ttc ggc tac ccc tgg gac atg ctg ctc atc acc cgc ttc tgg     720
Trp Asn Phe Gly Tyr Pro Trp Asp Met Leu Leu Ile Thr Arg Phe Trp
225                 230                 235                 240

acg tgg ctg gac aac ttt ctc ccc aag gca gtc agt gac tgg ctc tat     768
Thr Trp Leu Asp Asn Phe Leu Pro Lys Ala Val Ser Asp Trp Leu Tyr
                245                 250                 255

gtc agg agc atg aac cag cag tac aaa cac gag gat ttt ggc cta atg     816
Val Arg Ser Met Asn Gln Gln Tyr Lys His Glu Asp Phe Gly Leu Met
            260                 265                 270

ccg gtg gat gga tca tcc cgc agg gaa ccg gtg ttg aat gat gac atc     864

Pro Val Asp Gly Ser Ser Arg Arg Glu Pro Val Leu Asn Asp Asp Ile
        275                 280                 285

ctg agc cgc atc acc tgt ggt gtg gtg ctg ata aag cca aat gtg aag      912
Leu Ser Arg Ile Thr Cys Gly Val Val Leu Ile Lys Pro Asn Val Lys
    290                 295                 300

gaa ttc aga gaa aca tca gtc ttg ttt caa gat ggg act gtg cag gac      960
Glu Phe Arg Glu Thr Ser Val Leu Phe Gln Asp Gly Thr Val Gln Asp
305                 310                 315                 320

gac ctc gat gcg gtt atc ttt gcc tct ggt tac tct cac tcc ttt cct      1008
Asp Leu Asp Ala Val Ile Phe Ala Ser Gly Tyr Ser His Ser Phe Pro
                325                 330                 335

ttc atg gag gat gaa tcc atc atc gaa agc aag aac aac gag gct acc      1056
Phe Met Glu Asp Glu Ser Ile Ile Glu Ser Lys Asn Asn Glu Ala Thr
            340                 345                 350

ctc tac aaa tgc att gtt cct cct aag ttg gag aag cca acc atg gca      1104
Leu Tyr Lys Cys Ile Val Pro Pro Lys Leu Glu Lys Pro Thr Met Ala
        355                 360                 365

gtc att gga ttg gtc cag tcc ttt gga tcc gcc atc ccg aca gca gat      1152
Val Ile Gly Leu Val Gln Ser Phe Gly Ser Ala Ile Pro Thr Ala Asp
    370                 375                 380

gtc cag tgc cgc tgg gca gtc aag gtg ttt cag ggg ctc tgc act ctc      1200
Val Gln Cys Arg Trp Ala Val Lys Val Phe Gln Gly Leu Cys Thr Leu
385                 390                 395                 400

ccc tca gtc aac gag atg ctg gag gac ata gag gag aag aag agg aat      1248
Pro Ser Val Asn Glu Met Leu Glu Asp Ile Glu Glu Lys Lys Arg Asn
                405                 410                 415

aag atc aga tgg                                                      1260
Lys Ile Arg Trp
            420


<210> 4
<211> 420
<212> PRT
<213> Gallus gallus
<223> Description of Combined DNA/RNA Molecule:
      Predicted FMO3 protein (complete codons) from
      compiled predicted exonic regions

<400> 4
Met Val Arg Arg Val Ala Val Val Gly Ala Gly Ile Ser Gly Leu Ala
  1               5                  10                  15

Ala Thr Lys Cys Cys Leu Glu Glu Gly Leu Glu Pro Thr Cys Phe Glu
             20                  25                  30

Gln Ser Glu Asp Ile Gly Gly Leu Trp Arg Tyr Thr Glu Lys Pro Glu
         35                  40                  45

Glu Gly Arg Ala Ser Ile Tyr Arg Thr Val Phe Thr Asn Ser Cys Lys
     50                  55                  60

Glu Met Met Cys Tyr Pro Asp Phe Pro Phe Pro Asp Asp Tyr Pro Asn
 65                  70                  75                  80

Tyr Ile His Asn Ala Arg Leu His Lys Tyr Ile Arg Asp Tyr Ala Gln
                 85                  90                  95

His Phe Asp Leu Leu Arg His Ile Arg Phe Lys Thr Thr Val Thr Lys

            100                 105                 110
Ile Arg Lys Arg Pro Asp Phe Ser Ala Thr Gly Gln Trp Glu Val Val
        115                 120                 125
Thr Arg Ser Asp Gly Lys Glu Glu Ala Ala Val Phe Asp Ala Val Met
    130                 135                 140
Val Cys Thr Gly His His Val Tyr Pro Asn Leu Pro Leu Ala His Phe
145                 150                 155                 160
Pro Gly Ile Glu Lys Phe Lys Gly Cys Tyr Leu His Ser Arg Asp Tyr
                165                 170                 175
Lys Glu Pro Glu Lys Phe Arg Gly Lys Lys Val Leu Val Val Gly Leu
            180                 185                 190
Gly Asn Ser Gly Cys Asp Ile Ala Val Glu Leu Ser Thr Val Ala Ser
        195                 200                 205
Gln Val Tyr Leu Ser Ser Arg Arg Gly Ser Trp Val Met Ser Arg Val
    210                 215                 220
Trp Asn Phe Gly Tyr Pro Trp Asp Met Leu Leu Ile Thr Arg Phe Trp
225                 230                 235                 240
Thr Trp Leu Asp Asn Phe Leu Pro Lys Ala Val Ser Asp Trp Leu Tyr
                245                 250                 255
Val Arg Ser Met Asn Gln Gln Tyr Lys His Glu Asp Phe Gly Leu Met
            260                 265                 270
Pro Val Asp Gly Ser Ser Arg Arg Glu Pro Val Leu Asn Asp Asp Ile
        275                 280                 285
Leu Ser Arg Ile Thr Cys Gly Val Val Leu Ile Lys Pro Asn Val Lys
    290                 295                 300
Glu Phe Arg Glu Thr Ser Val Leu Phe Gln Asp Gly Thr Val Gln Asp
305                 310                 315                 320
Asp Leu Asp Ala Val Ile Phe Ala Ser Gly Tyr Ser His Ser Phe Pro
                325                 330                 335
Phe Met Glu Asp Glu Ser Ile Ile Glu Ser Lys Asn Asn Glu Ala Thr
            340                 345                 350
Leu Tyr Lys Cys Ile Val Pro Pro Lys Leu Glu Lys Pro Thr Met Ala
        355                 360                 365
Val Ile Gly Leu Val Gln Ser Phe Gly Ser Ala Ile Pro Thr Ala Asp
    370                 375                 380
Val Gln Cys Arg Trp Ala Val Lys Val Phe Gln Gly Leu Cys Thr Leu
385                 390                 395                 400
Pro Ser Val Asn Glu Met Leu Glu Asp Ile Glu Glu Lys Lys Arg Asn
                405                 410                 415
Lys Ile Arg Trp
            420

<210> 5
<211> 20
<212> DNA
<213> Artificial Sequence

<220>
<223> Description of Artificial Sequence: forward
      amplification primer for chicken (Gallus gallus)
      FMO3 gene

<400> 5
ctttgctctt ggcaggaaac                                                    20


<210> 6
<211> 20
<212> DNA
<213> Artificial Sequence

<220>
<223> Description of Artificial Sequence: reverse
      amplification primer for chicken (Gallus gallus)
      FMO3 gene

<400> 6
agccatcacc cagagctaac                                                    20


<210> 7
<211> 20
<212> DNA
<213> Artificial Sequence

<220>
<223> Description of Artificial Sequence: forward
      amplification primer for chicken (Gallus gallus)
      FMO3 gene

<400> 7
cctggatttt gttcccttga                                                    20


<210> 8
<211> 20
<212> DNA
<213> Artificial Sequence

<220>
<223> Description of Artificial Sequence: reverse
      amplification primer for chicken (Gallus gallus)
      FMO3 gene

<400> 8
tggtgaagac agtgcggtag                                                    20


<210> 9
<211> 20
<212> DNA
<213> Artificial Sequence

<220>
<223> Description of Artificial Sequence: forward
      amplification primer for chicken (Gallus gallus)
      FMO3 gene

<400> 9
tttggggtac ccatgactgt                                                    20


<210> 10
<211> 20

<212> DNA
<213> Artificial Sequence

<220>
<223> Description of Artificial Sequence: reverse
      amplification primer for chicken (Gallus gallus)
      FMO3 gene

<400> 10
ttttccccac cagagacaag                                                    20


<210> 11
<211> 20
<212> DNA
<213> Artificial Sequence

<220>
<223> Description of Artificial Sequence: forward
      amplification primer for chicken (Gallus gallus)
      FMO3 gene

<400> 11
tgtgatggct gaatcacgtt                                                    20


<210> 12
<211> 20
<212> DNA
<213> Artificial Sequence

<220>
<223> Description of Artificial Sequence: reverse
      amplification primer for chicken (Gallus gallus)
      FMO3 gene

<400> 12
tgctggttgg gtagtgtcaa                                                    20


<210> 13
<211> 20
<212> DNA
<213> Artificial Sequence

<220>
<223> Description of Artificial Sequence: forward
      amplification primer for chicken (Gallus gallus)
      FMO3 gene

<400> 13
gcaccatgtc tacccaaacc                                                    20


<210> 14
<211> 20
<212> DNA
<213> Artificial Sequence

<220>
<223> Description of Artificial Sequence: reverse
      amplification primer for chicken (Gallus gallus)
      FMO3 gene

<400> 14
accagcacct tcttccctct                                                    20

<210> 15
<211> 20
<212> DNA
<213> Artificial Sequence

<220>
<223> Description of Artificial Sequence: forward
      amplification primer for chicken (Gallus gallus)
      FMO3 gene

<400> 15
tggctttggg aacagatagc                                                    20


<210> 16
<211> 20
<212> DNA
<213> Artificial Sequence

<220>
<223> Description of Artificial Sequence: reverse
      amplification primer for chicken (Gallus gallus)
      FMO3 gene

<400> 16
ctgtgagcaa ctcccagaca                                                    20


<210> 17
<211> 20
<212> DNA
<213> Artificial Sequence

<220>
<223> Description of Artificial Sequence: forward
      amplification primer for chicken (Gallus gallus)
      FMO3 gene

<400> 17
tgtctggaac ttcggctacc                                                    20


<210> 18
<211> 20
<212> DNA
<213> Artificial Sequence

<220>
<223> Description of Artificial Sequence: reverse
      amplification primer for chicken (Gallus gallus)
      FMO3 gene

<400> 18
gatccaaagg actggaccaa                                                    20


<210> 19
<211> 20
<212> DNA
<213> Artificial Sequence

<220>
<223> Description of Artificial Sequence: forward
      amplification primer for chicken (Gallus gallus)
      FMO3 gene

<400> 19
cagggtggta tcaagcctgt                                                    20

<210> 20
<211> 20
<212> DNA
<213> Artificial Sequence

<220>
<223> Description of Artificial Sequence: reverse
      amplification primer for chicken (Gallus gallus)
      FMO3 gene

<400> 20
tctcttccct tgagcttgga                                                    20


<210> 21
<211> 20
<212> DNA
<213> Artificial Sequence

<220>
<223> Description of Artificial Sequence: forward
      amplification primer for chicken (Gallus gallus)
      FMO3 gene

<400> 21
cttatcaacc agccgcagat                                                    20


<210> 22
<211> 20
<212> DNA
<213> Artificial Sequence

<220>
<223> Description of Artificial Sequence: reverse
      amplification primer for chicken (Gallus gallus)
      FMO3 gene

<400> 22
gatttgccca attcaaccac                                                    20


<210> 23
<211> 20
<212> DNA
<213> Artificial Sequence

<220>
<223> Description of Artificial Sequence: forward
      amplification primer for chicken (Gallus gallus)
      FMO3 gene

<400> 23
gcactctccc ctcagtcaac                                                    20


<210> 24
<211> 20
<212> DNA
<213> Artificial Sequence

<220>
<223> Description of Artificial Sequence: reverse
      amplification primer for chicken (Gallus gallus)
      FMO3 gene

<400> 24
tctgcagcgt gttgcttatc                                                    20


<210> 25
<211> 20
<212> DNA
<213> Artificial Sequence

<220>
<223> Description of Artificial Sequence: forward
      amplification primer for putative chicken (Gallus
      gallus) FMO3 sequence

<400> 25
cctttagagc tgaccctcca                                                    20


<210> 26
<211> 20
<212> DNA
<213> Artificial Sequence

<220>
<223> Description of Artificial Sequence: reverse
      amplification primer for putative chicken (Gallus
      gallus) FMO3 sequence

<400> 26
atagttgggt gctggtcgag                                                    20


<210> 27
<211> 20
<212> DNA
<213> Artificial Sequence

<220>
<223> Description of Artificial Sequence: forward
      amplification primer for putative chicken (Gallus
      gallus) FMO3 sequence

<400> 27
tgctgtgaga gagggttgtg                                                    20


<210> 28
<211> 20
<212> DNA
<213> Artificial Sequence

<220>
<223> Description of Artificial Sequence: reverse
      amplification primer for putative chicken (Gallus
      gallus) FMO3 sequence

<400> 28
acgctagctg tcatcggttt                                                    20


<210> 29
<211> 20
<212> DNA
<213> Artificial Sequence

<220>
<223> Description of Artificial Sequence: forward
      amplification primer for putative chicken (Gallus

gallus) FMO3 sequence

<400> 29
tggtgggtct tgctaacctt                                                    20


<210> 30
<211> 20
<212> DNA
<213> Artificial Sequence

<220>
<223> Description of Artificial Sequence: reverse
      amplification primer for putative chicken (Gallus
      gallus) FMO3 sequence

<400> 30
   cagggggaag gtgtaggttt                                                     20


<210> 31
<211> 20
<212> DNA
<213> Artificial Sequence

<220>
<223> Description of Artificial Sequence: forward
      amplification primer for putative chicken (Gallus
      gallus) FMO3 sequence

<400> 31
ttgagaatgc ccagagtgtg                                                    20


<210> 32
<211> 20
<212> DNA
<213> Artificial Sequence

<220>
<223> Description of Artificial Sequence: reverse
      amplification primer for putative chicken (Gallus
      gallus) FMO3 sequence

<400> 32
aaagcgctgt ttttgctgat                                                    20


<210> 33
<211> 20
<212> DNA
<213> Artificial Sequence

<220>
<223> Description of Artificial Sequence: forward
      amplification primer for putative chicken (Gallus
      gallus) FMO3 sequence

<400> 33
ggaggtgacc cactgtcact                                                    20


<210> 34
<211> 20
<212> DNA
<213> Artificial Sequence

<220>

<223> Description of Artificial Sequence: reverse
      amplification primer for putative chicken (Gallus
      gallus) FMO3 sequence

<400> 34
tggagagcaa aggcaaaact                                                    20


<210> 35
<211> 29
<212> DNA
<213> Artificial Sequence

<220>
<223> Description of Artificial Sequence: single-base
      extension (SBE) primer for SNP detection in
      chicken (Gallus gallus) FMO3 gene

<400> 35
ctgactgact gaggactgga gcccacctg                                          29


<210> 36
<211> 34
<212> DNA
<213> Artificial Sequence

<220>
<223> Description of Artificial Sequence: single-base
      extension (SBE) primer for SNP detection in
      chicken (Gallus gallus) FMO3 gene

<400> 36
tttttttttt ttttttgctg cgagagccgc tcaa                                    34


<210> 37
<211> 39
<212> DNA
<213> Artificial Sequence

<220>
<223> Description of Artificial Sequence: single-base
      extension (SBE) primer for SNP detection in
      chicken (Gallus gallus) FMO3 gene

<400> 37
tttttttttt tttttttggc atagaaagca tgggaggaa                               39


<210> 38
<211> 44
<212> DNA
<213> Artificial Sequence

<220>
<223> Description of Artificial Sequence: single-base
      extension (SBE) primer for SNP detection in
      chicken (Gallus gallus) FMO3 gene

<400> 38
tttttttttt tttttttttt ttttttcccc gacttcccct tccc                         44


<210> 39
<211> 49
<212> DNA
<213> Artificial Sequence

<220>
<223> Description of Artificial Sequence: single-base
      extension (SBE) primer for SNP detection in
      chicken (Gallus gallus) FMO3 gene

<400> 39
   tttttttttt tttttttttt tttttttttt ctggttggag ctgatggcc                49


<210> 40
<211> 54
<212> DNA
<213> Artificial Sequence

<220>
<223> Description of Artificial Sequence: single-base
      extension (SBE) primer for SNP detection in
      chicken (Gallus gallus) FMO3 gene

<400> 40
   tttttttttt tttttttttt tttttttttt tttttacctg agctcccgaa gagg          54


<210> 41
<211> 59
<212> DNA
<213> Artificial Sequence

<220>
<223> Description of Artificial Sequence: single-base
      extension (SBE) primer for SNP detection in
      chicken (Gallus gallus) FMO3 gene

<400> 41
   tttttttttt tttttttttt tttttttttt ttttgaagta gcatttccca tcagcaatt   59


<210> 42
<211> 29
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 42
   tttttttgga gcatcacaca ccttttgac                                    29


<210> 43
<211> 34
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 43
tttttttttt tttcaaccca ttccatgctc ccag                              34

<210> 44
<211> 39
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 44
tttttttttt ttttttcaat atcacagccg gtgttccca                              39


<210> 45
<211> 44
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 45
tttttttttt tttttttttt ttttgagaag cgagcagggc tgtt                        44


<210> 46
<211> 49
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 46
tttttttttt tttttttttt ttttttgctt tacagtgagt gagctctgc                   49


<210> 47
<211> 54
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 47
tttttttttt tttttttttt tttttttttt ttttttagca tcagctgggc tggc             54


<210> 48
<211> 59
<212> DNA
<213> Artificial Sequence

<220>
<223> Description of Artificial Sequence: single-base
      exten- sion (SBE) primer for SNP detection in

chicken (Gallus gallus) sequences homologous to
human FMO gene

<400> 48
   tttttttttt tttttttttt tttttttttt tttttttttt tcggggccct gaaccaagg 59

<210> 49
<211> 64
<212> DNA
<213> Artificial Sequence

<220>
<223> Description of Artificial Sequence: single-base
exten- sion (SBE) primer for SNP detection in
chicken (Gallus gallus) sequences homologous to
human FMO gene

<400> 49
   tttttttttt tttttttttt tttttttttt tttttttttt tttggctacc ccattgacac 60
   catc 64

<210> 50
<211> 36
<212> DNA
<213> Gallus gallus

<220>
<223> /note="Sequence for chicken FMO3 gene
corresponding to exon 1"

<400> 50
ccactccaag ctctgaggca ccacaggctg cacagg 36

<210> 51
<211> 92
<212> DNA
<213> Gallus gallus

<220>
<223> /note="Sequence for chicken FMO3 gene
corresponding to intron 1"

<400> 51
taagcgagga cagatgaaaa agccccaggt gttgcatggg gccagactgg gcctaggagc 60
gcaacatggc gccatgacgg ctgctctccc ac 92

<210> 52
<211> 143
<212> DNA
<213> Gallus gallus

<220>
<223> /note="Sequence for chicken FMO3 gene
corresponding to exon 2"

<400> 52
cacaggacac catggtgcga cgcgtggctg tggtgggcgc gggcatcagc gggttggcgg 60
ccaccaagtg ctgcctggaa gagggactgg agcccacctg ctttgagcag agcgaggaca 120
tcggggggct ctggcgctac acg 143

<210> 53
<211> 153
<212> DNA

<213> Gallus gallus

<220>
<223> /note="Sequence for chicken FMO3 gene
      corresponding to partial intron 2A"

<400> 53
tgaggggttt gggctgcgtg gtcttctggc aatctggggc taggggagaa tctggggggg 60
cggtggctgc gagagccgct caagtggagg aactgaccgt gttaaaactc atgatgaggg 120
gcaaagacat caggttagct ctgggtgatg gct                              153


<210> 54
<211> 645
<212> DNA
<213> Gallus gallus

<220>
<223> /note="Sequence for chicken FMO3 gene
      corresponding to partial intron 2B"

<400> 54
ccttgagagc aataagcctg caatgtcccc tgtgtgctcc agctaagctg agaaaaatat 60
cactttgagc tctgcaagtg atgtttataa gaagcgaggc ttgaagttga aaataacagc 120
tgtgtgtgtg cccaaggtta agcccagaca acaacagaac tgatctttct acagtggtaa 180
atgtagaaga cattatgcca agtagaggac tctgaggtta ttacagggat gtactgtgta 240
tggctcttca ggatgagccc acaaagcctg tgagcacatg tggaaggtaa gcagtgggct 300
ggtgatgacc ctctggggtg ctcagtgccc ctttttctgg gcttggttgg tgaagctgag 360
tggaaggacg ggctgggaat tacctggcca gacaaggagg gcatagaaag catgggagga 420
actgggttgc aagtgaagac attgctctga gagccagaca ttgtggttca ggtccccaga 480
actgaggttc tcctagtcgt gttcttgggt cagttctcct gacccaaagt catgacattg 540
ggtgaggtta tctccagatt ggctcagcat cttcttgcct gtggcttatg actttttggg 600
gtacccatga ctgtgacccc tctaaccgaa gctctcttcc tccag                 645


<210> 55
<211> 189
<212> DNA
<213> Gallus gallus

<220>
<223> /note="Sequence for chicken FMO3 gene
      corresponding to exon 3"

<400> 55
gaaaagccag aggaaggcag agctagcatc taccgcactg tcttcaccaa ttcctgcaag 60
gagatgatgt gctaccccga cttccccttc cctgatgact accccaacta catacacaac 120
gcacggctcc ataagtacat ccgtgactac gcacagcatt ttgacctcct ccggcacatc 180
cgcttcaag                                                         189


<210> 56
<211> 317
<212> DNA
<213> Gallus gallus

<220>
<223> /note="Sequence for chicken FMO3 gene
      corresponding to intron 3"

<400> 56
gtaggaggga tggactttgc tgctgtgtct cagagaaaat cctgcggcag acatcctgag 60
ggaaagtctt gttggtttgg ttaaattggg tctcaccagc tgctggttgg gtagtgtcaa 120
atgatcagtc atcacgggga aaccattgct tctgagggtg gaaaatcacg tggaatggga 180
ggacaccagt gggttttctc tatccaaaat aattgccaag tctgtgacct tgtctctggt 240
ggggaaaagg acagagcctg cctggctgca gtggggtacc cggagcactt cccaaacctt 300
tatccctttc ctttaag                                                317

<210> 57
<211> 162
<212> DNA
<213> Gallus gallus

<220>
<223> /note="Sequence for chicken FMO3 gene
      corresponding to exon 4"

<400> 57
accacagtca ccaagataag gaagcgccct gacttcagtg ctacggggca gtgggaggtg 60
gttacacgga gtgacgggaa ggaagaggca gcggtttttg atgctgttat ggtttgcact 120
gggcaccatg tctacccaaa cctccccctt gcccacttcc ca                    162


<210> 58
<211> 821
<212> DNA
<213> Gallus gallus

<220>
<223> /note="Sequence for chicken FMO3 gene
      corresponding to intron 4"

<400> 58
gtaagctata tgaacgtgat tcagccatca cacagccagg tgtgcacttt acattcaaat 60
gttgggtttt ggtttttttt ttcagcatca aagattggtt ttaggggtga gctctgctca 120
ggaaatcaaa acttttattt aagacaaaag actgtatcca aaccatgtca aagcagggac 180
attcttactg gaggcaatgc taatatttcc tggaaaaatt attgcagggt ttccatttag 240
tagaagtttc ttcactaaag ggtttcgtaa agcacttctt ttggcatttt ttccatggtt 300
tctagtgcaa atgctgtgcg caaactttgc tcctgcttac aggatcagca gtgggaaaac 360
ttcctatgat aagaagtatt tggtgctggg ggtctgtttg gaggaggtat ggtcttggga 420
cactgactgg ctgggaaaac tcccattgtg tcctttgtca ccttctgttt tagaaattaa 480
agttgctttc ccatcacttg ctggcgcaaa ggcacgaaga cacaccaagc ggatggaacg 540
tggctggtct ggtgtctcag ggtgatgatg ctgagttgta tctttaaggc acacaaatac 600
cacacaaagt tatcctgttc tctcatgctg gggctcccct ctgtttaaat attgaatgaa 660
tccagagaca aaaacccatc tacaggtttg cagcatccct ctgctgtgag caactcccag 720
acatgctcca gcagcgcctg caggaatggg agcagccaag gttaacgcag ccacacttct 780
tcactgatga cttacatccc tttctgtctt cctgctccca g                     821


<210> 59
<211> 144
<212> DNA
<213> Gallus gallus

<220>
<223> /note="Sequence for chicken FMO3 gene
      corresponding to exon 5"

<400> 59
gggatagaga agtttaaagg ttgctactta cacagccggg actacaagga gccagaaaag 60
ttcagaggga agaaggtgct ggtggttggc ttgggcaact ccggctgtga cattgctgtg 120
gagctcagca ccgtggcctc gcag                                        144


<210> 60
<211> 82
<212> DNA
<213> Gallus gallus

<220>
<223> /note="Sequence for chicken FMO3 gene
      corresponding to intron 5"

<400> 60
gtatggggac ctgaaccagg gctggttgga gctgatggcc gccacgacgg tggctcaacc 60

ctgcctcacc atcccccccc ag                                              82

<210> 61
<211> 201
<212> DNA
<213> Gallus gallus

<220>
<223> /note="Sequence for chicken FMO3 gene
      corresponding to exon 6"

<400> 61
gtttacctga gctcccgaag aggttcctgg gtgatgagcc gtgtctggaa cttcggctac 60
ccctgggaca tgctgctcat cacccgcttc tggacgtggc tggacaactt tctccccaag 120
gcagtcagtg actggctcta tgtcaggagc atgaaccagc agtacaaaca cgaggatttt 180
ggcctaatgc cggtggatgg a                                              201

<210> 62
<211> 111
<212> DNA
<213> Gallus gallus

<220>
<223> /note="Sequence for chicken FMO3 gene
      corresponding to intron 6"

<400> 62
gtaagcatga ggctatctgt tcccaaagcc agaagtgctg gtcccaggca gggtggtatc 60
aagcctgtac agggccagca gcagcagcca acttaggctt tttccttgca g             111

<210> 63
<211> 357
<212> DNA
<213> Gallus gallus

<220>
<223> /note="Sequence for chicken FMO3 gene
      corresponding to exon 7"

<400> 63
tcatcccgca gggaaccggt gttgaatgat gacatcctga gccgcatcac ctgtggtgtg 60
gtgctgataa agccaaatgt gaaggaattc agagaaacat cagtcttgtt tcaagatggg 120
actgtgcagg acgacctcga tgcggttatc tttgccyctg gttactctca ctcctttcct 180
ttcatggagg atgaatccat catcgaaagc aagaacaacg aggctaccct ctacaaatgc 240
attgttcctc ctaagttgga gaagccaacc atggcagtca ttggattggt ccagtccttt 300
ggatccgcca tcccgacagc agatgtccag tgccgctggg cagtcaaggt gtttcag      357

<210> 64
<211> 391
<212> DNA
<213> Gallus gallus

<220>
<223> /note="Sequence for chicken FMO3 gene
      corresponding to intron 7"

<400> 64
gtaggtgaat gagctaaggt ccctaaatgg gaagtagcat ttcccatcag caatttcaca 60
gattccaact aaaaatgagg gcagtgcatg agtgctggaa ataccagcaa cgggttctcc 120
ccgggatggg ctgccttgtg ccatggagaa taggtcactt atcaaccagc cgcagatgtc 180
agtgaggtcc ctgtggtgag gcagctatct gggcatcgcc acacatccta aaggacaggg 240
ggatctgttt tatcgcaacc ctgatgccca gtttgatcca agctcaaggg aagagaagtg 300
cccaaggtga tccctacaga ccccacctgc tgcaggaggg atctcctggg catcatcacc 360
ctttgccgac actcttcctg ttctttccag g                                   391

<210> 65
<211> 75
<212> DNA
<213> Gallus gallus

<220>
<223> /note="Sequence for chicken FMO3 gene
      corresponding to exon 8"

<400> 65
gggctctgca ctctcccctc agtcaacgag atgctggagg acatagagga gaagaagagg 60
aataagatca gatgg                                                  75


<210> 66
<211> 1002
<212> DNA
<213> Gallus gallus

<220>
<223> /note="Sequence for chicken FMO3 gene
      corresponding to intron 8"

<400> 66
taggtgcagc tcatgggacg ctgcccagga aggaggagga aaaaccttgc tgtcttctca 60
gcctgggtgg gctggatgtg ggcatgggga gggctgcagc aggggcaagc tcctagcaag 120
gagaccattc tgaactgctc cgttcatgac ccagatgagc aaacccaatg ggaccaacac 180
aggaaggtca tacccagctt ctttggtgtc agttctcagg tttccacccg ccccagggtg 240
gttgaattgg gcaaatctgt gcagaaaatc tgtgatggta agatttcctg tctgaaaatg 300
ctgctttgca gcatttgtag agtgttattg gtctggaaaa aacacagaaa ttgaggcccc 360
agcaaaaact gaacattccc tcattcttat tcttcagtaa catttttttg tttcataaag 420
tacacagagg tcttgtttac ccctctttgc tcgtatctca cattatagtg aagcaatact 480
ttgccaatat gaggacggtc aaagctgaat ctggcccaag caatgttttc tgttgttttt 540
agaaactgtt gcgttgcagg aagttccaaa gcacagcgct gctgtgggag ccagatcccc 600
tcaccaccac cagcagcagt gatgtgtggc catgccccag tccagcacct ctccccagtt 660
ttaggggaca gcagggacag gtgccacctt ccctaggctg acaaggggca catcatcccc 720
ttctcaaggc tctgtggggg agcagatttc ctgtcccttg cattaatgtc atgggaaaag 780
atggctgcca agcacagaaa gctgtctgtg ttctctgcca aatcctcgtg ctattttgac 840
cctgcactga atgtgtctga atttaaggag gatttcctcc ctgcccatcc tggctgctgc 900
tacagggtcc cctcaagcag caagaacaca agatgatgtc atgtcgagag aaataaggag 960
atctaaagca gtccctctct tcttctaccc tctctctcaa ag                    1002


<210> 67
<211> 20
<212> DNA
<213> Artificial Sequence

<220>
<223> Description of Artificial Sequence: forward
      amplification primer

<220>
<223> /note="Forward amplification primer for
      amplification of chicken homologue of human FMO
      gene (contig 24165.1; unknow genomic location)"

<400> 67
acggtcaaag ctgaatctgg                                             20


<210> 68
<211> 20
<212> DNA
<213> Artificial Sequence

<220>
<223> Description of Artificial Sequence: reverse
      amplification primer

<220>
<223> /note="Reverse amplification primer for
      amplification of chicken homologue of human FMO
      gene (contig 24165.1; unknow genomic location)"

<400> 68
tcccgaacct ttgagagaga                                              20


<210> 69
<211> 514
<212> DNA
<213> Gallus gallus

<220>
<223> /not="Contig 24165.1 (chicken homologue of human
      FMO gene)"

<400> 69
acggtcaaag ctgaatctgg cccaagcaat gttttctgtt gtttttagaa actgttgcgt 60
tgcaggaagt tccaaagcac agcgctgctg tgggagccag atcccctcac caccaccagc 120
agcagtgatg tgtggccatg ccccagtcca gcacctctcc ccagttttag gggacagcag 180
ggacaggtgc caccttccct aggctgacaa ggggcacatc atccccttct caaggctctg 240
tgggggagca gatttcctgt cccttgcatt aatgtcatgg gaaaagatgg ctgccaagca 300
cagaaagctg tctgtgttct ctgccaaatc ctcgtgctat tttgaccctg cactgaatgt 360
gtctgaattt aaggaggatt tcctccctgc ccatcctggc tgctgctaca gggtcccctc 420
aagcagcaag aacacaagat gatgtcatgt cgagagaaat aaggagatct aaagcagtcc 480
ctctcttctt ctaccctctc tctcaaagtt cggg                              514


<210> 70
<211> 1307
<212> DNA
<213> Gallus gallus

<220>
<223> /note="Compiled sequence of 8 confirmed exonic
      regions for chicken (Gallus gallus) FMO3 gene"

<400> 70
ccactccaag ctctgaggca ccacaggctg cacaggcaca ggacaccatg gtgcgacgcg 60
tggctgtggt gggcgcgggc atcagcgggt tggcggccac caagtgctgc ctggaagagg 120
gactggagcc cacctgcttt gagcagagcg aggacatcgg ggggctctgg cgctacacgg 180
aaaagccaga ggaaggcaga gctagcatct accgcactgt cttcaccaat tcctgcaagg 240
agatgatgtg ctaccccgac ttccccttcc ctgatgacta ccccaactac atacacaacg 300
cacggctcca taagtacatc cgtgactacg cacagcattt tgacctcctc cggcacatcc 360
gcttcaagac cacagtcacc aagataagga agcgccctga cttcagtgct acggggcagt 420
gggaggtggt tacacggagt gacgggaagg aagaggcagc ggtttttgat gctgttatgg 480
tttgcactgg gcaccatgtc tacccaaacc tcccccttgc ccacttccca gggatagaga 540
agtttaaagg ttgctactta cacagccggg actacaagga gccagaaaag ttcagaggga 600
agaaggtgct ggtggttggc ttgggcaact ccggctgtga cattgctgtg gagctcagca 660
ccgtggcctc gcaggtttac ctgagctccc gaagaggttc ctgggtgatg agccgtgtct 720
ggaacttcgg ctacccctgg gacatgctgc tcatcacccg cttctggacg tggctggaca 780
actttctccc caaggcagtc agtgactggc tctatgtcag gagcatgaac cagcagtaca 840
aacacgagga ttttggccta atgccggtgg atggatcatc ccgcagggaa ccggtgttga 900
atgatgacat cctgagccgc atcacctgtg gtgtggtgct gataaagcca aatgtgaagg 960
aattcagaga aacatcagtc ttgtttcaag atgggactgt gcaggacgac ctcgatgcgg 1020
ttatctttgc ctctggttac tctcactcct ttcctttcat ggaggatgaa tccatcatcg 1080
aaagcaagaa caacgaggct accctctaca aatgcattgt tcctcctaag ttggagaagc 1140
caaccatggc agtcattgga ttggtccagt cctttggatc cgccatcccg acagcagatg 1200
tccagtgccg ctgggcagtc aaggtgtttc aggggctctg cactctcccc tcagtcaacg 1260
agatgctgga ggacatagag gagaagaaga ggaataagat cagatgg                1307

<210> 71
<211> 132
<212> DNA
<213> Gallus gallus

<220>
<223> Description of Combined DNA/RNA Molecule: sequence
      of exon 2 of FMO3 gene

<220>
<221> CDS
<222> (1)..(132)

<400> 71
atg gtg cga cgc gtg gct gtg gtg ggc gcg ggc atc agc ggg ttg gcg      48
Met Val Arg Arg Val Ala Val Val Gly Ala Gly Ile Ser Gly Leu Ala
  1               5                  10                  15

gcc acc aag tgc tgc ctg gaa gag gga ctg gag ccc acc tgc ttt gag      96
Ala Thr Lys Cys Cys Leu Glu Glu Gly Leu Glu Pro Thr Cys Phe Glu
             20                  25                  30

cag agc gag gac atc ggg ggg ctc tgg cgc tac acg                      132
Gln Ser Glu Asp Ile Gly Gly Leu Trp Arg Tyr Thr
         35                  40


<210> 72
<211> 44
<212> PRT
<213> Gallus gallus
<223> Description of Combined DNA/RNA Molecule: sequence
      of exon 2 of FMO3 gene

<400> 72
Met Val Arg Arg Val Ala Val Val Gly Ala Gly Ile Ser Gly Leu Ala
  1               5                  10                  15

Ala Thr Lys Cys Cys Leu Glu Glu Gly Leu Glu Pro Thr Cys Phe Glu
             20                  25                  30

Gln Ser Glu Asp Ile Gly Gly Leu Trp Arg Tyr Thr
         35                  40


<210> 73
<211> 189
<212> DNA
<213> Gallus gallus

<220>
<223> Description of Combined DNA/RNA Molecule: sequence
      of exon 3 of FMO3 gene

<220>
<221> CDS
<222> (1)..(189)

<400> 73
gaa aag cca gag gaa ggc aga gct agc atc tac cgc act gtc ttc acc      48
Glu Lys Pro Glu Glu Gly Arg Ala Ser Ile Tyr Arg Thr Val Phe Thr
  1               5                  10                  15

aat tcc tgc aag gag atg atg tgc tac ccc gac ttc ccc ttc cct gat      96
Asn Ser Cys Lys Glu Met Met Cys Tyr Pro Asp Phe Pro Phe Pro Asp
             20                  25                  30

gac tac ccc aac tac ata cac aac gca cgg ctc cat aag tac atc cgt     144
Asp Tyr Pro Asn Tyr Ile His Asn Ala Arg Leu His Lys Tyr Ile Arg
        35                  40                  45

gac tac gca cag cat ttt gac ctc ctc cgg cac atc cgc ttc aag         189
Asp Tyr Ala Gln His Phe Asp Leu Leu Arg His Ile Arg Phe Lys
    50                  55                  60


<210> 74
<211> 63
<212> PRT
<213> Gallus gallus
<223> Description of Combined DNA/RNA Molecule: sequence
      of exon 3 of FMO3 gene

<400> 74
Glu Lys Pro Glu Glu Gly Arg Ala Ser Ile Tyr Arg Thr Val Phe Thr
1               5                   10                  15

Asn Ser Cys Lys Glu Met Met Cys Tyr Pro Asp Phe Pro Phe Pro Asp
            20                  25                  30

Asp Tyr Pro Asn Tyr Ile His Asn Ala Arg Leu His Lys Tyr Ile Arg
        35                  40                  45

Asp Tyr Ala Gln His Phe Asp Leu Leu Arg His Ile Arg Phe Lys
    50                  55                  60



<210> 75
<211> 162
<212> DNA
<213> Gallus gallus

<220>
<223> Description of Combined DNA/RNA Molecule: sequence
      of exon 4 of FMO3 gene

<220>
<221> CDS
<222> (1)..(162)

<400> 75
acc aca gtc acc aag ata agg aag cgc cct gac ttc agt gct acg ggg     48
Thr Thr Val Thr Lys Ile Arg Lys Arg Pro Asp Phe Ser Ala Thr Gly
1               5                   10                  15

cag tgg gag gtg gtt aca cgg agt gac ggg aag gaa gag gca gcg gtt     96
Gln Trp Glu Val Val Thr Arg Ser Asp Gly Lys Glu Glu Ala Ala Val
            20                  25                  30

ttt gat gct gtt atg gtt tgc act ggg cac cat gtc tac cca aac ctc     144
Phe Asp Ala Val Met Val Cys Thr Gly His His Val Tyr Pro Asn Leu
        35                  40                  45

ccc ctt gcc cac ttc cca                                             162
Pro Leu Ala His Phe Pro
    50


<210> 76
<211> 54
<212> PRT
<213> Gallus gallus
<223> Description of Combined DNA/RNA Molecule: sequence
      of exon 4 of FMO3 gene

<400> 76
Thr Thr Val Thr Lys Ile Arg Lys Arg Pro Asp Phe Ser Ala Thr Gly
1               5                   10                  15

Gln Trp Glu Val Val Thr Arg Ser Asp Gly Lys Glu Glu Ala Ala Val
            20                  25                  30

Phe Asp Ala Val Met Val Cys Thr Gly His His Val Tyr Pro Asn Leu
        35                  40                  45

Pro Leu Ala His Phe Pro
    50

<210> 77
<211> 144
<212> DNA
<213> Gallus gallus

<220>
<223> Description of Combined DNA/RNA Molecule: sequence
      of exon 5 of FMO3 gene

<220>
<221> CDS
<222> (1)..(144)

<400> 77
ggg ata gag aag ttt aaa ggt tgc tac tta cac agc cgg gac tac aag     48
Gly Ile Glu Lys Phe Lys Gly Cys Tyr Leu His Ser Arg Asp Tyr Lys
1               5                   10                  15

gag cca gaa aag ttc aga ggg aag aag gtg ctg gtg gtt ggc ttg ggc     96
Glu Pro Glu Lys Phe Arg Gly Lys Lys Val Leu Val Val Gly Leu Gly
            20                  25                  30

aac tcc ggc tgt gac att gct gtg gag ctc agc acc gtg gcc tcg cag     144
Asn Ser Gly Cys Asp Ile Ala Val Glu Leu Ser Thr Val Ala Ser Gln
        35                  40                  45

<210> 78
<211> 48
<212> PRT
<213> Gallus gallus
<223> Description of Combined DNA/RNA Molecule: sequence
      of exon 5 of FMO3 gene

<400> 78
Gly Ile Glu Lys Phe Lys Gly Cys Tyr Leu His Ser Arg Asp Tyr Lys
1               5                   10                  15

Glu Pro Glu Lys Phe Arg Gly Lys Lys Val Leu Val Val Gly Leu Gly
            20                  25                  30

Asn Ser Gly Cys Asp Ile Ala Val Glu Leu Ser Thr Val Ala Ser Gln
        35                  40                  45

<210> 79
<211> 201
<212> DNA
<213> Gallus gallus

<220>

<223> Description of Combined DNA/RNA Molecule: sequence
      of exon 6 of FMO3 gene

<220>
<221> CDS
<222> (1)..(201)

<400> 79
gtt tac ctg agc tcc cga aga ggt tcc tgg gtg atg agc cgt gtc tgg     48
Val Tyr Leu Ser Ser Arg Arg Gly Ser Trp Val Met Ser Arg Val Trp
  1               5                  10                  15

aac ttc ggc tac ccc tgg gac atg ctg ctc atc acc cgc ttc tgg acg     96
Asn Phe Gly Tyr Pro Trp Asp Met Leu Leu Ile Thr Arg Phe Trp Thr
             20                  25                  30

tgg ctg gac aac ttt ctc ccc aag gca gtc agt gac tgg ctc tat gtc    144
Trp Leu Asp Asn Phe Leu Pro Lys Ala Val Ser Asp Trp Leu Tyr Val
         35                  40                  45

agg agc atg aac cag cag tac aaa cac gag gat ttt ggc cta atg ccg    192
Arg Ser Met Asn Gln Gln Tyr Lys His Glu Asp Phe Gly Leu Met Pro
     50                  55                  60

gtg gat gga                                                        201
Val Asp Gly
65


<210> 80
<211> 67
<212> PRT
<213> Gallus gallus
<223> Description of Combined DNA/RNA Molecule: sequence
      of exon 6 of FMO3 gene

<400> 80
Val Tyr Leu Ser Ser Arg Arg Gly Ser Trp Val Met Ser Arg Val Trp
  1               5                  10                  15

Asn Phe Gly Tyr Pro Trp Asp Met Leu Leu Ile Thr Arg Phe Trp Thr
             20                  25                  30

Trp Leu Asp Asn Phe Leu Pro Lys Ala Val Ser Asp Trp Leu Tyr Val
         35                  40                  45

Arg Ser Met Asn Gln Gln Tyr Lys His Glu Asp Phe Gly Leu Met Pro
     50                  55                  60

Val Asp Gly
65



<210> 81
<211> 357
<212> DNA
<213> Gallus gallus

<220>
<223> Description of Combined DNA/RNA Molecule: sequence
      of exon 7 of FMO3 gene

<220>
<221> CDS
<222> (1)..(357)

<400> 81

tca tcc cgc agg gaa ccg gtg ttg aat gat gac atc ctg agc cgc atc     48
Ser Ser Arg Arg Glu Pro Val Leu Asn Asp Asp Ile Leu Ser Arg Ile
 1               5                  10                  15

acc tgt ggt gtg gtg ctg ata aag cca aat gtg aag gaa ttc aga gaa     96
Thr Cys Gly Val Val Leu Ile Lys Pro Asn Val Lys Glu Phe Arg Glu
            20                  25                  30

aca tca gtc ttg ttt caa gat ggg act gtg cag gac gac ctc gat gcg     144
Thr Ser Val Leu Phe Gln Asp Gly Thr Val Gln Asp Asp Leu Asp Ala
        35                  40                  45

gtt atc ttt gcc tct ggt tac tct cac tcc ttt cct ttc atg gag gat     192
Val Ile Phe Ala Ser Gly Tyr Ser His Ser Phe Pro Phe Met Glu Asp
    50                  55                  60

gaa tcc atc atc gaa agc aag aac aac gag gct acc ctc tac aaa tgc     240
Glu Ser Ile Ile Glu Ser Lys Asn Asn Glu Ala Thr Leu Tyr Lys Cys
65                  70                  75                  80

att gtt cct cct aag ttg gag aag cca acc atg gca gtc att gga ttg     288
Ile Val Pro Pro Lys Leu Glu Lys Pro Thr Met Ala Val Ile Gly Leu
                85                  90                  95

gtc cag tcc ttt gga tcc gcc atc ccg aca gca gat gtc cag tgc cgc     336
Val Gln Ser Phe Gly Ser Ala Ile Pro Thr Ala Asp Val Gln Cys Arg
            100                 105                 110

tgg gca gtc aag gtg ttt cag                                         357
Trp Ala Val Lys Val Phe Gln
        115

<210> 82
<211> 119
<212> PRT
<213> Gallus gallus
<223> Description of Combined DNA/RNA Molecule: sequence
      of exon 7 of FMO3 gene

<400> 82
Ser Ser Arg Arg Glu Pro Val Leu Asn Asp Asp Ile Leu Ser Arg Ile
 1               5                  10                  15

Thr Cys Gly Val Val Leu Ile Lys Pro Asn Val Lys Glu Phe Arg Glu
            20                  25                  30

Thr Ser Val Leu Phe Gln Asp Gly Thr Val Gln Asp Asp Leu Asp Ala
        35                  40                  45

Val Ile Phe Ala Ser Gly Tyr Ser His Ser Phe Pro Phe Met Glu Asp
    50                  55                  60

Glu Ser Ile Ile Glu Ser Lys Asn Asn Glu Ala Thr Leu Tyr Lys Cys
65                  70                  75                  80

Ile Val Pro Pro Lys Leu Glu Lys Pro Thr Met Ala Val Ile Gly Leu
                85                  90                  95

Val Gln Ser Phe Gly Ser Ala Ile Pro Thr Ala Asp Val Gln Cys Arg
            100                 105                 110

Trp Ala Val Lys Val Phe Gln
        115

<210> 83

<211> 75
<212> DNA
<213> Gallus gallus

<220>
<223> Description of Combined DNA/RNA Molecule: sequence
      of exon 8 of FMO3 gene

<220>
<221> CDS
<222> (1)..(75)

<400> 83
ggg ctc tgc act ctc ccc tca gtc aac gag atg ctg gag gac ata gag    48
Gly Leu Cys Thr Leu Pro Ser Val Asn Glu Met Leu Glu Asp Ile Glu
  1               5                  10                  15

gag aag aag agg aat aag atc aga tgg                                75
Glu Lys Lys Arg Asn Lys Ile Arg Trp
            20                  25


<210> 84
<211> 25
<212> PRT
<213> Gallus gallus
<223> Description of Combined DNA/RNA Molecule: sequence
      of exon 8 of FMO3 gene

<400> 84
Gly Leu Cys Thr Leu Pro Ser Val Asn Glu Met Leu Glu Asp Ile Glu
  1               5                  10                  15

Glu Lys Lys Arg Asn Lys Ile Arg Trp
            20                  25

SEQUENCE LISTING

<110> Hendrix Poultry Breeders B.V.

<120> FMO gene polymorphisms

<130> P70434EP00

<140> 04077506.6
<141> 2004-09-10

<160> 84

<170> PatentIn Ver. 2.1

<210> 1
<211> 424
<212> DNA
<213> Gallus gallus

<220>
<223> /note="FMO3 gene exon 1 - partial intron 2"

<400> 1
ccactccaag ctctgaggca ccacaggctg cacaggtaag cgaggacaga tgaaaaagcc 60
ccaggtgttg catggggcca gactgggcct aggagcgcaa catggcgcca tgacggctgc 120
tctcccacca caggacacca tggtgcgacg cgtggctgtg gtgggcgcgg gcatcagcgg 180
gttggcggcc accaagtgct gcctggaaga gggactggag cccacctgct ttgagcagag 240
cgaggacatc ggggggctct ggcgctacac gtgaggggtt tgggctgcgt ggtcttctgg 300
caatctgggg ctaggggaga atctgggggg gcggtggctg cgagagccgc tcaagtggag 360
gaactgaccg tgttaaaact catgatgagg ggcaaagaca tcaggttagc tctgggtgat 420
ggct 424

<210> 2
<211> 4497
<212> DNA
<213> Gallus gallus

<220>
<223> /note="FMO3 gene partial intron 2 - intron 8"

<400> 2
ccttgagagc aataagcctg caatgtcccc tgtgtgctcc agctaagctg agaaaaatat 60
cactttgagc tctgcaagtg atgtttataa gaagcgaggc ttgaagttga aaataacagc 120
tgtgtgtgtg cccaaggtta agcccagaca acaacagaac tgatctttct acagtggtaa 180
atgtagaaga cattatgcca agtagaggac tctgaggtta ttacagggat gtactgtgta 240
tggctcttca ggatgagccc acaaagcctg tgagcacatg tggaaggtaa gcagtgggct 300
ggtgatgacc ctctggggtg ctcagtgccc ctttttctgg gcttggttgg tgaagctgag 360
tggaaggacg ggctgggaat tacctggcca gacaaggagg gcatagaaag catgggagga 420
actgggttgc aagtgaagac attgctctga gagccagaca ttgtggttca ggtccccaga 480
actgaggttc tcctagtcgt gttcttgggt cagttctcct gacccaaagt catgacattg 540
ggtgaggtta tctccagatt ggctcagcat cttcttgcct gtggcttatg actttttggg 600
gtacccatga ctgtgacccc tctaaccgaa gctctcttcc tccaggaaaa gccagaggaa 660
ggcagagcta gcatctaccg cactgtcttc accaattcct gcaaggagat gatgtgctac 720
cccgacttcc ccttccctga tgactacccc aactacatac acaacgcacg gctccataag 780
tacatccgtg actacgcaca gcattttgac ctcctccggc acatccgctt caaggtagga 840
gggatggact ttgctgctgt gtctcagaga aaatcctgcg gcagacatcc tgagggaaag 900
tcttgttggt tttgttaaat tgggtctcac cagctgctgg ttgggtagtg tcaaatgatc 960
agtcatcacg gggaaaccat tgcttctgag ggtggaaaat cacgtggaat gggaggacac 1020
cagtgggttt tctctatcca aaataattgc caagtctgtg accttgtctc tggtggggaa 1080
aaggacagag cctgcctggc tgcagtgggg tacccggagc acttcccaaa cctttatccc 1140
tttcctttaa gaccacagtc accaagataa ggaagcgccc tgacttcagt gctacggggc 1200
agtgggaggt ggttacacgg agtgacggga aggaagaggc agcggttttt gatgctgtta 1260
tggtttgcac tgggcaccat gtctacccaa acctccccct tgcccacttc ccagtaagct 1320
atatgaacgt gattcagcca tcacacagcc aggtgtgcac tttacattca aatgttgggt 1380
tttggttttt tttttcagca tcaaagattg gttttagggg tgagctctgc tcaggaaatc 1440
aaaactttta tttaagacaa aagactgtat ccaaaccatg tcaaagcagg gacattctta 1500

ctggaggcaa tgctaatatt tcctggaaaa attattgcag ggtttccatt tagtagaagt 1560
ttcttcacta aagggtttcg taaagcactt cttttggcat tttttccatg gtttctagtg 1620
caaatgctgt gcgcaaactt tgctcctgct tacaggatca gcagtgggaa aacttcctat 1680
gataagaagt atttggtgct gggggtctgt ttggaggagg tatggtcttg ggacactgac 1740
tggctgggaa aactcccatt gtgtcctttg tcaccttctg ttttagaaat taaagttgct 1800
ttcccatcac ttgctggcgc aaaggcacga agacacacca agcggatgga acgtggctgg 1860
tctggtgtct cagggtgatg atgctgagtt gtatctttaa ggcacacaaa taccacacaa 1920
agttatcctg ttctctcatg ctggggctcc cctctgttta aatattgaat gaatccagag 1980
acaaaaaccc atctacaggt ttgcagcatc cctctgctgt gagcaactcc cagacatgct 2040
ccagcagcgc ctgcaggaat gggagcagcc aaggttaacg cagccacact tcttcactga 2100
tgacttacat ccctttctgt cttcctgctc ccaggggata gagaagttta aaggttgcta 2160
cttacacagc cgggactaca aggagccaga aaagttcaga gggaagaagg tgctggtggt 2220
tggcttgggc aactccggct gtgacattgc tgtggagctc agcaccgtgg cctcgcaggt 2280
atggggacct gaaccagggc tggttggagc tgatggccgc cacgacggtg gctcaaccct 2340
gcctcaccat ccccccccag gtttacctga gctcccgaag aggttcctgg gtgatgagcc 2400
gtgtctggaa cttcggctac ccctgggaca tgctgctcat cacccgcttc tggacgtggc 2460
tggacaactt tctccccaag gcagtcagtg actggctcta tgtcaggagc atgaaccagc 2520
agtacaaaca cgaggatttt ggcctaatgc cggtggatgg agtaagcatg aggctatctg 2580
ttcccaaagc cagaagtgct ggtcccaggc agggtggtat caagcctgta cagggccagc 2640
agcagcagcc aacttaggct ttttccttgc agtcatcccg cagggaaccg gtgttgaatg 2700
atgacatcct gagccgcatc acctgtggtg tggtgctgat aaagccaaat gtgaaggaat 2760
tcagagaaac atcagtcttg tttcaagatg ggactgtgca ggacgacctc gatgcggtta 2820
tctttgccyc tggttactct cactcctttc ctttcatgga ggatgaatcc atcatcgaaa 2880
gcaagaacaa cgaggctacc ctctacaaat gcattgttcc tcctaagttg gagaagccaa 2940
ccatggcagt cattggattg gtccagtcct ttggatccgc catcccgaca gcagatgtcc 3000
agtgccgctg ggcagtcaag gtgtttcagg taggtgaatg agctaaggtc cctaaatggg 3060
aagtagcatt tcccatcagc aatttcacag attccaacta aaaatgaggg cagtgcatga 3120
gtgctggaaa taccagcaac gggttctccc cgggatgggc tgccttgtgc catggagaat 3180
aggtcactta tcaaccagcc gcagatgtca gtgaggtccc tgtggtgagg cagctatctg 3240
ggcatcgcca cacatcctaa aggacagggg gatctgtttt atcgcaaccc tgatgcccag 3300
tttgatccaa gctcaaggga agagaagtgc ccaaggtgat ccctacagac cccacctgct 3360
gcaggaggga tctcctgggc atcatcaccc tttgccgaca ctcttcctgt tctttccagg 3420
gggctctgca ctctcccctc agtcaacgag atgctggagg acatagagga gaagaagagg 3480
aataagatca gatggtaggt gcagctcatg ggacgctgcc caggaaggag gaggaaaaac 3540
cttgctgtct tctcagcctg ggtgggctgg atgtgggcat ggggagggct gcagcagggg 3600
caagctccta gcaaggagac cattctgaac tgctccgttc atgacccaga tgagcaaacc 3660
caatgggacc aacacaggaa ggtcataccc agcttctttg gtgtcagttc tcaggtttcc 3720
acccgcccca gggtggttga attgggcaaa tctgtgcaga aaatctgtga tggtaagatt 3780
tcctgtctga aaatgctgct ttgcagcatt tgtagagtgt tattggtctg gaaaaaacac 3840
agaaattgag gccccagcaa aaactgaaca ttccctcatt cttattcttc agtaacattt 3900
ttttgtttca taaagtacac agaggtcttg tttacccctc tttgctcgta tctcacatta 3960
tagtgaagca atactttgcc aatatgagga cggtcaaagc tgaatctggc ccaagcaatg 4020
ttttctgttg tttttagaaa ctgttgcgtt gcaggaagtt ccaaagcaca gcgctgctgt 4080
gggagccaga tcccctcacc accaccagca gcagtgatgt gtggccatgc cccagtccag 4140
cacctctccc cagttttagg ggacagcagg gacaggtgcc accttcccta ggctgacaag 4200
gggcacatca tccccttctc aaggctctgt gggggagcag atttcctgtc ccttgcatta 4260
atgtcatggg aaaagatggc tgccaagcac agaaagctgt ctgtgttctc tgccaaatcc 4320
tcgtgctatt ttgaccctgc actgaatgtg tctgaattta aggaggattt cctccctgcc 4380
catcctggct gctgctacag ggtcccctca agcagcaaga acacaagatg atgtcatgtc 4440
gagagaaata aggagatcta aagcagtccc tctcttcttc taccctctct ctcaaag 4497

<210> 3
<211> 1260
<212> DNA
<213> Gallus gallus

<220>
<223> Description of Combined DNA/RNA Molecule:
      Predicted FMO3 protein (complete codons) from
      compiled predicted exonic regions

<220>
<221> CDS
<222> (1)..(1260)

<400> 3
atg gtg cga cgc gtg gct gtg gtg ggc gcg ggc atc agc ggg ttg gcg 48

Met Val Arg Arg Val Ala Val Val Gly Ala Gly Ile Ser Gly Leu Ala
1               5                   10                  15

gcc acc aag tgc tgc ctg gaa gag gga ctg gag ccc acc tgc ttt gag    96
Ala Thr Lys Cys Cys Leu Glu Glu Gly Leu Glu Pro Thr Cys Phe Glu
            20                  25                  30

cag agc gag gac atc ggg ggg ctc tgg cgc tac acg gaa aag cca gag    144
Gln Ser Glu Asp Ile Gly Gly Leu Trp Arg Tyr Thr Glu Lys Pro Glu
        35                  40                  45

gaa ggc aga gct agc atc tac cgc act gtc ttc acc aat tcc tgc aag    192
Glu Gly Arg Ala Ser Ile Tyr Arg Thr Val Phe Thr Asn Ser Cys Lys
    50                  55                  60

gag atg atg tgc tac ccc gac ttc ccc ttc cct gat gac tac ccc aac    240
Glu Met Met Cys Tyr Pro Asp Phe Pro Phe Pro Asp Asp Tyr Pro Asn
65                  70                  75                  80

tac ata cac aac gca cgg ctc cat aag tac atc cgt gac tac gca cag    288
Tyr Ile His Asn Ala Arg Leu His Lys Tyr Ile Arg Asp Tyr Ala Gln
                85                  90                  95

cat ttt gac ctc ctc cgg cac atc cgc ttc aag acc aca gtc acc aag    336
His Phe Asp Leu Leu Arg His Ile Arg Phe Lys Thr Thr Val Thr Lys
            100                 105                 110

ata agg aag cgc cct gac ttc agt gct acg ggg cag tgg gag gtg gtt    384
Ile Arg Lys Arg Pro Asp Phe Ser Ala Thr Gly Gln Trp Glu Val Val
        115                 120                 125

aca cgg agt gac ggg aag gaa gag gca gcg gtt ttt gat gct gtt atg    432
Thr Arg Ser Asp Gly Lys Glu Glu Ala Ala Val Phe Asp Ala Val Met
    130                 135                 140

gtt tgc act ggg cac cat gtc tac cca aac ctc ccc ctt gcc cac ttc    480
Val Cys Thr Gly His His Val Tyr Pro Asn Leu Pro Leu Ala His Phe
145                 150                 155                 160

cca ggg ata gag aag ttt aaa ggt tgc tac tta cac agc cgg gac tac    528
Pro Gly Ile Glu Lys Phe Lys Gly Cys Tyr Leu His Ser Arg Asp Tyr
                165                 170                 175

aag gag cca gaa aag ttc aga ggg aag aag gtg ctg gtg gtt ggc ttg    576
Lys Glu Pro Glu Lys Phe Arg Gly Lys Lys Val Leu Val Val Gly Leu
            180                 185                 190

ggc aac tcc ggc tgt gac att gct gtg gag ctc agc acc gtg gcc tcg    624
Gly Asn Ser Gly Cys Asp Ile Ala Val Glu Leu Ser Thr Val Ala Ser
        195                 200                 205

cag gtt tac ctg agc tcc cga aga ggt tcc tgg gtg atg agc cgt gtc    672
Gln Val Tyr Leu Ser Ser Arg Arg Gly Ser Trp Val Met Ser Arg Val
    210                 215                 220

tgg aac ttc ggc tac ccc tgg gac atg ctg ctc atc acc cgc ttc tgg    720
Trp Asn Phe Gly Tyr Pro Trp Asp Met Leu Leu Ile Thr Arg Phe Trp
225                 230                 235                 240

acg tgg ctg gac aac ttt ctc ccc aag gca gtc agt gac tgg ctc tat    768
Thr Trp Leu Asp Asn Phe Leu Pro Lys Ala Val Ser Asp Trp Leu Tyr
                245                 250                 255

gtc agg agc atg aac cag cag tac aaa cac gag gat ttt ggc cta atg    816
Val Arg Ser Met Asn Gln Gln Tyr Lys His Glu Asp Phe Gly Leu Met
            260                 265                 270

ccg gtg gat gga tca tcc cgc agg gaa ccg gtg ttg aat gat gac atc    864

Pro Val Asp Gly Ser Ser Arg Arg Glu Pro Val Leu Asn Asp Asp Ile
        275                 280                 285

ctg agc cgc atc acc tgt ggt gtg gtg ctg ata aag cca aat gtg aag     912
Leu Ser Arg Ile Thr Cys Gly Val Val Leu Ile Lys Pro Asn Val Lys
    290                 295                 300

gaa ttc aga gaa aca tca gtc ttg ttt caa gat ggg act gtg cag gac     960
Glu Phe Arg Glu Thr Ser Val Leu Phe Gln Asp Gly Thr Val Gln Asp
305                 310                 315                 320

gac ctc gat gcg gtt atc ttt gcc tct ggt tac tct cac tcc ttt cct     1008
Asp Leu Asp Ala Val Ile Phe Ala Ser Gly Tyr Ser His Ser Phe Pro
                325                 330                 335

ttc atg gag gat gaa tcc atc atc gaa agc aag aac aac gag gct acc     1056
Phe Met Glu Asp Glu Ser Ile Ile Glu Ser Lys Asn Asn Glu Ala Thr
            340                 345                 350

ctc tac aaa tgc att gtt cct cct aag ttg gag aag cca acc atg gca     1104
Leu Tyr Lys Cys Ile Val Pro Pro Lys Leu Glu Lys Pro Thr Met Ala
        355                 360                 365

gtc att gga ttg gtc cag tcc ttt gga tcc gcc atc ccg aca gca gat     1152
Val Ile Gly Leu Val Gln Ser Phe Gly Ser Ala Ile Pro Thr Ala Asp
    370                 375                 380

gtc cag tgc cgc tgg gca gtc aag gtg ttt cag ggg ctc tgc act ctc     1200
Val Gln Cys Arg Trp Ala Val Lys Val Phe Gln Gly Leu Cys Thr Leu
385                 390                 395                 400

ccc tca gtc aac gag atg ctg gag gac ata gag gag aag aag agg aat     1248
Pro Ser Val Asn Glu Met Leu Glu Asp Ile Glu Glu Lys Lys Arg Asn
                405                 410                 415

aag atc aga tgg                                                     1260
Lys Ile Arg Trp
            420


<210> 4
<211> 420
<212> PRT
<213> Gallus gallus
<223> Description of Combined DNA/RNA Molecule:
      Predicted FMO3 protein (complete codons) from
      compiled predicted exonic regions

<400> 4
Met Val Arg Arg Val Ala Val Val Gly Ala Gly Ile Ser Gly Leu Ala
  1               5                  10                  15

Ala Thr Lys Cys Cys Leu Glu Glu Gly Leu Glu Pro Thr Cys Phe Glu
             20                  25                  30

Gln Ser Glu Asp Ile Gly Gly Leu Trp Arg Tyr Thr Glu Lys Pro Glu
         35                  40                  45

Glu Gly Arg Ala Ser Ile Tyr Arg Thr Val Phe Thr Asn Ser Cys Lys
     50                  55                  60

Glu Met Met Cys Tyr Pro Asp Phe Pro Phe Pro Asp Asp Tyr Pro Asn
 65                  70                  75                  80

Tyr Ile His Asn Ala Arg Leu His Lys Tyr Ile Arg Asp Tyr Ala Gln
                 85                  90                  95

His Phe Asp Leu Leu Arg His Ile Arg Phe Lys Thr Thr Val Thr Lys

            100                 105                 110

Ile Arg Lys Arg Pro Asp Phe Ser Ala Thr Gly Gln Trp Glu Val Val
        115                 120                 125

Thr Arg Ser Asp Gly Lys Glu Glu Ala Ala Val Phe Asp Ala Val Met
    130                 135                 140

Val Cys Thr Gly His His Val Tyr Pro Asn Leu Pro Leu Ala His Phe
145                 150                 155                 160

Pro Gly Ile Glu Lys Phe Lys Gly Cys Tyr Leu His Ser Arg Asp Tyr
                165                 170                 175

Lys Glu Pro Glu Lys Phe Arg Gly Lys Lys Val Leu Val Val Gly Leu
            180                 185                 190

Gly Asn Ser Gly Cys Asp Ile Ala Val Glu Leu Ser Thr Val Ala Ser
        195                 200                 205

Gln Val Tyr Leu Ser Ser Arg Arg Gly Ser Trp Val Met Ser Arg Val
    210                 215                 220

Trp Asn Phe Gly Tyr Pro Trp Asp Met Leu Leu Ile Thr Arg Phe Trp
225                 230                 235                 240

Thr Trp Leu Asp Asn Phe Leu Pro Lys Ala Val Ser Asp Trp Leu Tyr
                245                 250                 255

Val Arg Ser Met Asn Gln Gln Tyr Lys His Glu Asp Phe Gly Leu Met
            260                 265                 270

Pro Val Asp Gly Ser Ser Arg Arg Glu Pro Val Leu Asn Asp Asp Ile
        275                 280                 285

Leu Ser Arg Ile Thr Cys Gly Val Val Leu Ile Lys Pro Asn Val Lys
    290                 295                 300

Glu Phe Arg Glu Thr Ser Val Leu Phe Gln Asp Gly Thr Val Gln Asp
305                 310                 315                 320

Asp Leu Asp Ala Val Ile Phe Ala Ser Gly Tyr Ser His Ser Phe Pro
                325                 330                 335

Phe Met Glu Asp Glu Ser Ile Ile Glu Ser Lys Asn Asn Glu Ala Thr
            340                 345                 350

Leu Tyr Lys Cys Ile Val Pro Pro Lys Leu Glu Lys Pro Thr Met Ala
        355                 360                 365

Val Ile Gly Leu Val Gln Ser Phe Gly Ser Ala Ile Pro Thr Ala Asp
    370                 375                 380

Val Gln Cys Arg Trp Ala Val Lys Val Phe Gln Gly Leu Cys Thr Leu
385                 390                 395                 400

Pro Ser Val Asn Glu Met Leu Glu Asp Ile Glu Glu Lys Lys Arg Asn
                405                 410                 415

Lys Ile Arg Trp
            420

<210> 5
<211> 20
<212> DNA
<213> Artificial Sequence

<220>
<223> Description of Artificial Sequence: forward
      amplification primer for chicken (Gallus gallus)
      FMO3 gene

<400> 5
ctttgctctt ggcaggaaac                                                20


<210> 6
<211> 20
<212> DNA
<213> Artificial Sequence

<220>
<223> Description of Artificial Sequence: reverse
      amplification primer for chicken (Gallus gallus)
      FMO3 gene

<400> 6
agccatcacc cagagctaac                                                20


<210> 7
<211> 20
<212> DNA
<213> Artificial Sequence

<220>
<223> Description of Artificial Sequence: forward
      amplification primer for chicken (Gallus gallus)
      FMO3 gene

<400> 7
cctggatttt gttcccttga                                                20


<210> 8
<211> 20
<212> DNA
<213> Artificial Sequence

<220>
<223> Description of Artificial Sequence: reverse
      amplification primer for chicken (Gallus gallus)
      FMO3 gene

<400> 8
tggtgaagac agtgcggtag                                                20


<210> 9
<211> 20
<212> DNA
<213> Artificial Sequence

<220>
<223> Description of Artificial Sequence: forward
      amplification primer for chicken (Gallus gallus)
      FMO3 gene

<400> 9
tttggggtac ccatgactgt                                                20


<210> 10
<211> 20

<212> DNA
<213> Artificial Sequence

<220>
<223> Description of Artificial Sequence: reverse
      amplification primer for chicken (Gallus gallus)
      FMO3 gene

<400> 10
ttttccccac cagagacaag                                                    20


<210> 11
<211> 20
<212> DNA
<213> Artificial Sequence

<220>
<223> Description of Artificial Sequence: forward
      amplification primer for chicken (Gallus gallus)
      FMO3 gene

<400> 11
tgtgatggct gaatcacgtt                                                    20


<210> 12
<211> 20
<212> DNA
<213> Artificial Sequence

<220>
<223> Description of Artificial Sequence: reverse
      amplification primer for chicken (Gallus gallus)
      FMO3 gene

<400> 12
tgctggttgg gtagtgtcaa                                                    20


<210> 13
<211> 20
<212> DNA
<213> Artificial Sequence

<220>
<223> Description of Artificial Sequence: forward
      amplification primer for chicken (Gallus gallus)
      FMO3 gene

<400> 13
gcaccatgtc tacccaaacc                                                    20


<210> 14
<211> 20
<212> DNA
<213> Artificial Sequence

<220>
<223> Description of Artificial Sequence: reverse
      amplification primer for chicken (Gallus gallus)
      FMO3 gene

<400> 14
accagcacct tcttccctct                                                    20

<210> 15
<211> 20
<212> DNA
<213> Artificial Sequence

<220>
<223> Description of Artificial Sequence: forward
      amplification primer for chicken (Gallus gallus)
      FMO3 gene

<400> 15
tggctttggg aacagatagc                                                    20


<210> 16
<211> 20
<212> DNA
<213> Artificial Sequence

<220>
<223> Description of Artificial Sequence: reverse
      amplification primer for chicken (Gallus gallus)
      FMO3 gene

<400> 16
ctgtgagcaa ctcccagaca                                                    20


<210> 17
<211> 20
<212> DNA
<213> Artificial Sequence

<220>
<223> Description of Artificial Sequence: forward
      amplification primer for chicken (Gallus gallus)
      FMO3 gene

<400> 17
tgtctggaac ttcggctacc                                                    20


<210> 18
<211> 20
<212> DNA
<213> Artificial Sequence

<220>
<223> Description of Artificial Sequence: reverse
      amplification primer for chicken (Gallus gallus)
      FMO3 gene

<400> 18
gatccaaagg actggaccaa                                                    20


<210> 19
<211> 20
<212> DNA
<213> Artificial Sequence

<220>
<223> Description of Artificial Sequence: forward
      amplification primer for chicken (Gallus gallus)
      FMO3 gene

<400> 19
cagggtggta tcaagcctgt                                                    20

<210> 20
<211> 20
<212> DNA
<213> Artificial Sequence

<220>
<223> Description of Artificial Sequence: reverse
      amplification primer for chicken (Gallus gallus)
      FMO3 gene

<400> 20
tctcttccct tgagcttgga                                                    20


<210> 21
<211> 20
<212> DNA
<213> Artificial Sequence

<220>
<223> Description of Artificial Sequence: forward
      amplification primer for chicken (Gallus gallus)
      FMO3 gene

<400> 21
cttatcaacc agccgcagat                                                    20


<210> 22
<211> 20
<212> DNA
<213> Artificial Sequence

<220>
<223> Description of Artificial Sequence: reverse
      amplification primer for chicken (Gallus gallus)
      FMO3 gene

<400> 22
gatttgccca attcaaccac                                                    20


<210> 23
<211> 20
<212> DNA
<213> Artificial Sequence

<220>
<223> Description of Artificial Sequence: forward
      amplification primer for chicken (Gallus gallus)
      FMO3 gene

<400> 23
gcactctccc ctcagtcaac                                                    20


<210> 24
<211> 20
<212> DNA
<213> Artificial Sequence

<220>
<223> Description of Artificial Sequence: reverse
      amplification primer for chicken (Gallus gallus)
      FMO3 gene

<400> 24
tctgcagcgt gttgcttatc                                                    20


<210> 25
<211> 20
<212> DNA
<213> Artificial Sequence

<220>
<223> Description of Artificial Sequence: forward
      amplification primer for putative chicken (Gallus
      gallus) FMO3 sequence

<400> 25
cctttagagc tgaccctcca                                                    20


<210> 26
<211> 20
<212> DNA
<213> Artificial Sequence

<220>
<223> Description of Artificial Sequence: reverse
      amplification primer for putative chicken (Gallus
      gallus) FMO3 sequence

<400> 26
atagttgggt gctggtcgag                                                    20


<210> 27
<211> 20
<212> DNA
<213> Artificial Sequence

<220>
<223> Description of Artificial Sequence: forward
      amplification primer for putative chicken (Gallus
      gallus) FMO3 sequence

<400> 27
tgctgtgaga gagggttgtg                                                    20


<210> 28
<211> 20
<212> DNA
<213> Artificial Sequence

<220>
<223> Description of Artificial Sequence: reverse
      amplification primer for putative chicken (Gallus
      gallus) FMO3 sequence

<400> 28
acgctagctg tcatcggttt                                                    20


<210> 29
<211> 20
<212> DNA
<213> Artificial Sequence

<220>
<223> Description of Artificial Sequence: forward
      amplification primer for putative chicken (Gallus

gallus) FMO3 sequence

<400> 29
tggtgggtct tgctaacctt                                                    20


<210> 30
<211> 20
<212> DNA
<213> Artificial Sequence

<220>
<223> Description of Artificial Sequence: reverse
      amplification primer for putative chicken (Gallus
      gallus) FMO3 sequence

<400> 30
   cagggggaag gtgtaggttt                                                   20


<210> 31
<211> 20
<212> DNA
<213> Artificial Sequence

<220>
<223> Description of Artificial Sequence: forward
      amplification primer for putative chicken (Gallus
      gallus) FMO3 sequence

<400> 31
ttgagaatgc ccagagtgtg                                                    20


<210> 32
<211> 20
<212> DNA
<213> Artificial Sequence

<220>
<223> Description of Artificial Sequence: reverse
      amplification primer for putative chicken (Gallus
      gallus) FMO3 sequence

<400> 32
aaagcgctgt ttttgctgat                                                    20


<210> 33
<211> 20
<212> DNA
<213> Artificial Sequence

<220>
<223> Description of Artificial Sequence: forward
      amplification primer for putative chicken (Gallus
      gallus) FMO3 sequence

<400> 33
ggaggtgacc cactgtcact                                                    20


<210> 34
<211> 20
<212> DNA
<213> Artificial Sequence

<220>

<223> Description of Artificial Sequence: reverse
      amplification primer for putative chicken (Gallus
      gallus) FMO3 sequence

<400> 34
tggagagcaa aggcaaaact                                                20


<210> 35
<211> 29
<212> DNA
<213> Artificial Sequence

<220>
<223> Description of Artificial Sequence: single-base
      extension (SBE) primer for SNP detection in
      chicken (Gallus gallus) FMO3 gene

<400> 35
ctgactgact gaggactgga gcccacctg                                      29


<210> 36
<211> 34
<212> DNA
<213> Artificial Sequence

<220>
<223> Description of Artificial Sequence: single-base
      extension (SBE) primer for SNP detection in
      chicken (Gallus gallus) FMO3 gene

<400> 36
tttttttttt ttttttgctg cgagagccgc tcaa                                34


<210> 37
<211> 39
<212> DNA
<213> Artificial Sequence

<220>
<223> Description of Artificial Sequence: single-base
      extension (SBE) primer for SNP detection in
      chicken (Gallus gallus) FMO3 gene

<400> 37
tttttttttt tttttttggc atagaaagca tgggaggaa                           39


<210> 38
<211> 44
<212> DNA
<213> Artificial Sequence

<220>
<223> Description of Artificial Sequence: single-base
      extension (SBE) primer for SNP detection in
      chicken (Gallus gallus) FMO3 gene

<400> 38
tttttttttt tttttttttt ttttttcccc gacttcccct tccc                     44


<210> 39
<211> 49
<212> DNA
<213> Artificial Sequence

<220>
<223> Description of Artificial Sequence: single-base
      extension (SBE) primer for SNP detection in
      chicken (Gallus gallus) FMO3 gene

<400> 39
   tttttttttt tttttttttt tttttttttt ctggttggag ctgatggcc                49


<210> 40
<211> 54
<212> DNA
<213> Artificial Sequence

<220>
<223> Description of Artificial Sequence: single-base
      extension (SBE) primer for SNP detection in
      chicken (Gallus gallus) FMO3 gene

<400> 40
   tttttttttt tttttttttt tttttttttt tttttacctg agctcccgaa gagg          54


<210> 41
<211> 59
<212> DNA
<213> Artificial Sequence

<220>
<223> Description of Artificial Sequence: single-base
      extension (SBE) primer for SNP detection in
      chicken (Gallus gallus) FMO3 gene

<400> 41
   tttttttttt tttttttttt tttttttttt ttttgaagta gcatttccca tcagcaatt  59


<210> 42
<211> 29
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 42
   tttttttgga gcatcacaca ccttttgac                                    29


<210> 43
<211> 34
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 43
tttttttttt tttcaaccca ttccatgctc ccag                              34

<210> 44
<211> 39
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 44
tttttttttt ttttttcaat atcacagccg gtgttccca                    39


<210> 45
<211> 44
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 45
tttttttttt tttttttttt ttttgagaag cgagcagggc tgtt              44


<210> 46
<211> 49
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 46
tttttttttt tttttttttt ttttttgctt tacagtgagt gagctctgc         49


<210> 47
<211> 54
<212> DNA
<213> Artificial Sequence

<220>
<223> Description of Artificial Sequence: single-base
      exten- sion (SBE) primer for SNP detection in
      chicken (Gallus gallus) sequences homologous to
      human FMO gene

<400> 47
tttttttttt tttttttttt tttttttttt ttttttagca tcagctgggc tggc   54


<210> 48
<211> 59
<212> DNA
<213> Artificial Sequence

<220>
<223> Description of Artificial Sequence: single-base
      exten- sion (SBE) primer for SNP detection in

chicken (Gallus gallus) sequences homologous to
human FMO gene

<400> 48
ttttttttt ttttttttt ttttttttt ttttttttt tcggggccct gaaccaagg 59


<210> 49
<211> 64
<212> DNA
<213> Artificial Sequence

<220>
<223> Description of Artificial Sequence: single-base
exten- sion (SBE) primer for SNP detection in
chicken (Gallus gallus) sequences homologous to
human FMO gene

<400> 49
   tttttttttt tttttttttt tttttttttt tttttttttt tttggctacc ccattgacac 60
   catc 64


<210> 50
<211> 36
<212> DNA
<213> Gallus gallus

<220>
<223> /note="Sequence for chicken FMO3 gene
corresponding to exon 1"

<400> 50
ccactccaag ctctgaggca ccacaggctg cacagg 36


<210> 51
<211> 92
<212> DNA
<213> Gallus gallus

<220>
<223> /note="Sequence for chicken FMO3 gene
corresponding to intron 1"

<400> 51
taagcgagga cagatgaaaa agccccaggt gttgcatggg gccagactgg gcctaggagc 60
gcaacatggc gccatgacgg ctgctctccc ac 92


<210> 52
<211> 143
<212> DNA
<213> Gallus gallus

<220>
<223> /note="Sequence for chicken FMO3 gene
corresponding to exon 2"

<400> 52
cacaggacac catggtgcga cgcgtggctg tggtgggcgc gggcatcagc gggttggcgg 60
ccaccaagtg ctgcctggaa gagggactgg agcccacctg ctttgagcag agcgaggaca 120
tcggggggct ctggcgctac acg 143


<210> 53
<211> 153
<212> DNA

<213> Gallus gallus

<220>
<223> /note="Sequence for chicken FMO3 gene
      corresponding to partial intron 2A"

<400> 53
tgaggggttt gggctgcgtg gtcttctggc aatctggggc taggggagaa tctggggggg 60
cggtggctgc gagagccgct caagtggagg aactgaccgt gttaaaactc atgatgaggg 120
gcaaagacat caggttagct ctgggtgatg gct                              153


<210> 54
<211> 645
<212> DNA
<213> Gallus gallus

<220>
<223> /note="Sequence for chicken FMO3 gene
      corresponding to partial intron 2B"

<400> 54
ccttgagagc aataagcctg caatgtcccc tgtgtgctcc agctaagctg agaaaaatat 60
cactttgagc tctgcaagtg atgtttataa gaagcgaggc ttgaagttga aaataacagc 120
tgtgtgtgtg cccaaggtta agcccagaca acaacagaac tgatctttct acagtggtaa 180
atgtagaaga cattatgcca agtagaggac tctgaggtta ttacagggat gtactgtgta 240
tggctcttca ggatgagccc acaaagcctg tgagcacatg tggaaggtaa gcagtgggct 300
ggtgatgacc ctctggggtg ctcagtgccc ctttttctgg gcttggttgg tgaagctgag 360
tggaaggacg ggctgggaat tacctggcca gacaaggagg gcatagaaag catgggagga 420
actgggttgc aagtgaagac attgctctga gagccagaca ttgtggttca ggtccccaga 480
actgaggttc tcctagtcgt gttcttgggt cagttctcct gacccaaagt catgacattg 540
ggtgaggtta tctccagatt ggctcagcat cttcttgcct gtggcttatg acttttgggg 600
gtacccatga ctgtgacccc tctaaccgaa gctctcttcc tccag                 645


<210> 55
<211> 189
<212> DNA
<213> Gallus gallus

<220>
<223> /note="Sequence for chicken FMO3 gene
      corresponding to exon 3"

<400> 55
gaaaagccag aggaaggcag agctagcatc taccgcactg tcttcaccaa ttcctgcaag 60
gagatgatgt gctaccccga cttccccttc cctgatgact accccaacta catacacaac 120
gcacggctcc ataagtacat ccgtgactac gcacagcatt ttgacctcct ccggcacatc 180
cgcttcaag                                                         189


<210> 56
<211> 317
<212> DNA
<213> Gallus gallus

<220>
<223> /note="Sequence for chicken FMO3 gene
      corresponding to intron 3"

<400> 56
gtaggaggga tggactttgc tgctgtgtct cagagaaaat cctgcggcag acatcctgag 60
ggaaagtctt gttggttttg ttaaattggg tctcaccagc tgctggttgg gtagtgtcaa 120
atgatcagtc atcacgggga aaccattgct tctgagggtg gaaaatcacg tggaatggga 180
ggacaccagt gggttttctc tatccaaaat aattgccaag tctgtgacct tgtctctggt 240
ggggaaaagg acagagcctg cctggctgca gtggggtacc cggagcactt cccaaacctt 300
tatccctttc ctttaag                                                317

<210> 57
<211> 162
<212> DNA
<213> Gallus gallus

<220>
<223> /note="Sequence for chicken FMO3 gene
      corresponding to exon 4"

<400> 57
accacagtca ccaagataag gaagcgccct gacttcagtg ctacggggca gtgggaggtg 60
gttacacgga gtgacgggaa ggaagaggca gcggtttttg atgctgttat ggtttgcact 120
gggcaccatg tctacccaaa cctccccctt gcccacttcc ca                    162


<210> 58
<211> 821
<212> DNA
<213> Gallus gallus

<220>
<223> /note="Sequence for chicken FMO3 gene
      corresponding to intron 4"

<400> 58
gtaagctata tgaacgtgat tcagccatca cacagccagg tgtgcacttt acattcaaat 60
gttgggtttt ggtttttttt ttcagcatca aagattggtt ttaggggtga gctctgctca 120
ggaaatcaaa acttttattt aagacaaaag actgtatcca aaccatgtca aagcagggac 180
attcttactg gaggcaatgc taatatttcc tggaaaaatt attgcagggt ttccatttag 240
tagaagtttc ttcactaaag ggtttcgtaa agcacttctt ttggcatttt ttccatggtt 300
tctagtgcaa atgctgtgcg caaactttgc tcctgcttac aggatcagca gtgggaaaac 360
ttcctatgat aagaagtatt tggtgctggg ggtctgtttg gaggaggtat ggtcttggga 420
cactgactgg ctgggaaaac tcccattgtg tcctttgtca ccttctgttt tagaaattaa 480
agttgctttc ccatcacttg ctggcgcaaa ggcacgaaga cacaccaagc ggatggaacg 540
tggctggtct ggtgtctcag ggtgatgatg ctgagttgta tctttaaggc acacaaatac 600
cacacaaagt tatcctgttc tctcatgctg gggctcccct ctgtttaaat attgaatgaa 660
tccagagaca aaaacccatc tacaggtttg cagcatccct ctgctgtgag caactcccag 720
acatgctcca gcagcgcctg caggaatggg agcagccaag gttaacgcag ccacacttct 780
tcactgatga cttacatccc tttctgtctt cctgctccca g                     821


<210> 59
<211> 144
<212> DNA
<213> Gallus gallus

<220>
<223> /note="Sequence for chicken FMO3 gene
      corresponding to exon 5"

<400> 59
gggatagaga agtttaaagg ttgctactta cacagccggg actacaagga gccagaaaag 60
ttcagaggga agaaggtgct ggtggttggc ttgggcaact ccggctgtga cattgctgtg 120
gagctcagca ccgtggcctc gcag                                        144


<210> 60
<211> 82
<212> DNA
<213> Gallus gallus

<220>
<223> /note="Sequence for chicken FMO3 gene
      corresponding to intron 5"

<400> 60
gtatggggac ctgaaccagg gctggttgga gctgatggcc gccacgacgg tggctcaacc 60

ctgcctcacc atcccccccc ag 82

<210> 61
<211> 201
<212> DNA
<213> Gallus gallus

<220>
<223> /note="Sequence for chicken FMO3 gene
      corresponding to exon 6"

<400> 61
gtttacctga gctcccgaag aggttcctgg gtgatgagcc gtgtctggaa cttcggctac 60
ccctgggaca tgctgctcat cacccgcttc tggacgtggc tggacaactt tctccccaag 120
gcagtcagtg actggctcta tgtcaggagc atgaaccagc agtacaaaca cgaggatttt 180
ggcctaatgc cggtggatgg a 201

<210> 62
<211> 111
<212> DNA
<213> Gallus gallus

<220>
<223> /note="Sequence for chicken FMO3 gene
      corresponding to intron 6"

<400> 62
gtaagcatga ggctatctgt tcccaaagcc agaagtgctg gtcccaggca gggtggtatc 60
aagcctgtac agggccagca gcagcagcca acttaggctt tttccttgca g 111

<210> 63
<211> 357
<212> DNA
<213> Gallus gallus

<220>
<223> /note="Sequence for chicken FMO3 gene
      corresponding to exon 7"

<400> 63
tcatcccgca gggaaccggt gttgaatgat gacatcctga gccgcatcac ctgtggtgtg 60
gtgctgataa agccaaatgt gaaggaattc agagaaacat cagtcttgtt tcaagatggg 120
actgtgcagg acgacctcga tgcggttatc tttgccyctg gttactctca ctcctttcct 180
ttcatggagg atgaatccat catcgaaagc aagaacaacg aggctaccct ctacaaatgc 240
attgttcctc ctaagttgga gaagccaacc atggcagtca ttggattggt ccagtccttt 300
ggatccgcca tcccgacagc agatgtccag tgccgctggg cagtcaaggt gtttcag 357

<210> 64
<211> 391
<212> DNA
<213> Gallus gallus

<220>
<223> /note="Sequence for chicken FMO3 gene
      corresponding to intron 7"

<400> 64
gtaggtgaat gagctaaggt ccctaaatgg gaagtagcat ttcccatcag caatttcaca 60
gattccaact aaaaatgagg gcagtgcatg agtgctggaa ataccagcaa cgggttctcc 120
ccgggatggg ctgccttgtg ccatggagaa taggtcactt atcaaccagc cgcagatgtc 180
agtgaggtcc ctgtggtgag gcagctatct gggcatcgcc acacatccta aaggacaggg 240
ggatctgttt tatcgcaacc ctgatgccca gtttgatcca agctcaaggg aagagaagtg 300
cccaaggtga tccctacaga ccccacctgc tgcaggaggg atctcctggg catcatcacc 360
ctttgccgac actcttcctg ttctttccag g 391

<210> 65
<211> 75
<212> DNA
<213> Gallus gallus

<220>
<223> /note="Sequence for chicken FMO3 gene
      corresponding to exon 8"

<400> 65
gggctctgca ctctcccctc agtcaacgag atgctggagg acatagagga gaagaagagg 60
aataagatca gatgg                                                  75


<210> 66
<211> 1002
<212> DNA
<213> Gallus gallus

<220>
<223> /note="Sequence for chicken FMO3 gene
      corresponding to intron 8"

<400> 66
taggtgcagc tcatgggacg ctgcccagga aggaggagga aaaaccttgc tgtcttctca 60
gcctgggtgg gctggatgtg ggcatgggga gggctgcagc aggggcaagc tcctagcaag 120
gagaccattc tgaactgctc cgttcatgac ccagatgagc aaacccaatg ggaccaacac 180
aggaaggtca tacccagctt ctttggtgtc agttctcagg tttccacccg ccccagggtg 240
gttgaattgg gcaaatctgt gcagaaaatc tgtgatggta agatttcctg tctgaaaatg 300
ctgctttgca gcatttgtag agtgttattg gtctggaaaa aacacagaaa ttgaggcccc 360
agcaaaaact gaacattccc tcattcttat tcttcagtaa catttttttg tttcataaag 420
tacacagagg tcttgtttac ccctctttgc tcgtatctca cattatagtg aagcaatact 480
ttgccaatat gaggacggtc aaagctgaat ctggcccaag caatgttttc tgttgttttt 540
agaaactgtt gcgttgcagg aagttccaaa gcacagcgct gctgtgggag ccagatcccc 600
tcaccaccac cagcagcagt gatgtgtggc catgccccag tccagcacct ctccccagtt 660
ttaggggaca gcagggacag gtgccacctt ccctaggctg acaaggggca catcatcccc 720
ttctcaaggc tctgtggggg agcagatttc ctgtcccttg cattaatgtc atgggaaaag 780
atggctgcca agcacagaaa gctgtctgtg ttctctgcca aatcctcgtg ctattttgac 840
cctgcactga atgtgtctga atttaaggag gatttcctcc ctgcccatcc tggctgctgc 900
tacagggtcc cctcaagcag caagaacaca agatgatgtc atgtcgagag aaataaggag 960
atctaaagca gtccctctct tcttctaccc tctctctcaa ag                    1002


<210> 67
<211> 20
<212> DNA
<213> Artificial Sequence

<220>
<223> Description of Artificial Sequence: forward
      amplification primer

<220>
<223> /note="Forward amplification primer for
      amplification of chicken homologue of human FMO
      gene (contig 24165.1; unknow genomic location)"

<400> 67
acggtcaaag ctgaatctgg                                             20


<210> 68
<211> 20
<212> DNA
<213> Artificial Sequence

<220>
<223> Description of Artificial Sequence: reverse
      amplification primer

<220>
<223> /note="Reverse amplification primer for
      amplification of chicken homologue of human FMO
      gene (contig 24165.1; unknow genomic location)"

<400> 68
tcccgaacct ttgagagaga                                                20


<210> 69
<211> 514
<212> DNA
<213> Gallus gallus

<220>
<223> /not="Contig 24165.1 (chicken homologue of human
      FMO gene)"

<400> 69
acggtcaaag ctgaatctgg cccaagcaat gttttctgtt gtttttagaa actgttgcgt 60
tgcaggaagt tccaaagcac agcgctgctg tgggagccag atcccctcac caccaccagc 120
agcagtgatg tgtggccatg ccccagtcca gcacctctcc ccagttttag gggacagcag 180
ggacaggtgc caccttccct aggctgacaa ggggcacatc atccccttct caaggctctg 240
tgggggagca gatttcctgt cccttgcatt aatgtcatgg gaaaagatgg ctgccaagca 300
cagaaagctg tctgtgttct ctgccaaatc ctcgtgctat tttgaccctg cactgaatgt 360
gtctgaattt aaggaggatt tcctccctgc ccatcctggc tgctgctaca gggtcccctc 420
aagcagcaag aacacaagat gatgtcatgt cgagagaaat aaggagatct aaagcagtcc 480
ctctcttctt ctaccctctc tctcaaagtt cggg                                514


<210> 70
<211> 1307
<212> DNA
<213> Gallus gallus

<220>
<223> /note="Compiled sequence of 8 confirmed exonic
      regions for chicken (Gallus gallus) FMO3 gene"

<400> 70
ccactccaag ctctgaggca ccacaggctg cacaggcaca ggacaccatg gtgcgacgcg 60
tggctgtggt gggcgcgggc atcagcgggt tggcggccac caagtgctgc ctggaagagg 120
gactggagcc cacctgcttt gagcagagcg aggacatcgg ggggctctgg cgctacacgg 180
aaaagccaga ggaaggcaga gctagcatct accgcactgt cttcaccaat tcctgcaagg 240
agatgatgtg ctaccccgac ttccccttcc ctgatgacta ccccaactac atacacaacg 300
cacggctcca taagtacatc cgtgactacg cacagcattt tgacctcctc cggcacatcc 360
gcttcaagac cacagtcacc aagataagga agcgccctga cttcagtgct acggggcagt 420
gggaggtggt tacacggagt gacgggaagg aagaggcagc ggtttttgat gctgttatgg 480
tttgcactgg gcaccatgtc tacccaaacc tcccccttgc ccacttccca gggatagaga 540
agtttaaagg ttgctactta cacagccggg actacaagga gccagaaaag ttcagaggga 600
agaaggtgct ggtggttggc ttgggcaact ccggctgtga cattgctgtg gagctcagca 660
ccgtggcctc gcaggtttac ctgagctccc gaagaggttc ctgggtgatg agccgtgtct 720
ggaacttcgg ctacccctgg gacatgctgc tcatcacccg cttctggacg tggctggaca 780
actttctccc caaggcagtc agtgactggc tctatgtcag gagcatgaac cagcagtaca 840
aacacgagga ttttggccta atgccggtgg atggatcatc ccgcagggaa ccggtgttga 900
atgatgacat cctgagccgc atcacctgtg gtgtggtgct gataaagcca aatgtgaagg 960
aattcagaga aacatcagtc ttgtttcaag atgggactgt gcaggacgac ctcgatgcgg 1020
ttatctttgc ctctggttac tctcactcct ttcctttcat ggaggatgaa tccatcatcg 1080
aaagcaagaa caacgaggct accctctaca aatgcattgt tcctcctaag ttggagaagc 1140
caaccatggc agtcattgga ttggtccagt cctttggatc cgccatcccg acagcagatg 1200
tccagtgccg ctgggcagtc aaggtgtttc aggggctctg cactctcccc tcagtcaacg 1260
agatgctgga ggacatagag gagaagaaga ggaataagat cagatgg                  1307

<210> 71
<211> 132
<212> DNA
<213> Gallus gallus

<220>
<223> Description of Combined DNA/RNA Molecule: sequence
      of exon 2 of FMO3 gene

<220>
<221> CDS
<222> (1)..(132)

<400> 71
atg gtg cga cgc gtg gct gtg gtg ggc gcg ggc atc agc ggg ttg gcg     48
Met Val Arg Arg Val Ala Val Val Gly Ala Gly Ile Ser Gly Leu Ala
1               5                   10                  15

gcc acc aag tgc tgc ctg gaa gag gga ctg gag ccc acc tgc ttt gag     96
Ala Thr Lys Cys Cys Leu Glu Glu Gly Leu Glu Pro Thr Cys Phe Glu
            20                  25                  30

cag agc gag gac atc ggg ggg ctc tgg cgc tac acg                     132
Gln Ser Glu Asp Ile Gly Gly Leu Trp Arg Tyr Thr
        35                  40


<210> 72
<211> 44
<212> PRT
<213> Gallus gallus
<223> Description of Combined DNA/RNA Molecule: sequence
      of exon 2 of FMO3 gene

<400> 72
Met Val Arg Arg Val Ala Val Val Gly Ala Gly Ile Ser Gly Leu Ala
1               5                   10                  15

Ala Thr Lys Cys Cys Leu Glu Glu Gly Leu Glu Pro Thr Cys Phe Glu
            20                  25                  30

Gln Ser Glu Asp Ile Gly Gly Leu Trp Arg Tyr Thr
        35                  40



<210> 73
<211> 189
<212> DNA
<213> Gallus gallus

<220>
<223> Description of Combined DNA/RNA Molecule: sequence
      of exon 3 of FMO3 gene

<220>
<221> CDS
<222> (1)..(189)

<400> 73
gaa aag cca gag gaa ggc aga gct agc atc tac cgc act gtc ttc acc     48
Glu Lys Pro Glu Glu Gly Arg Ala Ser Ile Tyr Arg Thr Val Phe Thr
1               5                   10                  15

aat tcc tgc aag gag atg atg tgc tac ccc gac ttc ccc ttc cct gat     96
Asn Ser Cys Lys Glu Met Met Cys Tyr Pro Asp Phe Pro Phe Pro Asp
            20                  25                  30

gac tac ccc aac tac ata cac aac gca cgg ctc cat aag tac atc cgt     144
Asp Tyr Pro Asn Tyr Ile His Asn Ala Arg Leu His Lys Tyr Ile Arg
        35                  40                  45

gac tac gca cag cat ttt gac ctc ctc cgg cac atc cgc ttc aag         189
Asp Tyr Ala Gln His Phe Asp Leu Leu Arg His Ile Arg Phe Lys
    50                  55                  60


<210> 74
<211> 63
<212> PRT
<213> Gallus gallus
<223> Description of Combined DNA/RNA Molecule: sequence
      of exon 3 of FMO3 gene

<400> 74
Glu Lys Pro Glu Glu Gly Arg Ala Ser Ile Tyr Arg Thr Val Phe Thr
 1               5                  10                  15

Asn Ser Cys Lys Glu Met Met Cys Tyr Pro Asp Phe Pro Phe Pro Asp
            20                  25                  30

Asp Tyr Pro Asn Tyr Ile His Asn Ala Arg Leu His Lys Tyr Ile Arg
        35                  40                  45

Asp Tyr Ala Gln His Phe Asp Leu Leu Arg His Ile Arg Phe Lys
    50                  55                  60



<210> 75
<211> 162
<212> DNA
<213> Gallus gallus

<220>
<223> Description of Combined DNA/RNA Molecule: sequence
      of exon 4 of FMO3 gene

<220>
<221> CDS
<222> (1)..(162)

<400> 75
acc aca gtc acc aag ata agg aag cgc cct gac ttc agt gct acg ggg     48
Thr Thr Val Thr Lys Ile Arg Lys Arg Pro Asp Phe Ser Ala Thr Gly
 1               5                  10                  15

cag tgg gag gtg gtt aca cgg agt gac ggg aag gaa gag gca gcg gtt     96
Gln Trp Glu Val Val Thr Arg Ser Asp Gly Lys Glu Glu Ala Ala Val
            20                  25                  30

ttt gat gct gtt atg gtt tgc act ggg cac cat gtc tac cca aac ctc     144
Phe Asp Ala Val Met Val Cys Thr Gly His His Val Tyr Pro Asn Leu
        35                  40                  45

ccc ctt gcc cac ttc cca                                             162
Pro Leu Ala His Phe Pro
    50


<210> 76
<211> 54
<212> PRT
<213> Gallus gallus
<223> Description of Combined DNA/RNA Molecule: sequence
      of exon 4 of FMO3 gene

<400> 76
Thr Thr Val Thr Lys Ile Arg Lys Arg Pro Asp Phe Ser Ala Thr Gly
1               5                   10                  15

Gln Trp Glu Val Val Thr Arg Ser Asp Gly Lys Glu Glu Ala Ala Val
            20                  25                  30

Phe Asp Ala Val Met Val Cys Thr Gly His His Val Tyr Pro Asn Leu
        35                  40                  45

Pro Leu Ala His Phe Pro
    50

<210> 77
<211> 144
<212> DNA
<213> Gallus gallus

<220>
<223> Description of Combined DNA/RNA Molecule: sequence
      of exon 5 of FMO3 gene

<220>
<221> CDS
<222> (1)..(144)

<400> 77
ggg ata gag aag ttt aaa ggt tgc tac tta cac agc cgg gac tac aag      48
Gly Ile Glu Lys Phe Lys Gly Cys Tyr Leu His Ser Arg Asp Tyr Lys
1               5                   10                  15

gag cca gaa aag ttc aga ggg aag aag gtg ctg gtg gtt ggc ttg ggc      96
Glu Pro Glu Lys Phe Arg Gly Lys Lys Val Leu Val Val Gly Leu Gly
            20                  25                  30

aac tcc ggc tgt gac att gct gtg gag ctc agc acc gtg gcc tcg cag      144
Asn Ser Gly Cys Asp Ile Ala Val Glu Leu Ser Thr Val Ala Ser Gln
        35                  40                  45

<210> 78
<211> 48
<212> PRT
<213> Gallus gallus
<223> Description of Combined DNA/RNA Molecule: sequence
      of exon 5 of FMO3 gene

<400> 78
Gly Ile Glu Lys Phe Lys Gly Cys Tyr Leu His Ser Arg Asp Tyr Lys
1               5                   10                  15

Glu Pro Glu Lys Phe Arg Gly Lys Lys Val Leu Val Val Gly Leu Gly
            20                  25                  30

Asn Ser Gly Cys Asp Ile Ala Val Glu Leu Ser Thr Val Ala Ser Gln
        35                  40                  45

<210> 79
<211> 201
<212> DNA
<213> Gallus gallus

<220>

<223> Description of Combined DNA/RNA Molecule: sequence
      of exon 6 of FMO3 gene

<220>
<221> CDS
<222> (1)..(201)

<400> 79
gtt tac ctg agc tcc cga aga ggt tcc tgg gtg atg agc cgt gtc tgg      48
Val Tyr Leu Ser Ser Arg Arg Gly Ser Trp Val Met Ser Arg Val Trp
1               5                   10                  15

aac ttc ggc tac ccc tgg gac atg ctg ctc atc acc cgc ttc tgg acg      96
Asn Phe Gly Tyr Pro Trp Asp Met Leu Leu Ile Thr Arg Phe Trp Thr
            20                  25                  30

tgg ctg gac aac ttt ctc ccc aag gca gtc agt gac tgg ctc tat gtc      144
Trp Leu Asp Asn Phe Leu Pro Lys Ala Val Ser Asp Trp Leu Tyr Val
        35                  40                  45

agg agc atg aac cag cag tac aaa cac gag gat ttt ggc cta atg ccg      192
Arg Ser Met Asn Gln Gln Tyr Lys His Glu Asp Phe Gly Leu Met Pro
    50                  55                  60

gtg gat gga                                                          201
Val Asp Gly
65


<210> 80
<211> 67
<212> PRT
<213> Gallus gallus
<223> Description of Combined DNA/RNA Molecule: sequence
      of exon 6 of FMO3 gene

<400> 80
Val Tyr Leu Ser Ser Arg Arg Gly Ser Trp Val Met Ser Arg Val Trp
1               5                   10                  15

Asn Phe Gly Tyr Pro Trp Asp Met Leu Leu Ile Thr Arg Phe Trp Thr
            20                  25                  30

Trp Leu Asp Asn Phe Leu Pro Lys Ala Val Ser Asp Trp Leu Tyr Val
        35                  40                  45

Arg Ser Met Asn Gln Gln Tyr Lys His Glu Asp Phe Gly Leu Met Pro
    50                  55                  60

Val Asp Gly
65



<210> 81
<211> 357
<212> DNA
<213> Gallus gallus

<220>
<223> Description of Combined DNA/RNA Molecule: sequence
      of exon 7 of FMO3 gene

<220>
<221> CDS
<222> (1)..(357)

<400> 81

tca tcc cgc agg gaa ccg gtg ttg aat gat gac atc ctg agc cgc atc     48
Ser Ser Arg Arg Glu Pro Val Leu Asn Asp Asp Ile Leu Ser Arg Ile
1               5                   10                  15

acc tgt ggt gtg gtg ctg ata aag cca aat gtg aag gaa ttc aga gaa     96
Thr Cys Gly Val Val Leu Ile Lys Pro Asn Val Lys Glu Phe Arg Glu
            20                  25                  30

aca tca gtc ttg ttt caa gat ggg act gtg cag gac gac ctc gat gcg     144
Thr Ser Val Leu Phe Gln Asp Gly Thr Val Gln Asp Asp Leu Asp Ala
        35                  40                  45

gtt atc ttt gcc tct ggt tac tct cac tcc ttt cct ttc atg gag gat     192
Val Ile Phe Ala Ser Gly Tyr Ser His Ser Phe Pro Phe Met Glu Asp
    50                  55                  60

gaa tcc atc atc gaa agc aag aac aac gag gct acc ctc tac aaa tgc     240
Glu Ser Ile Ile Glu Ser Lys Asn Asn Glu Ala Thr Leu Tyr Lys Cys
65                  70                  75                  80

att gtt cct cct aag ttg gag aag cca acc atg gca gtc att gga ttg     288
Ile Val Pro Pro Lys Leu Glu Lys Pro Thr Met Ala Val Ile Gly Leu
                85                  90                  95

gtc cag tcc ttt gga tcc gcc atc ccg aca gca gat gtc cag tgc cgc     336
Val Gln Ser Phe Gly Ser Ala Ile Pro Thr Ala Asp Val Gln Cys Arg
            100                 105                 110

tgg gca gtc aag gtg ttt cag                                         357
Trp Ala Val Lys Val Phe Gln
        115

<210> 82
<211> 119
<212> PRT
<213> Gallus gallus
<223> Description of Combined DNA/RNA Molecule: sequence
      of exon 7 of FMO3 gene

<400> 82
Ser Ser Arg Arg Glu Pro Val Leu Asn Asp Asp Ile Leu Ser Arg Ile
1               5                   10                  15

Thr Cys Gly Val Val Leu Ile Lys Pro Asn Val Lys Glu Phe Arg Glu
            20                  25                  30

Thr Ser Val Leu Phe Gln Asp Gly Thr Val Gln Asp Asp Leu Asp Ala
        35                  40                  45

Val Ile Phe Ala Ser Gly Tyr Ser His Ser Phe Pro Phe Met Glu Asp
    50                  55                  60

Glu Ser Ile Ile Glu Ser Lys Asn Asn Glu Ala Thr Leu Tyr Lys Cys
65                  70                  75                  80

Ile Val Pro Pro Lys Leu Glu Lys Pro Thr Met Ala Val Ile Gly Leu
                85                  90                  95

Val Gln Ser Phe Gly Ser Ala Ile Pro Thr Ala Asp Val Gln Cys Arg
            100                 105                 110

Trp Ala Val Lys Val Phe Gln
        115

<210> 83

<211> 75
<212> DNA
<213> Gallus gallus

<220>
<223> Description of Combined DNA/RNA Molecule: sequence
      of exon 8 of FMO3 gene

<220>
<221> CDS
<222> (1)..(75)

<400> 83
ggg ctc tgc act ctc ccc tca gtc aac gag atg ctg gag gac ata gag    48
Gly Leu Cys Thr Leu Pro Ser Val Asn Glu Met Leu Glu Asp Ile Glu
  1               5                  10                  15

gag aag aag agg aat aag atc aga tgg                                75
Glu Lys Lys Arg Asn Lys Ile Arg Trp
             20                  25


<210> 84
<211> 25
<212> PRT
<213> Gallus gallus
<223> Description of Combined DNA/RNA Molecule: sequence
      of exon 8 of FMO3 gene

<400> 84
Gly Leu Cys Thr Leu Pro Ser Val Asn Glu Met Leu Glu Asp Ile Glu
  1               5                  10                  15

Glu Lys Lys Arg Asn Lys Ile Arg Trp
             20                  25